# EUROPEAN PATENT APPLICATION

(11) **EP 4 793 331 A1**
(43) Date of publication of application: **19.08.2026**
(21) Application number: 26157678.9
(22) Date of filing: 10.02.2026
(51) Int. Cl.: C09K 19/34, C09K 19/30

(54) **LIQUID CRYSTALLINE MEDIUM AND COMPOUNDS**

(30) Priority: 13.02.2025 WO PCT/CN2025/077160
(71) Applicant: Merck Patent GmbH, 64293 Darmstadt (DE)
(72) Inventor: Laut, Sven Christian, 64293 Darmstadt (DE); Jao, Mobius, 114 TAIPEI (TW); Yang, Minghui, SHANGHAI, 201206 (CN); Kodek, Thorsten, 64293 Darmstadt (DE); Tavakoli, Ramin, 64293 Darmstadt (DE)
(74) Representative: Merck Patent Association

(57) **Abstract**

A liquid-crystalline medium (also called liquid crystal material) having a nematic phase comprising one or more compounds with a terminal 5,6-difluoro-benzo[b]furan ring as provided by the formulae, the use thereof in an electro-optical display, particularly in an active-matrix display based on the IPS or FFS effect, and to displays, especially energy-saving LC displays or LC displays with a low power consumption, which contain a liquid crystal material of this type.

## Description

The invention relates to a liquid-crystalline medium (also called liquid crystal material) having a nematic phase comprising one or more benzofuran compounds of formula I 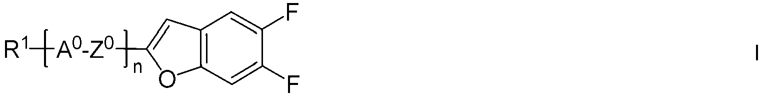
wherein the parameters have the meaning given in the text below,
to novel compounds under formula I, the use thereof in an electro-optical display, particularly in an active-matrix display based on the IPS or the FFS (fringe field switching) effect using dielectrically positive liquid crystals, and to displays of this type which contain a liquid-crystalline medium of this type.

### Background and prior art

IPS and FFS displays using dielectrically positive liquid crystals are well known in the field and have been widely adopted for various types of displays like e. g. desktop monitors and TV sets, but also for gaming, mobile, and automotive applications.

However, recently, IPS and in particular FFS displays using dielectrically negative liquid crystals are widely adopted. The latter ones are sometimes also called UB-FFS (ultra-bright FFS). Such displays are disclosed e. g. in US 20130207038 A1. These displays are characterized by a markedly increased transmission compared to the previously used IPS- and FFS displays, which employ dielectrically positive liquid crystals. These displays using conventional, dielectrically negative liquid crystals, however, have the severe disadvantage of requiring a higher operation voltage than the respective displays using dielectrically positive liquid crystals. Liquid crystalline media used for UB-FFS have a dielectric anisotropy of -0.5 or less and preferably of -1.5 or less.

Liquid crystalline media used for HB-FFS (high brightness FFS) have a dielectric anisotropy of 0.5 or more and preferably of 1.5 or more. Liquid crystalline media used for HB-FFS comprising both dielectrically negative and dielectrically positive liquid crystalline compounds, respectively mesogenic compounds are disclosed e. g. in US 20130207038 A1. These media feature rather large values of ε_{⊥} and of ε_{avg} already, however, their ratio of (ε_{⊥} / Δε) is relatively small.

According to the present application, however, the IPS or the FFS effect with dielectrically positive liquid crystalline media in a homogeneous alignment are preferred.

Industrial application of this effect in electro-optical display elements requires LC phases which have to meet a multiplicity of requirements. Particularly important here are chemical resistance to moisture, air and physical influences, such as heat, radiation in the infrared, visible and ultraviolet regions, and direct (DC) and alternating (AC) electric fields.

Furthermore, LC phases which can be used industrially are required to have a liquid-crystalline mesophase in a suitable temperature range and low viscosity.

None of the series of compounds having a liquid-crystalline mesophase that have been disclosed hitherto includes a single compound which meets all these requirements. Mixtures of two to 25, preferably three to 18, compounds are therefore generally prepared in order to obtain substances which can be used as LC phases.

Development in the area of liquid-crystalline materials is far from complete. In order to improve the properties of liquid-crystalline display elements, attempts are constantly being made to develop novel compounds which enable such displays to be optimised.

### Short description of the invention

An object of the present invention was to provide liquid-crystalline media having advantageous properties. Beside the physical properties required for the materials, their sustainability plays an increasing role, like environment-related properties (e.g. perfluoroalkyl content).

With regard to the most diverse application areas of such compounds with high Δε it was desirable to have further compounds, preferably with high clarification point and low viscosity, which have precisely tailored properties to the respective applications.

The invention was thus based as an object to find new stable compounds which are suitable as components(s) of liquid crystalline media, in particular for e. g. TN, STN, IPS, FFS and TN TFT displays.

In addition, the compounds according to the invention should be thermally and photochemically stable under the conditions prevailing in the applications. As mesogens, they should enable a broad nematic phase in mixtures with liquid crystalline co-components and be excellently miscible with nematic base mixtures, especially at low temperatures. Substances with a low melting point and a low melting enthalpy are also preferred, since these quantities in turn are indicative of the desirable properties mentioned above, such as high solubility, a wide liquid crystalline phase and a low tendency to spontaneous crystallization in mixtures at low temperatures. Solubility at low temperature, avoiding any crystallization, is particularly important for the safe operation and transport of displays in aircraft and in the open air.

Liquid crystalline media having a positive dielectric anisotropy for IPS and FFS displays have already been disclosed.

Some benzofurans as liquid crystal materials and their synthesis are disclosed in CN108018049 A. Some mixture applications are disclosed in CN108003895 A.

The phase range of the liquid-crystal mixture must be sufficiently broad for the intended application of the display. The response times of the liquid-crystal media in the displays should be as low as possible, especially for video, animated simulation and gaming applications. This is particularly important for displays for television or multimedia applications. In order to improve the response times, it has repeatedly been proposed in the past to optimise the rotational viscosity of the liquid-crystal media (γ₁), i.e. to achieve media having the lowest possible rotational viscosity. However, the results achieved here are inadequate for many applications and therefore make it appear desirable to find further optimisation approaches.

Adequate stability of the media to extreme loads, in particular to UV exposure and heating, is very particularly important. In particular in the case of applications in displays in mobile equipment, such as, for example, mobile telephones, this may be crucial.

Besides their relatively poor transmission and their relatively long response times the active-matrix displays disclosed hitherto have further disadvantages. These are e.g. their comparatively low contrast, their relatively high viewing-angle dependence and the difficulty in the reproduction of grey scales in these displays, especially when observed from an oblique viewing angle, as well as their inadequate VHR and their inadequate lifetime. The desired improvements of the transmission of the displays and of their response times are required in order to improve their energy efficiency, respectively their capacity to render rapidly moving pictures.

There thus continues to be a great demand for displays having very high specific resistance at the same time as a large working-temperature range, short response times and a relatively low threshold voltage, with the aid of which various grey shades can be produced and which have, in particular, a good and stable VHR.

The invention has the object of providing displays, not only for monitor and TV applications, but also for mobile applications such as e.g. telephones and navigation systems, which are based on the ECB, IPS or FFS effect, do not have the disadvantages indicated above, or only do so to a lesser extent, and at the same time have very high specific resistance values. In particular, it must be ensured for mobile telephones and navigation systems that they also work at extremely high and extremely low temperatures.

Surprisingly, it has been found that it is possible to achieve environmental friendly liquid-crystal displays which have, in particular in IPS and FFS displays, a low threshold voltage with short response times, a sufficiently broad nematic phase, favourable birefringence (Δn) and, at the same time, a high transmission, high contrast, good stability to decomposition by heating and by UV exposure, and a stable, high VHR if use is made in these display elements of nematic liquid-crystal media, which comprise at least one compound, preferably two or more compounds, of formula I, preferably additionally one or more compounds, preferably two or more compounds, selected from the group of the compounds of the formulae T, II and III, and/or one or more compounds, preferably two or more compounds selected from the group of formulae IV and/or V.

Media of this type can be used, in particular, for electro-optical displays having active-matrix addressing for IPS or FFS displays.

The contrast of an LC display can be improved, at one hand by a higher transmittance, which can be achieved by a higher ε_{⊥} in an FFS cell layout. And, on the other hand, it can be improved by a better dark state. The latter, i.e. the dark state, is strongly influenced, amongst others, by the scattering parameter. Here liquid crystalline media having high elastic constants (K¹¹, K³³) lower the scattering and therefore improve the contrast of an LCD. This also leads to their excellent performance in the displays according to the invention.

The media are distinguished by a particularly high transmission, a particularly good representation of the black state, primarily attributable to their very low scattering and reduced response time in respective displays, which is brought about by their unique combination of physical properties, especially by their high values of the elastic constant(s), in particular by high K₁₁, high K_{avg} and their excellent, low ratio (γ₁/K₁₁) of the rotational viscosity (γ₁) and the elastic constant (K₁₁). This also leads to their excellent performance in the displays according to the invention.

The media according to the present invention preferably additionally comprise a one or more compounds selected from the group of compounds of formulae T, II and III, preferably one or more compounds of formula II, more preferably in addition one or more compounds of formula III and, most preferably, additionally one or more compounds selected from the group of the compounds of formulae IV and V and, again preferably, one or more compounds selected from the group of compounds of formulae VI to IX (all formulae as defined below).

The mixtures according to the invention exhibit very broad nematic phase ranges with clearing points ≥ 70°C, very favourable values for the capacitive threshold, relatively high values for the holding ratio and at the same time good low-temperature stabilities at -20°C and -30°C, as well as very low rotational viscosities. The mixtures according to the invention are furthermore distinguished by a good ratio of clearing point and rotational viscosity and by a relatively high positive dielectric anisotropy.

The media of the current invention are characterised by a particular combination of physical properties. Most decisive amongst these are their high values of the elastic constant(s), in particular by their excellent, low ratio (γ₁/k₁₁) of the rotational viscosity (γ₁) and the elastic constant (k₁₁). Besides this, they show high ε_{⊥}, leading to a good transmission.

The first embodiment of the current invention is a liquid crystalline medium comprising
a) one or more compounds of formula I, preferably in a concentration in the range from 1 % to 40 %, more preferably in the range from 2 % to 30 %, particularly preferably in the range from 3 % to 20 %, 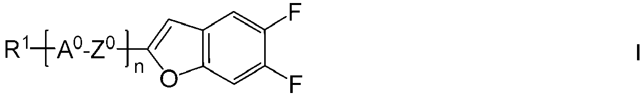 in which
   - R¹: denotes an alkyl radical having 1 to 15 C atoms, wherein one or more CH₂ groups, including terminal C atoms, in this radical may each be replaced, independently of one another, by -C=C-, -CH=CH-, 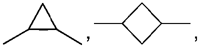 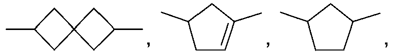 -O-, -S-, -(CO)-O-, -O-(CO)- in such a way that O or S atoms are not linked directly to one another, and in which, in addition, one or more H atoms may be replaced by F or CI,
   - A⁰,: independently of one another, on each appearance, denotes phenylene-1,4-diyl, in which, in addition, one or two CH groups may be replaced by N and one or more H atoms may be replaced by halogen, CN, CH₃, CHF₂, CH₂F, OCH₃, OCHF₂ or OCF₃, cyclohexane-1,4-diyl, in which, in addition, one or two non-adjacent CH₂ groups may be replaced, independently of one another, by O and/or S and one or more H atoms may be replaced by F, cyclohexene-1,4-diyl, bicyclo[1.1.1]pentane-1,3-diyl, bicyclo-[2.2.2]octane-1,4-diyl, spiro[3.3]heptane-2,6-diyl, tetrahydropyran-2,5-diyl or 1,3-dioxane-2,5-diyl,
   - A⁰: preferably denotes 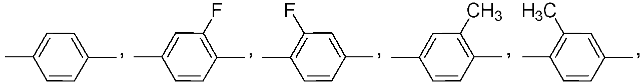 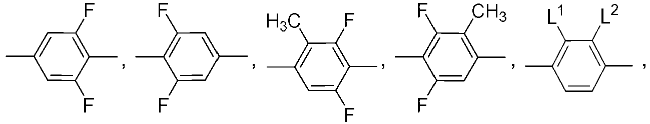 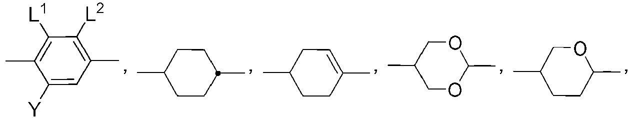 more preferably 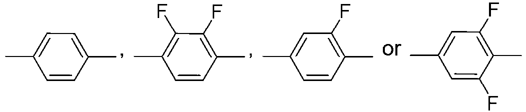 wherein
   - L¹, L²: each, independently of one another, denote F, Cl, CH₃ or OCH₃, preferably F, and
   - Y: denotes CH₃ or CH₂CH₃,
   - Z⁰: independently of one another, identically or differently, denotes a single bond, -CH₂O-, -(CO)O-, -CF₂O-, -CH₂CH₂CF₂O-, -CF₂CF₂-, -CH₂CF₂-, -CH₂CH₂-, -(CH₂)₄-, -OCH₂-, -OCF₂-, -CH=CH-, -CH=CF-, -CF=CF- or -C=C-, and
   - n: denotes 1, 2 or 3, preferably 1 or 2.

A further embodiment of this invention are compounds of formula I, their use in liquid crystalline media in liquid crystal displays, particularly in positive dielectric media (preferably IPS, FFS).

### Detailed description of the invention

The media according to the invention preferably further comprise one or more additional compounds selected from the groups of compounds according to the following conditions b) to f):
b) one or more, preferably dielectrically neutral, compounds of formula T 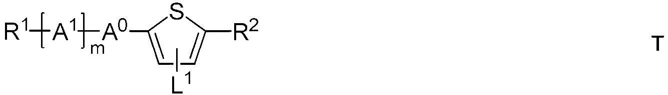 in which
   - R¹ and R²: denote H, F, Cl, Br, -CN or straight-chain or branched alkyl having 1 to 12 C atoms, in which, in addition, one or more CH₂ groups, including terminal C atoms, may each be replaced, independently of one another, by -CH=CH-, -C=C-, 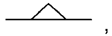 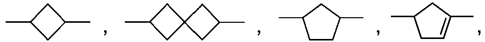 -O-, -S-, -CO-, -CO-O-, -O-CO-, in such a way that O or S atoms are not linked directly to one another, and in which, in addition, one or more H atoms may be replaced by F or CI,
   - L¹: denotes H, F or CH₃, preferably H,
   - A⁰: denotes 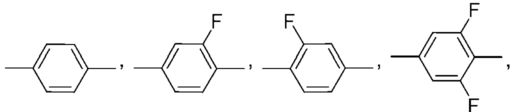 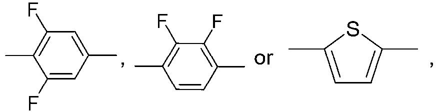 wherein the (fluoro)phenylene rings may optionally be substituted by one or two methyl and/or ethyl groups,
   - A¹,: independently of one another, denotes phenylene-1,4-diyl, in which, in addition, one or two CH groups may be replaced by N and one or more H atoms may be replaced by halogen, CN, CH₃, CHF₂, CH₂F, OCH₃, OCHF₂ or OCF₃, cyclohexane-1,4-diyl, in which, in addition, one or two non-adjacent CH₂ groups may be replaced, independently of one another, by O and/or S and one or more H atoms may be replaced by F, cyclohexene-1,4-diyl, bicyclo[1.1.1]pentane-1,3-diyl, bicyclo[2.2.2]octane-1,4-diyl, spiro[3.3]heptane-2,6-diyl, tetrahydropyran-2,5-diyl or 1,3-dioxane-2,5-diyl,
   - m: in each case, independently of one another, denotes 0, 1 or 2, preferably 1,
c) one or more, preferably dielectrically positive, compounds selected from the group of compounds of formulae II and III, preferably of compounds having a dielectric anisotropy of greater than 3 each, preferably one or more compounds of formula II: 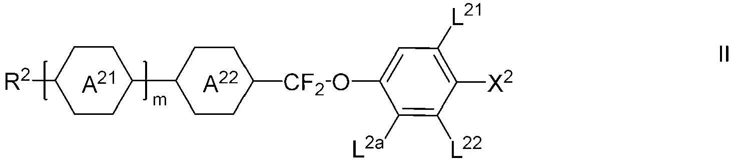 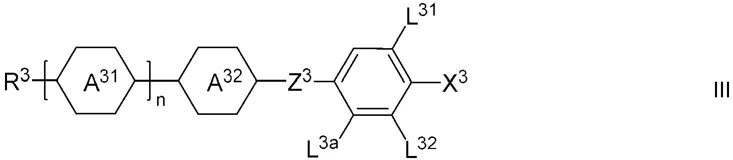 in which
   - R²: denotes an alkyl radical having 1 to 15 C atoms, wherein one or more CH₂ groups, including terminal C atoms, in this radical may each be replaced, independently of one another, by -C=C-, -CH=CH-, 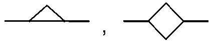 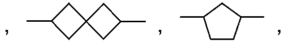 -O-, -S-, -(CO)-O-, -O-(CO)- in such a way that O or S atoms are not linked directly to one another, and in which, in addition, one or more H atoms may be replaced by F or Cl, or H, preferably denotes alkyl, alkoxy, fluorinated alkyl or fluorinated alkoxy having 1 to 7 C atoms, alkenyl, alkenyloxy, alkoxyalkyl, fluorinated alkenyl having 2 to 7 C atoms, cycloalkyl with 3 to 5 C atoms, cyclalkylalkyl, cycloalkylalkoxy and most preferably alkyl, cyclopropyl, cyclopentyl or alkenyl, 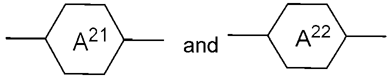 on each appearance, independently of one another, denote 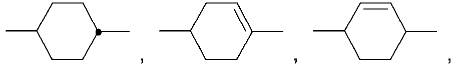 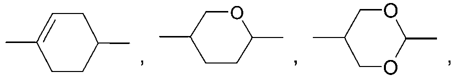 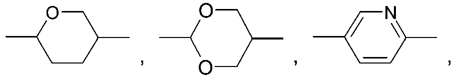 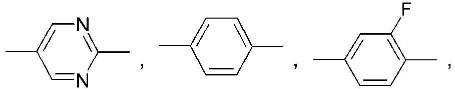 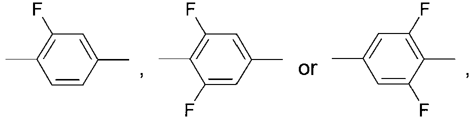 wherein the (fluoro)phenylene rings may optionally be substituted by one or two methyl and/or ethyl groups,
   preferably 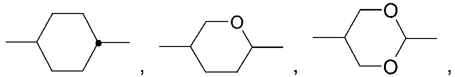 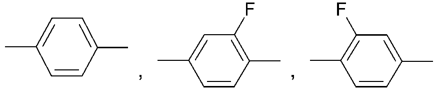 or 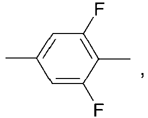
   - L²¹ and L²²: denote H or F,
   - L^{2a}: denotes H or CH₃, preferably H,
   - X²: denotes halogen, halogenated alkyl or alkoxy having 1 to 3 C atoms or halogenated alkenyl or alkenyloxy having 2 or 3 C atoms,
   preferably F, Cl, -OCF₃, -OCHF₂, -OCH₂F, -O-CH₂CF₃, -O-CH=CH₂, -O-CH=CF₂ or -CF₃, very preferably F, Cl, -O-CH=CF₂ or -OCF₃,
   - m: denotes 0, 1, 2 or 3, preferably 1 or 2 and particularly preferably 2,
   - R³: denotes an alkyl radical having 1 to 15 C atoms, wherein one or more CH₂ groups, including terminal C atoms, in this radical may each be replaced, independently of one another, by -C=C-, -CH=CH-, 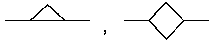 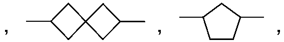 -O-, -S-, -(CO)-O-, -O-(CO)- in such a way that O or S atoms are not linked directly to one another, and in which, in addition, one or more H atoms may be replaced by F or Cl, or H, preferably denotes alkyl, alkoxy, fluorinated alkyl or fluorinated alkoxy having 1 to 7 C atoms, alkenyl, alkenyloxy, alkoxyalkyl, fluorinated alkenyl having 2 to 7 C atoms, cycloalkyl with 3 to 5 C atoms, cycloalkylalkyl, cycloalkylalkoxy and most preferably alkyl, cyclopropyl, cyclopentyl or alkenyl, 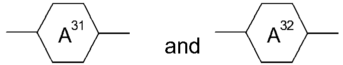 on each appearance, independently of one another, are 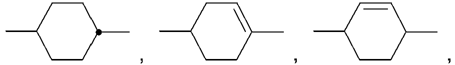 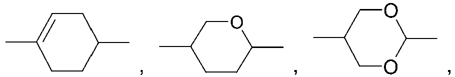 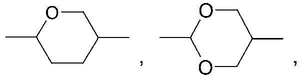 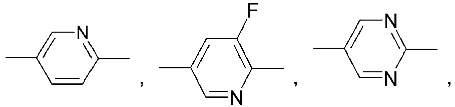 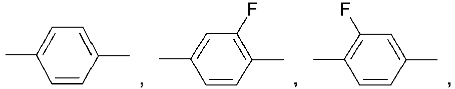 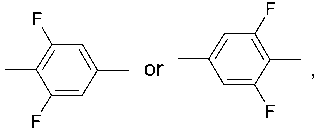 wherein the (fluoro)phenylene rings may optionally be substituted by one or two methyl and/or ethyl groups,
   preferably 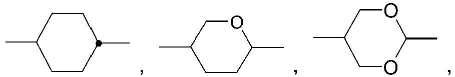 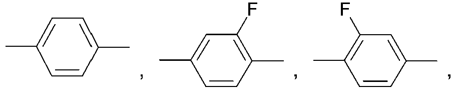 or 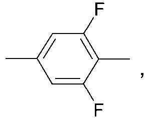
   - L³¹ and L³²,: independently of one another, denote H or F, preferably L³¹ denotes F,
   - L^{3a}: H or CH₃, preferably H,
   - X³: denotes halogen, halogenated alkyl or alkoxy having 1 to 3 C atoms or halogenated alkenyl or alkenyloxy having 2 or 3 C atoms, F, Cl, - OCF₃, -OCHF₂, -OCH₂F, -O-CH₂CF₃, -O-CH=CF₂, -O-CH=CH₂ or - CF₃,
   preferably F, Cl, -O-CH=CF₂, -OCHF₂, -OCH₂F or -OCF₃,
   - Z³: denotes -CH₂CH₂-, -CF₂CF₂-, -COO-, *trans*-CH=CH-,
   *trans*-CF=CF-, -CH₂O- or a single bond, preferably -CH₂CH₂-, -COO-, *trans*-CH=CH- or a single bond and very preferably -COO-, *trans-*CH=CH- or a single bond, and
   - n: denotes 0, 1, 2 or 3, preferably 1, 2 or 3 and particularly preferably 1,
d) one or more dielectrically neutral compounds selected from the group of formulae IV and V: 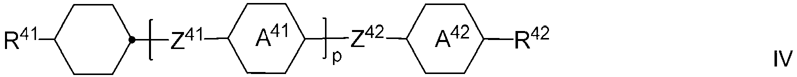 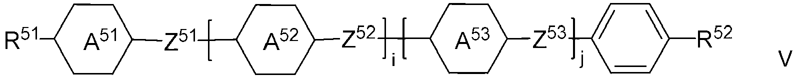 in which
   - R⁴¹ and R⁴²,: independently of one another, have the meaning indicated above for R³ under formula III, preferably R⁴¹ denotes alkyl and R⁴² denotes alkyl, cyclopropyl, cyclopentyl or alkoxy or R⁴¹ denotes alkenyl and R⁴² denotes alkyl, 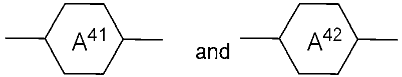 on each occurrence, independently of one another, denote 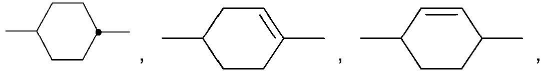 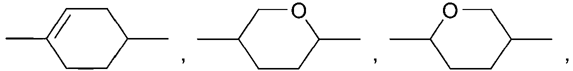 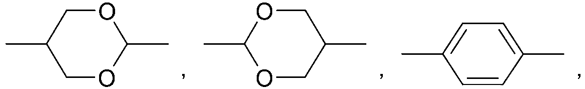 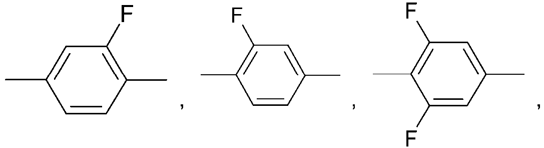 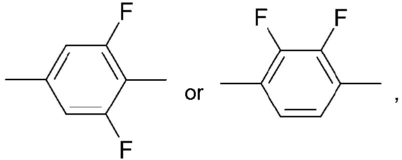 wherein the (fluoro)phenylene rings may optionally be substituted by one or two methyl and/or ethyl groups, preferably one or more of 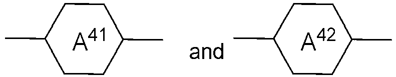 denotes or denote, 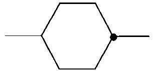
   - Z⁴¹ and Z⁴²,: independently of one another and, if Z⁴¹ occurs twice, also these independently of one another, denote -CH₂CH₂-, -COO-, *trans-*CH=CH-, *trans*-CF=CF-, -CH₂O-, -CF₂O-, -C=Cor a single bond, preferably one or more thereof denotes/denote a single bond,
   - p: denotes 0, 1 or 2, preferably 0 or 1, and
   - R⁵¹ and R⁵²,: independently of one another, have one of the meanings given for R⁴¹ and R⁴² and preferably denote alkyl having 1 to 7 C atoms, preferably *n*-alkyl, particularly preferably *n*-alkyl having 1 to 5 C atoms, alkoxy having 1 to 7 C atoms, preferably *n*-alkoxy, particularly preferably *n*-alkoxy having 2 to 5 C atoms, alkoxyalkyl, alkenyl or alkenyloxy having 2 to 7 C atoms, preferably having 2 to 4 C atoms, preferably alkenyloxy, 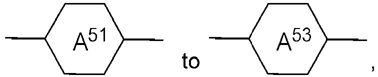 on each appearance, independently of one another, denote 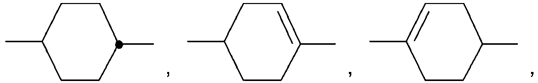 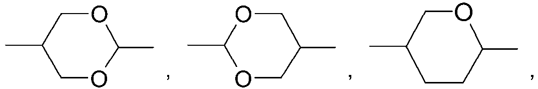 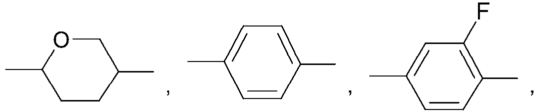 or 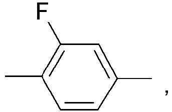 wherein the (fluoro)phenylene rings may optionally be substituted by one or two methyl and/or ethyl groups,
   preferably 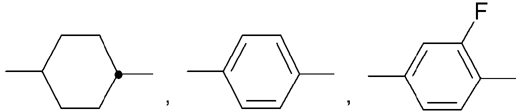 or 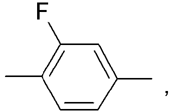
   - Z⁵¹ to Z⁵³: each, independently of one another, denote -CH₂-CH₂-, -CH₂-O-, -CH=CH-, -C=C-, -COO- or a single bond, preferably - CH₂-CH₂-, -CH₂-O- or a single bond and particularly preferably a single bond,
   - i and j: each, independently of one another, denote 0 or 1,
   - (i + j): preferably denotes 0, 1 or 2, more preferably 0 or 1,
   and
e) one or more dielectrically negative compounds selected from the group of formulae VI to IX: 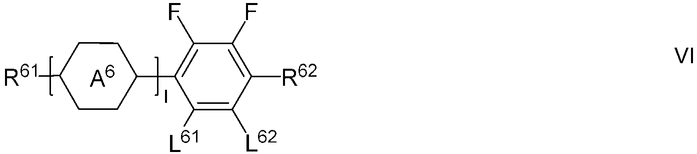 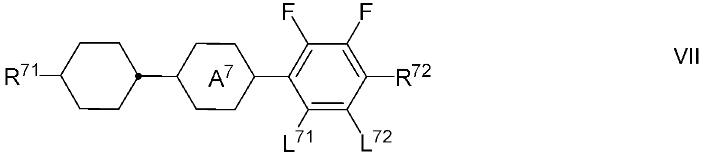 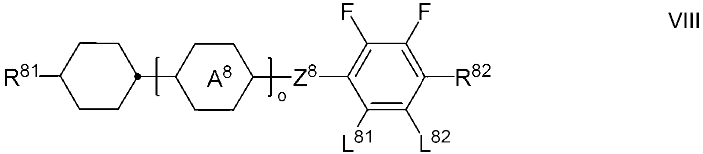 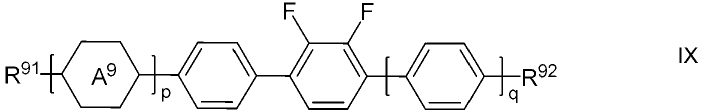 wherein
   - R⁶¹: denotes an unsubstituted alkyl radical having 1 to 7 C atoms, preferably a straight-chain alkyl radical, more preferably an n-alkyl radical, most preferably propyl or pentyl, an unsubstituted alkenyl radical having 2 to 7 C atoms, preferably a straight-chain alkenyl radical, particularly preferably having 2 to 5 C atoms, an unsubstituted alkoxy radical having 1 to 6 C atoms, an unsubstituted alkenyloxy radical having 2 to 6 C atoms or C₃₋₅-cycloalkyl-(CH₂)₀₋₁,
   - R⁶²: denotes an unsubstituted alkyl radical having 1 to 7 C atoms, an unsubstituted alkoxy radical having 1 to 6 C atoms, C₃₋₅-cycloalkyloxy or an unsubstituted alkenyloxy radical having 2 to 6 C atoms, and
   - L⁶¹, L⁶²: independently H or methyl, preferably H,
   - I: denotes 0 or 1,
   - R⁷¹: denotes an unsubstituted alkyl radical having 1 to 7 C atoms, preferably a straight-chain alkyl radical, more preferably an *n*-alkyl radical, most preferably propyl or pentyl, an unsubstituted alkenyl radical having 2 to 7 C atoms, preferably a straight-chain alkenyl radical, particularly preferably having 2 to 5 C atoms or C₃₋₅-cycloalkyl-(CH₂)₀₋₁,
   - R⁷²: denotes an unsubstituted alkyl radical having 1 to 7 C atoms, preferably having 2 to 5 C atoms, an unsubstituted alkoxy radical having 1 to 6 C atoms, preferably having 1, 2, 3 or 4 C atoms, or an unsubstituted alkenyloxy radical having 2 to 6 C atoms, preferably having 2, 3 or 4 C atoms, and
   - L⁷¹, L⁷²: independently H or methyl, preferably H, 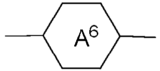 and 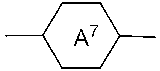 independently denote 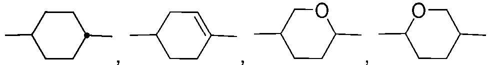 or 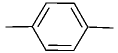
   - R⁸¹: denotes an unsubstituted alkyl radical having 1 to 7 C atoms, preferably a straight-chain alkyl radical, more preferably an *n*-alkyl radical, most preferably propyl or pentyl, an unsubstituted alkenyl radical having 2 to 7 C atoms, preferably a straight-chain alkenyl radical, particularly preferably having 2 to 5 C atoms or C₃₋₅-cycloalkyl-(CH₂)₀₋₁,
   - R⁸²: denotes an unsubstituted alkyl radical having 1 to 7 C atoms, preferably having 2 to 5 C atoms, an unsubstituted alkoxy radical having 1 to 6 C atoms, preferably having 1, 2, 3 or 4 C atoms, an unsubstituted alkenyloxy radical having 2 to 6 C atoms, preferably having 2, 3 or 4 C atoms, or C₃₋₅-cycloalkyloxy,
   - L⁸¹, L⁸²: independently H or methyl, preferably H, 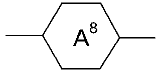 denotes 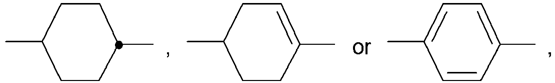 preferably 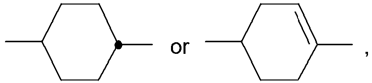 more preferably 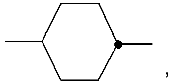
   - Z⁸: denotes -(C=O)-O-, -CH₂-O-, -CF₂-O- or -CH₂-CH₂-, preferably -(C=O)-O- or -CH₂-O-, and
   - o: denotes 0 or 1,
   - R⁹¹ and R⁹²: independently of one another have the meaning given for R⁷² above,
   - R⁹¹: preferably denotes an alkyl radical having 2 to 5 C atoms, preferably having 3 to 5 C atoms,
   - R⁹²: preferably denotes an alkyl or alkoxy radical having 2 to 5 C atoms, more preferably an alkoxy radical having 2 to 4 C atoms, or an alkenyloxy radical having 2 to 4 C atoms. 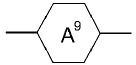 denotes 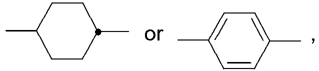
   - p and q: independently of each other denote 0 or 1, and
   - (p + q): preferably denotes 0 or 1, in case 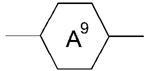 denotes 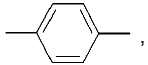 preferably p = q = 1,
f) one or more compounds of formula X, preferably in a concentration in the range from 1 % to 30 %, more preferably in the range from 2 % to 20 %, particularly preferably in the range from 3 % to 10 %, 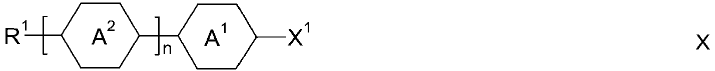 in which 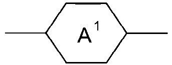 denotes 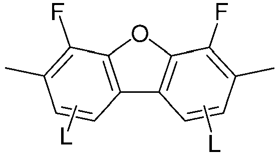 or 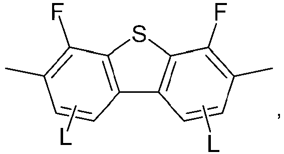
   - L: independently denotes H or CH₃ or CH₂CH₃, preferably H, 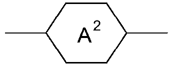 denotes, in each occurrence independently of one another, 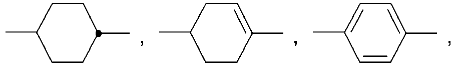 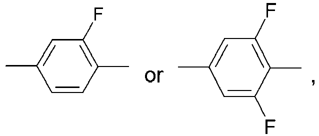
   - preferably: 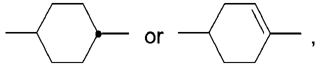
   - n: denotes 1 or 2, preferably 1,
   - R¹: denotes alkyl, alkoxy, fluorinated alkyl or fluorinated alkoxy, preferably having 1 to 7 C atoms, alkenyl, alkenyloxy, alkoxyalkyl or fluorinated alkenyl having 2 to 7 C atoms, C₃₋₅-cycloalkyl, C₃₋₅-cycloalkyl-alkyl, C₃₋₅-cycloalkyl-alkyloxy, preferably alkyl, alkoxy, alkenyl or alkenyloxy, more preferably alkyl, alkenyl, alkoxy or alkenyloxy, and, most preferably alkyl, and
   - X¹: denotes F, Cl, fluorinated alkyl, fluorinated alkenyl, fluorinated alkoxy or fluorinated alkenyloxy, the latter four groups preferably having 1 to 4 C atoms,
   preferably F, Cl, CF₃ or OCF₃,
   wherein the respective rings, and preferably the phenylene rings, optionally may each be substituted by one or two alkyl groups, preferably by methyl and/or ethyl groups, preferably by one methyl group, and
g) again optionally, preferably obligatory, either alternatively or additionally, one or more compounds of formula XI: 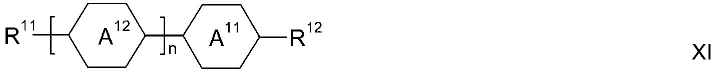 in which 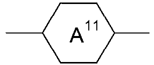 denotes 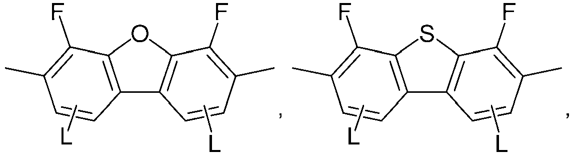 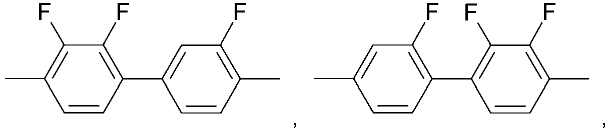 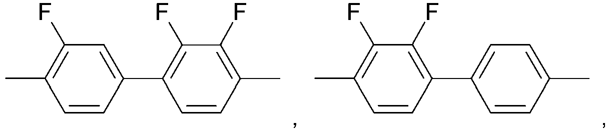 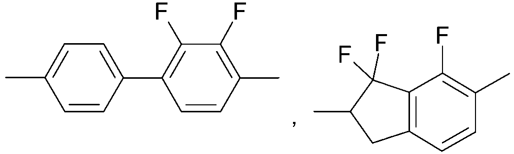 or 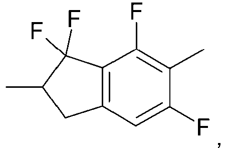
   - L: independently denotes H or CH₃ or CH₂CH₃, preferably H, 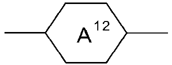 denotes 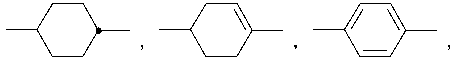 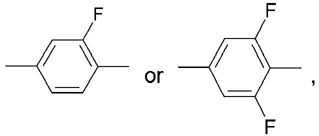 preferably 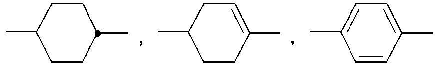 or 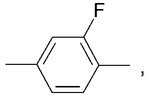
   - n: denotes 0 or 1,
   - R¹¹ and R¹²: independently of each other denote alkyl, alkoxy, fluorinated alkyl or fluorinated alkoxy, preferably having 1 to 7 C atoms, wherein one CH₂ group may be replaced by a 1,2-cyclopropyl group, by a 1,3-cyclopentyl group or by a 1,3-cyclopentenylene group, alkenyl, alkenyloxy, alkoxyalkyl or fluorinated alkenyl having 2 to 7 C atoms and preferably alkyl, alkoxy, alkenyl or alkenyloxy, most preferably alkyl, alkoxy or alkenyloxy,

   wherein the respective rings, and preferably the phenylene rings, optionally may each be substituted by one or two alkyl groups, preferably by methyl and/or ethyl groups, preferably by one methyl group,
   and from which the compounds of formula X are excluded.

The compounds of formula I are distinguished by a surprisingly large positive dielectric anisotropy (Δε) and are therefore suitable, in particular, for use in TN-TFT displays, and in IPS- and FFS displays. The compounds have a comparatively low melting point, a very high clearing point and they exhibit very good compatibility with the conventional substances used in liquid-crystal mixtures for displays and are well soluble in such media. The compounds do not have any perfluoroalkyl groups in their structures, making them innocuous towards the environmental aspect of accumulation of perfluoroalkyl structured substances.

The compounds according to the invention preferably have a Δε of > 9, preferably of >15 and particularly preferably a Δε of >17.

Optionally the media according to the present application comprise one or more compounds of formula L, preferably in a concentration in the range from 1 % to 40 %, more preferably in the range from 2 % to 30 %, particularly preferably in the range from 3 % to 20 %, 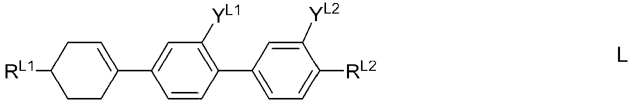 in which
R^{L1} and R ^{L2}, independently of one another, denote, an alkyl radical having 1 to 15 C atoms, wherein one or more CH₂ groups, preferably one CH₂ group, in these radicals may each be replaced, independently of one another, by -C=C-, -CF₂O-, -OCF₂-, -O-, -(CO)-O-, -O-(C=O)-, cyclo-propylene, 1,3-cyclobutylene, 1,3-cyclopentylene, 1,3-cyclo-pentenylene in such a way that O atoms are not linked directly to one another, preferably by cyclopropylene or 1,3-cyclopentylene,
   an alkenyl, alkenyloxy, alkoxyalkyl or fluorinated alkenyl having 2 to 7 C atoms, and in which one or more H atoms may be replaced by halogen, and
Y^{L1} and Y^{L2}, identically or differently, denote H, F or Cl, preferably at least one of Y^{L1} and Y^{L2} is H, preferably Y^{L2} is H, and most preferably Y^{L1} and Y^{L2} are H.

The liquid-crystalline media in accordance with the present application preferably have a nematic phase.

Throughout this application and especially for the definition of R¹, R², R³, R⁴¹, R⁵¹, R⁶¹, R⁷¹, R⁸¹, R⁹¹, R^{L1}, etc. and R^{L2} alkyl means an alkyl group, which may be straight-chain or branched. Each of these radicals is preferably straight-chain and preferably has 1, 2, 3, 4, 5, 6, 7 or 8 C atoms and is accordingly preferably methyl, ethyl, *n*-propyl, *n*-butyl, *n*-pentyl, *n*-hexyl or *n*-heptyl.

Throughout this application alkenyl means an alkenyl group, which may be straight-chain or branched and preferably is straight chain and preferably has 2, 3, 4, 5, 6 or 7 or 8 C atoms. Preferably it is vinyl, 1-*E*-alkenyl or 3-*E*-alkenyl, most preferably it is vinyl, 1-*E*-propenyl, 1-*E*-butenyl, 1-*E*-pentenyl, 3-butenyl or 3-*E*-pentenyl.

The compounds of the general formula I, L, T and II to XI are prepared by methods known per se, as described in the literature (for example in the standard works, such as Houben-Weyl, Methoden der organischen Chemie [Methods of Organic Chemistry], Georg-Thieme-Verlag, Stuttgart), to be precise under reaction conditions which are known and are suitable for the said reactions. Use can be made here of variants which are known per se, but are not mentioned here in greater detail.

The compounds of formula I may be synthesized as in the synthesis examples or in accordance with the following reaction scheme:

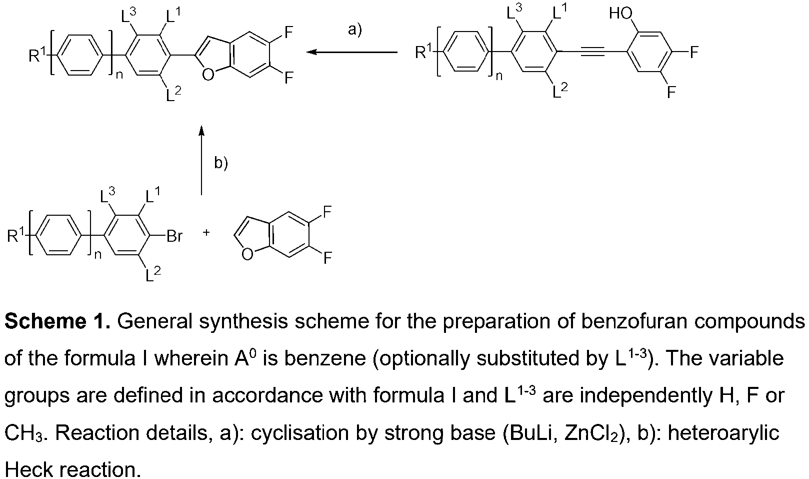

The invention furthermore relates to a liquid-crystal display containing a liquid-crystalline medium according to the invention, in particular an IPS or FFS display, particularly preferably a FFS or SG-FFS display.

The displays in accordance with the present invention are preferably addressed by an active matrix (active matrix LCDs, AMDs for short), preferably by a matrix of thin-film transistors (TFTs). However, the liquid crystals according to the invention can also be used in an advantageous manner in displays having other known addressing means.

The invention furthermore relates to a process for the preparation of a liquid-crystalline medium according to the invention by mixing one or more compounds of formula I or its subformulae with one or more low-molecular-weight liquid-crystalline compounds, or a liquid-crystal mixture and optionally with further liquid-crystalline compounds and/or additives.

The following meanings apply above and below:
Particular preference is given to liquid-crystal mixtures and liquid-crystalline media which have a nematic phase, in particular at room temperature (20 °C).

In a preferred embodiment of the present invention, the liquid-crystal medium comprises one or more compounds selected from the group of the compounds of the formulae I-a and I-b: 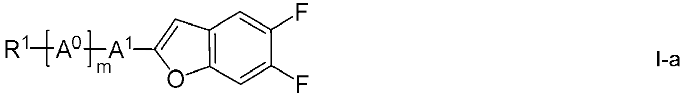 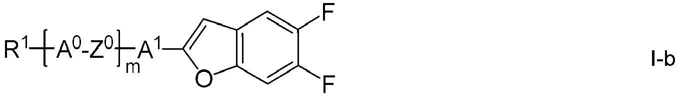
wherein the variables A⁰ and A¹ are independently defined as group A⁰ in formula I above and below, and
m is 0, 1 or 2.

Particular preference is given here to compounds of formula I in which n is 1 or 2, preferably 2, and to compounds of formula I-a/I-b wherein m is 0 or 1, preferably 1. The group A¹ preferably denotes phenylene, which is optionally fluorinated, particularly preferably

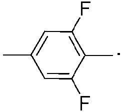

The group A⁰ in the formulae I and I-a/I-b preferably denotes

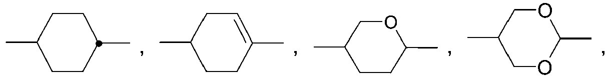

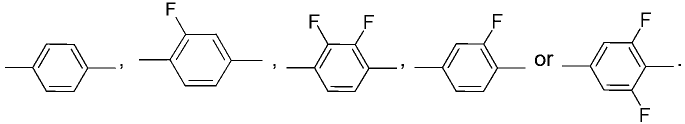

Preferred are compounds of formula I-a wherein A⁰ denotes

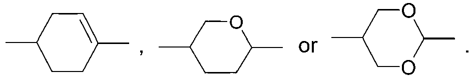

In the formulae I and in the related more specific formulae above and below compounds are preferred, wherein independently:
n is 1 or 2, or in formulae I-a and I-b respectively m is 0 or 1,
R¹ is an alkyl radical having 1 to 7 C atoms, wherein one or more CH₂ groups, including terminal C atoms, in this radical may each be replaced, independently of one another, by -C=C-, -CH=CH-, 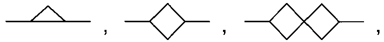 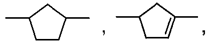 or -O- in such a way that O atoms are not linked directly to one another, and in which, in addition, one or more H atoms may be replaced by F, more preferably an alkyl radical having 2 to 7 C atoms.

In a preferred embodiment of the present invention the liquid crystalline medium comprises one or more compounds selected from the group of the compounds of the following formulae: 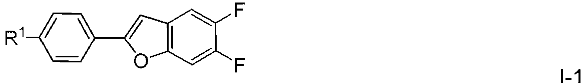 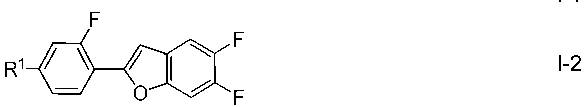 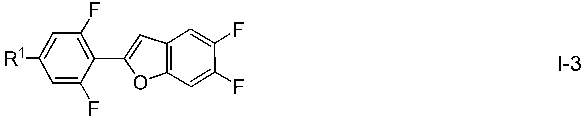 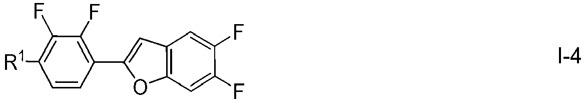 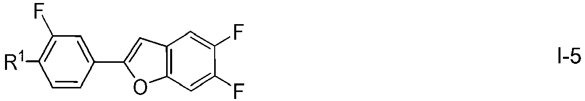 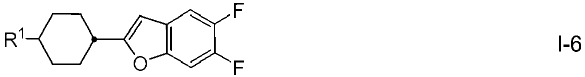 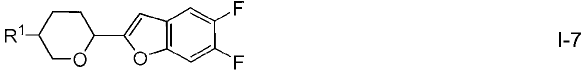 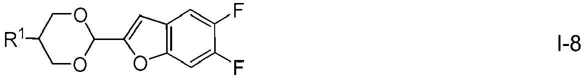 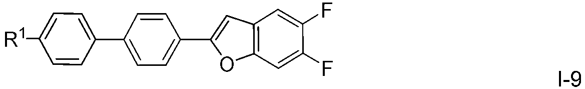 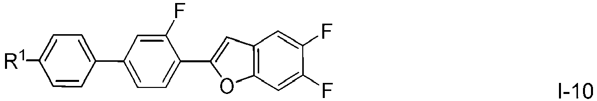 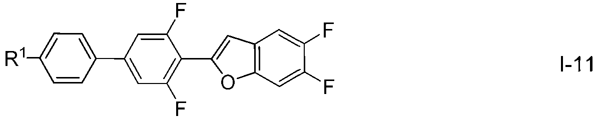 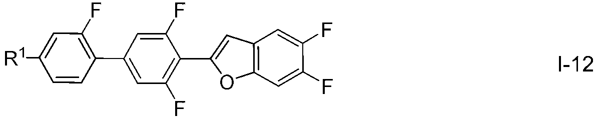 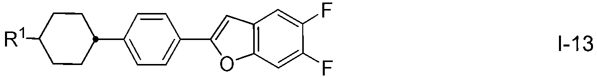 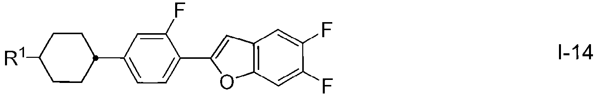 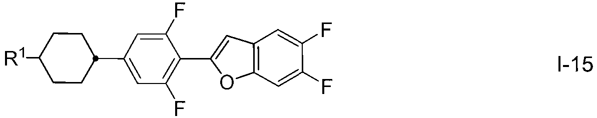 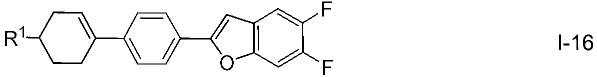 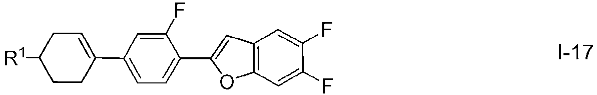 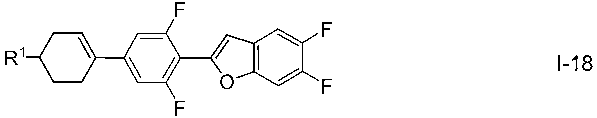 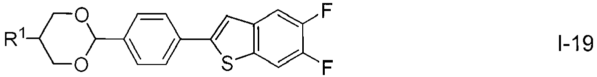 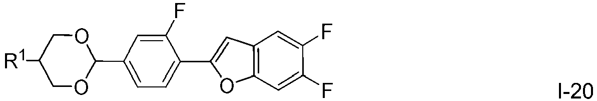 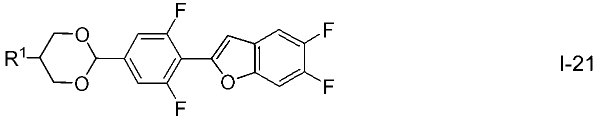 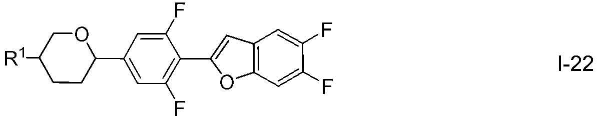 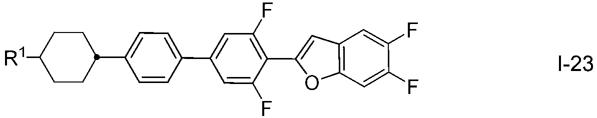 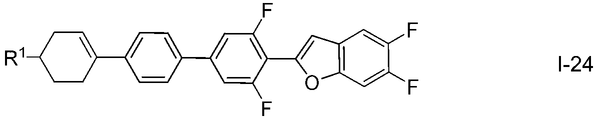 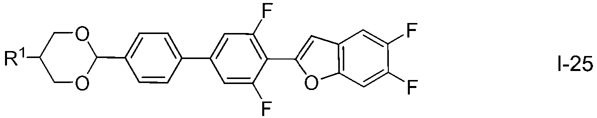 wherein R¹, X¹ are defined as for formula I above and below, including preferred definitions. Compounds of formula I-10 and I-11 are especially preferred.

Preferred compounds of formula T are selected from compounds of formula 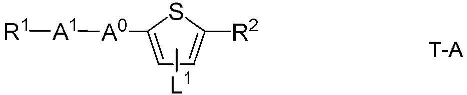 wherein the groups are defined as above, and L¹ is preferably H.

In the formula T and its more defined formulae above and below the group R¹ preferably is straight-chain or branched alkyl having 1 to 12 C atoms, in which, in addition, one or more CH₂ groups, including terminal C atoms, may each be replaced, independently of one another, by -CH=CH-, -C=C-, 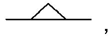 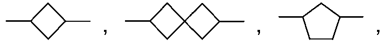 -O-, -S-, -CO-, -CO-O-, -O-CO-, in such a way that O or S atoms are not linked directly to one another, and in which, in addition, one or more H atoms may be replaced by F or Cl, more preferably alkyl with 1 to 7 C atoms or alkenyl with 2 to 7 C atoms, cyclopentylmethyl or cyclopentyl.

In the formula T and its more defined formulae above and below the group R² preferably is straight-chain or branched alkyl having 1 to 12 C atoms, in which, in addition, one or more CH₂ groups, including terminal C atoms, may each be replaced, independently of one another, by -CH=CH-, -C≡C-, 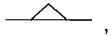 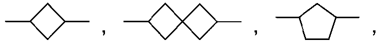 -O-, -S-, -CO-, -CO-O-, -O-CO-, in such a way that O or S atoms are not linked directly to one another, a group -CF₃, -OCF₃ or F, more preferably methyl, ethyl, n-propyl, n-butyl, CF₃ or F, most preferably methyl, ethyl or propyl.

In a preferred embodiment of the present invention the liquid crystalline medium comprises one or more compounds selected from the group of the compounds of the formulae T-1 to T-4: 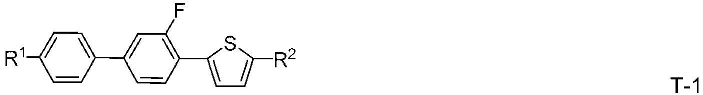 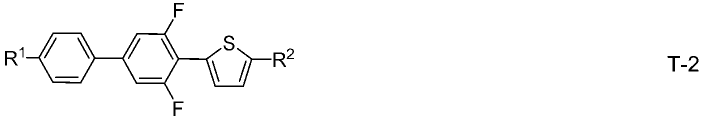 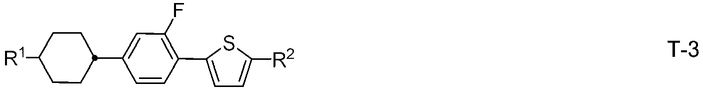 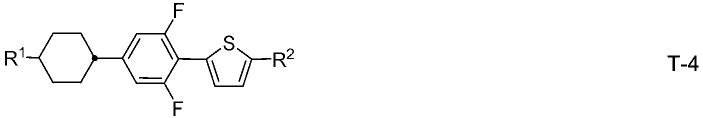
wherein R² is CH₃, CH₂CH₃, CF₃ or F, preferably CH₃ or CF₃, and
R¹ is defined as in formula T above, preferably R¹ denotes an alkyl radical having 1 to 7 C atoms, wherein one or more CH₂ groups, including terminal C atoms, in this radical may each be replaced, independently of one another, by -C=C-, -CH=CH-, 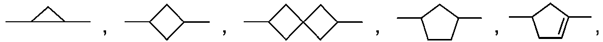 -O-, -S-, -(CO)-O-, - O-(CO)- in such a way that O or S atoms are not linked directly to one another, and in which, in addition, one or more H atoms may be replaced by F,
preferably denotes alkyl, alkoxy, fluorinated alkyl or fluorinated alkoxy having 1 to 7 C atoms, alkenyl, alkenyloxy, alkoxyalkyl, fluorinated alkenyl having 2 to 7 C atoms, cycloalkyl with 3 to 5 C atoms, cycloalkylalkyl, cycloalkylalkoxy and most preferably alkyl, cyclopropyl, cyclopropylmethyl, cyclopentyl, cyclopentylmethyl or alkenyl.

In a preferred embodiment of the present invention, the liquid-crystal medium comprises one or more, preferably dielectrically positive, compounds, preferably having a dielectric anisotropy of greater than 3, selected from the group of the compounds of the formulae II-1 and II-2: 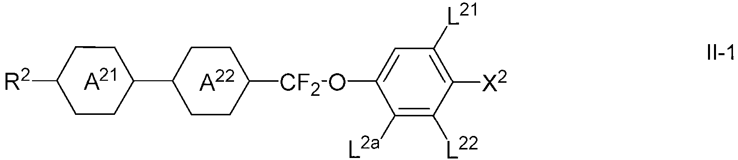 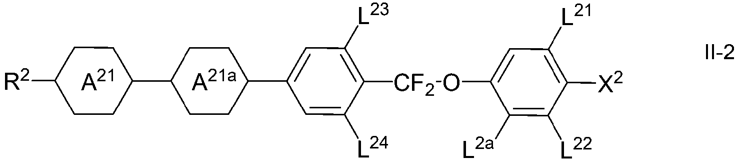
in which the parameters have the respective meanings indicated above under formula II, and L²³ and L²⁴, independently of one another, denote H or F, preferably L²³ denotes F, and 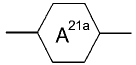
has one of the meanings given for 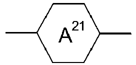 and, in the case of formulae II-1 and II-2, X² preferably denotes F or OCF₃, particularly preferably F, and, in the case of formula II-2, 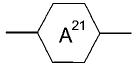 and 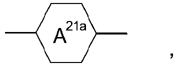 independently of one another, preferably denote 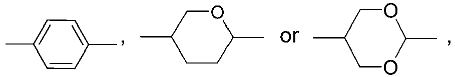 and/or selected from the group of the compounds of the formulae III-1 and III-2: 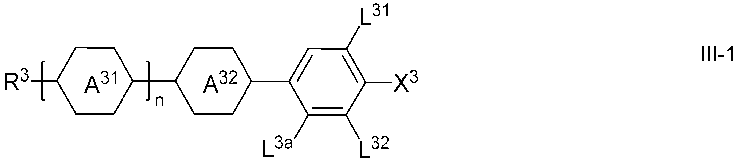 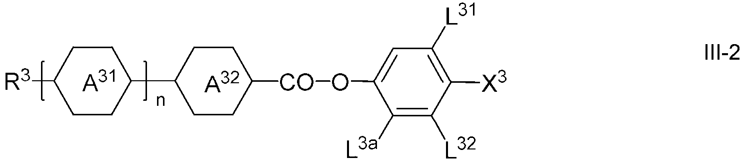
in which the parameters have the meanings given under formula III,
and the media in accordance with the present invention may comprise, alternatively or in addition to the compounds of the formulae III-1 and/or III-2, one or more compounds of the formula III-3 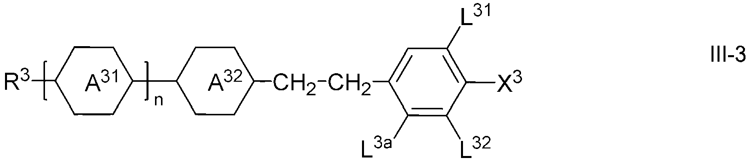
in which the parameters have the respective meanings indicated above, and the parameters L³¹ and L³², independently of one another and of the other parameters, denote H or F.

The liquid-crystal medium preferably comprises compounds selected from the group of the compounds of the formulae II-1 and II-2 in which L²¹ and L²² and/or L²⁴ and L²⁵ both denote F.

In a preferred embodiment, the liquid-crystal medium comprises compounds selected from the group of the compounds of the formulae II-2 in which L²¹, L²², L²⁴ and L²⁵ all denote F.

The liquid-crystal medium preferably comprises one or more compounds of the formula II-1. The compounds of the formula II-1 are preferably selected from the group of the compounds of the formulae II-1a to II-1e, preferably one or more compounds of formulaeII-1a and/or II-1b and/or II-1d, preferably of formula II-1a and/or II-1e or II-1c and/or II-1e, most preferably of formula II-1e: 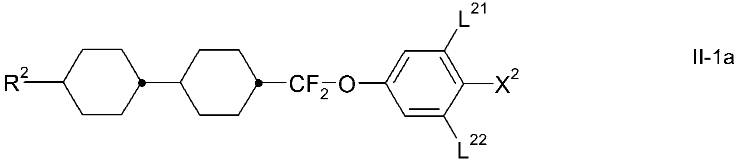 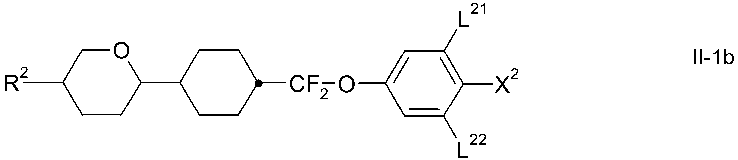 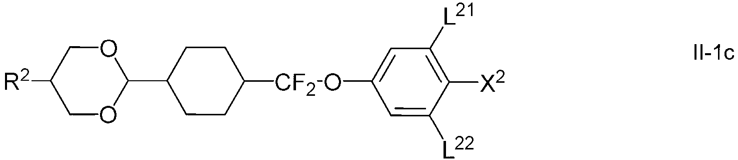 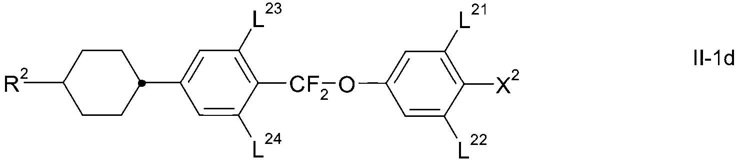 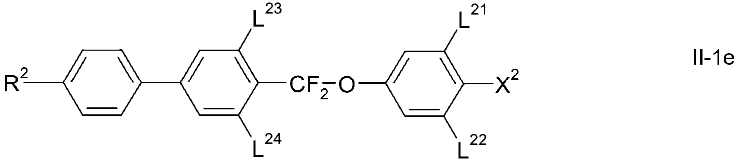 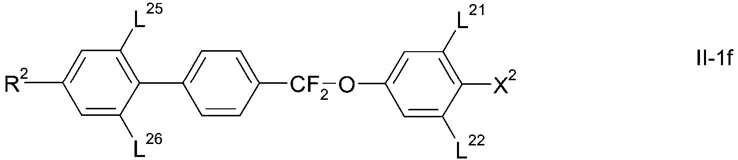
in which the parameters have the respective meanings indicated above, and L²⁵ and L²⁶, independently of one another and of the other parameters, denote H or F, and preferably
in the formulae II-1a and II-1b,
L²¹ and L²² both denote F,
in the formulae II-1d and II-1e,
L²¹ and L²² both denote F and/or L²³ and L²⁴ both denote F, and
in formula II-1f,
L²¹ and L²² denote F,
and wherein the respective rings, and preferably the phenylene rings, optionally may each be substituted by one or two alkyl groups, preferably by methyl and/or ethyl groups, preferably by one methyl group.

The liquid-crystal medium preferably comprises one or more compounds of the formula II-2, which are preferably selected from the group of the compounds of the formulae II-2a to II-2k, preferably one or more compounds of formulae II-2a and/or II-2h and/or II-2j and/or II-2k: 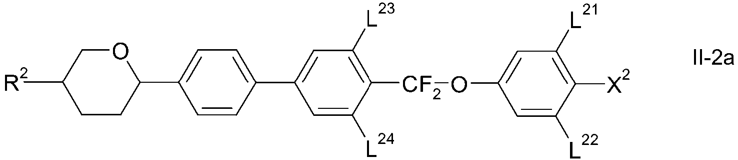 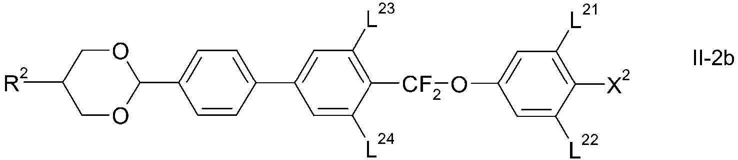 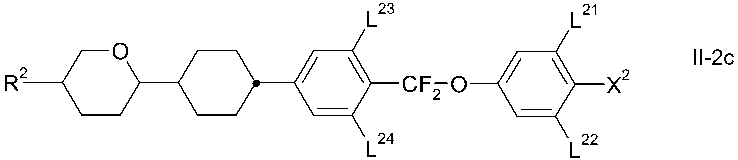 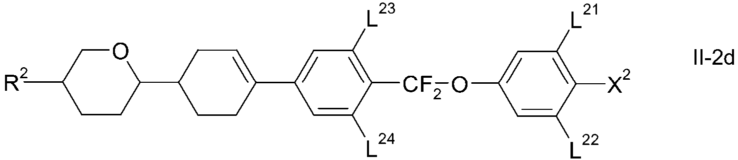 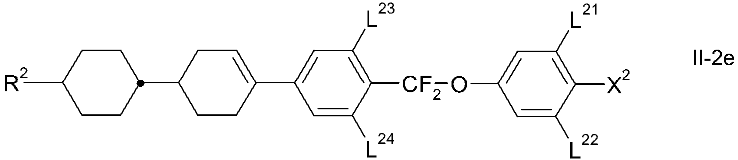 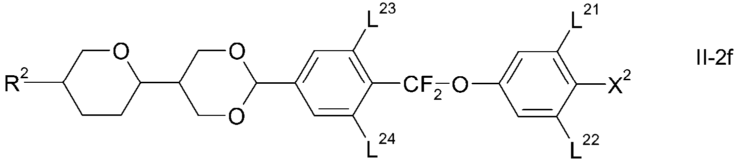 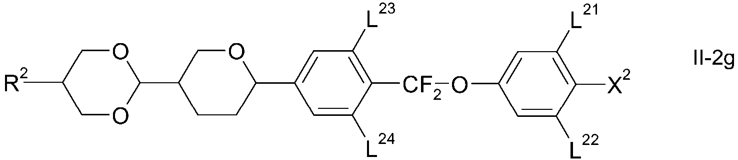 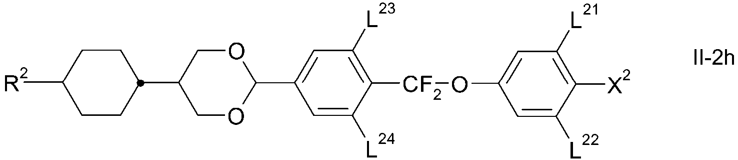 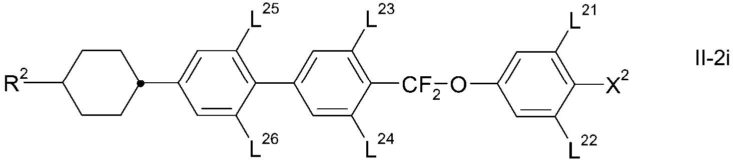 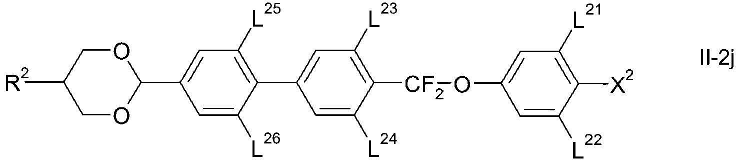 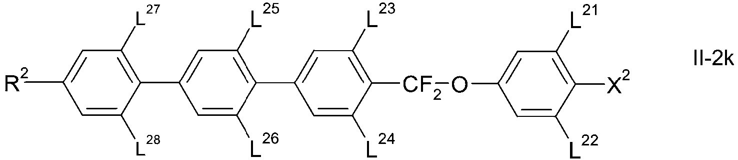 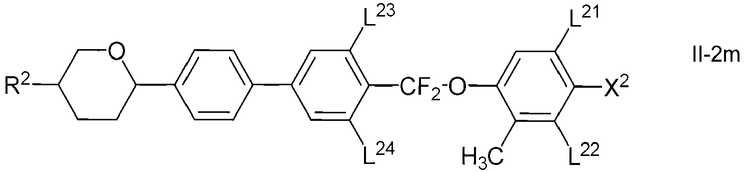 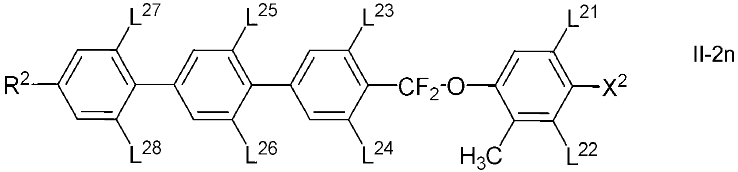 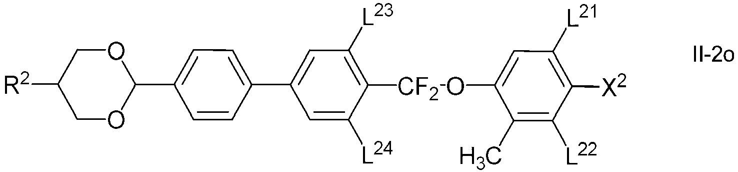 in which the parameters have the respective meanings indicated above, and L²⁵ to L²³, independently of one another, denote H or F, preferably L²⁷ and L²⁸ both denote H, particularly preferably L²⁶ denotes H.

The liquid-crystal medium preferably comprises compounds selected from the group of the compounds of the formulae II-2a to II-2k in which L²¹ and L²² both denote F and/or L²³ and L²⁴ both denote F.

In a preferred embodiment, the liquid-crystal medium comprises compounds selected from the group of the compounds of the formulae II-2a to II-2k in which L²¹, L²², L²³ and L²⁴ all denote F.

Especially preferred compounds of the formula II-2 are the compounds of the following formulae, particularly preferred of formulae II-2a-1 and/or II-2h-1 and/or II-2k-2: 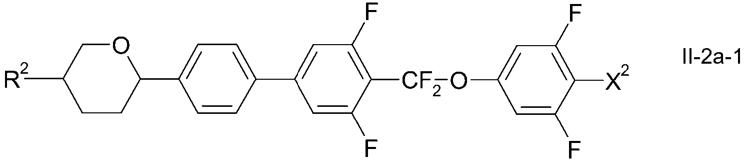 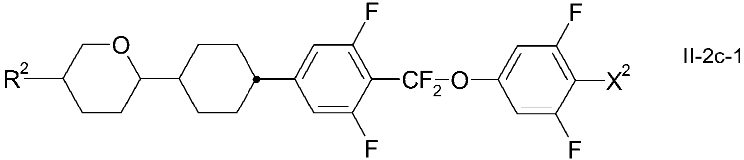 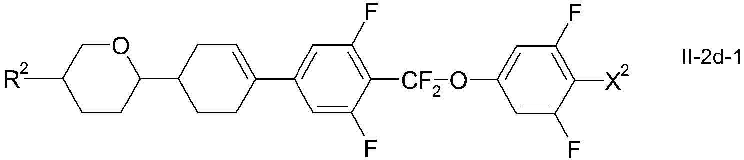 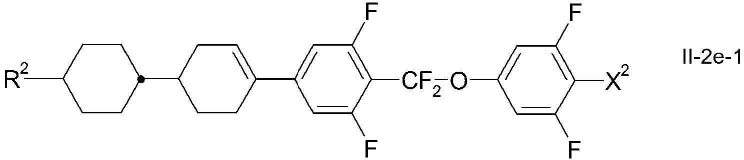 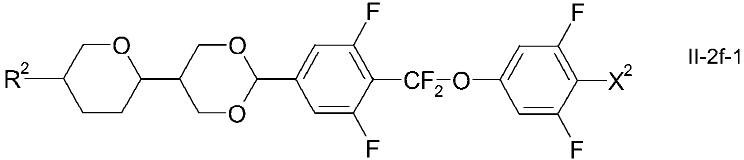 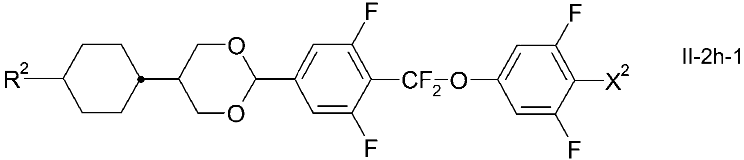 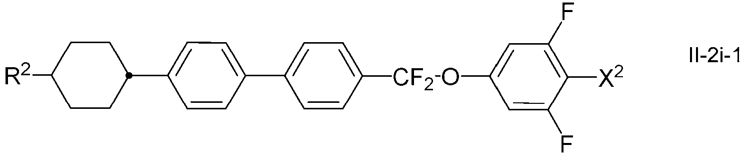 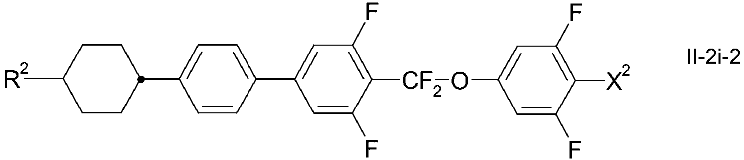 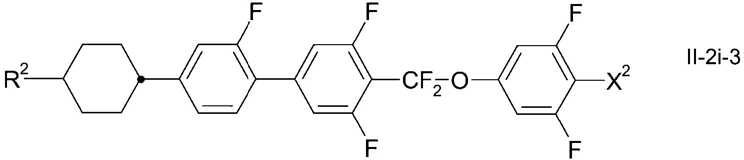 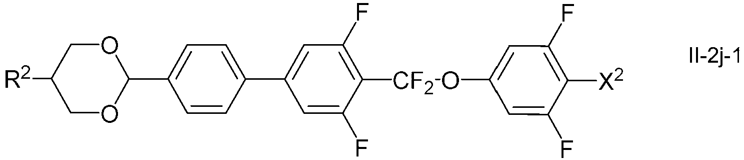 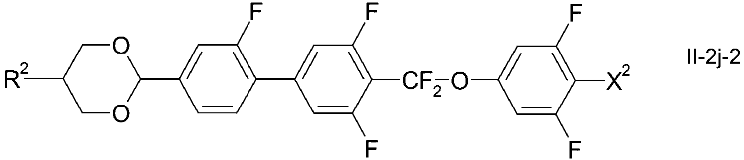 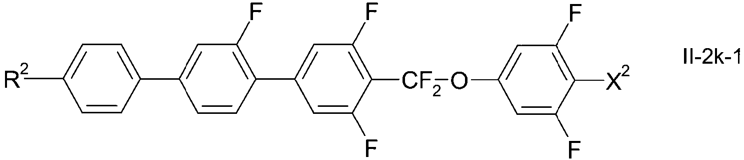 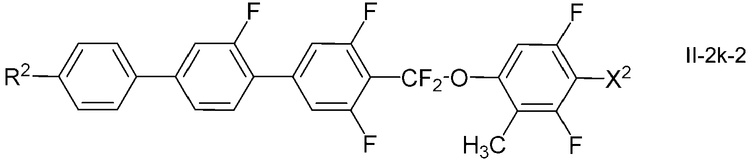 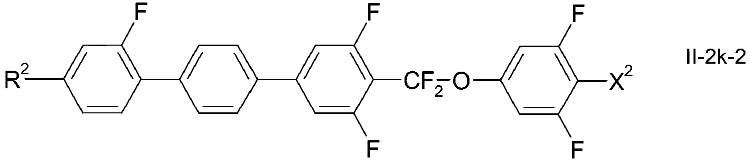 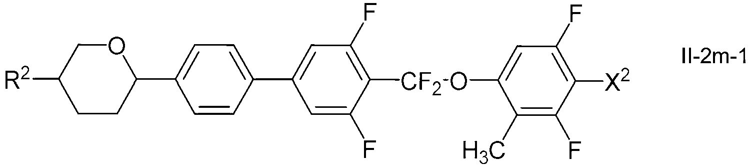 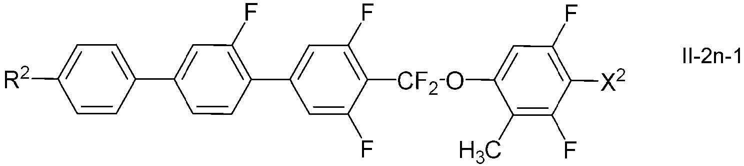 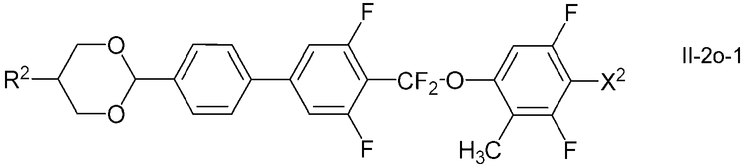 in which R² and X² have the meanings indicated above, and X² preferably denotes F.

The liquid-crystal medium preferably comprises one or more compounds of the formula III-1. The compounds of the formula III-1 are preferably selected from the group of the compounds of the formulae III-1a to III-1j, preferably from formulae III-1d, III-1e, III-1g, III-1h, III-1j, III-1k, III-1m and III-1n, more preferably selected from formulae III-1j, III-1k, III-1m and III-1n: 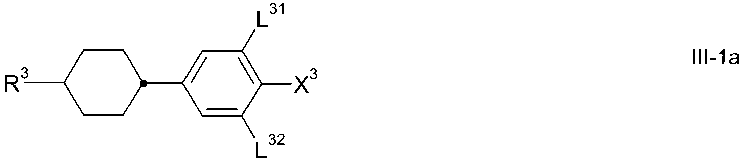 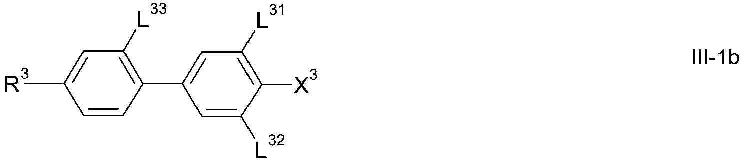 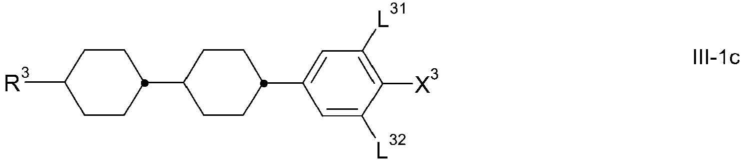 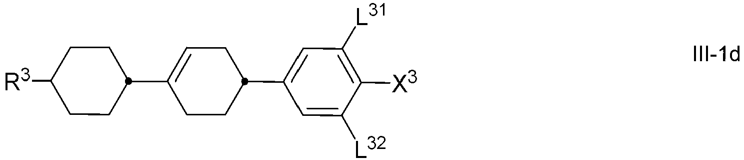 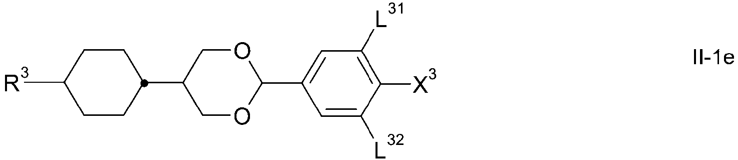 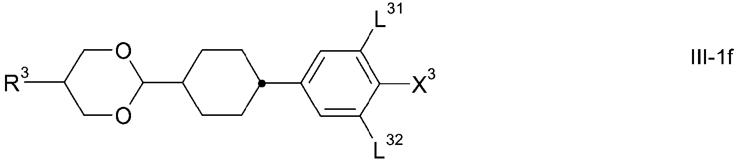 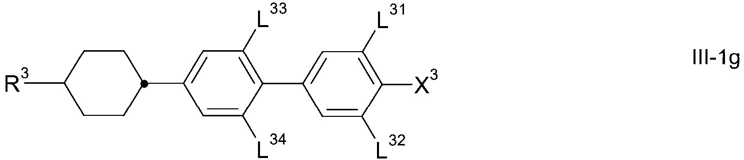 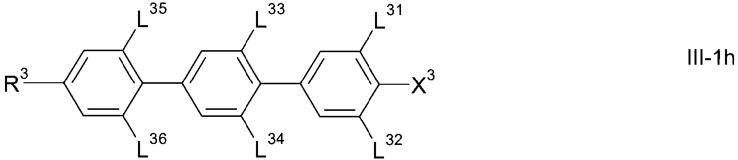 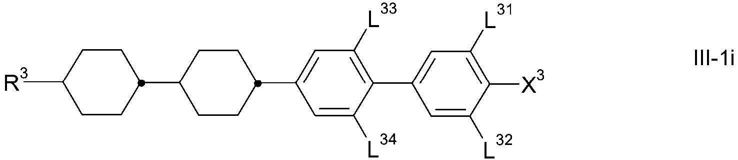 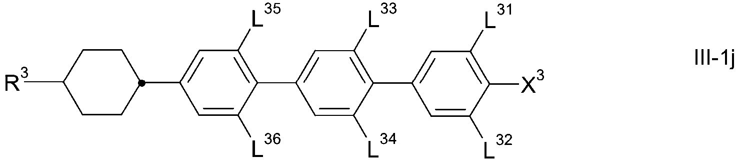 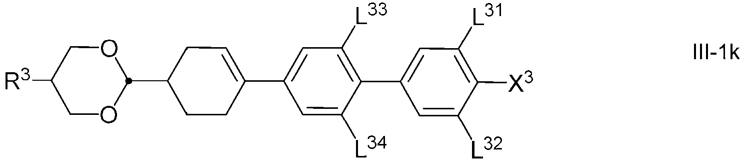 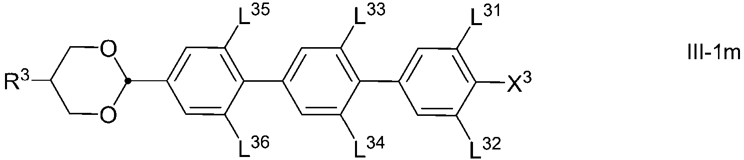 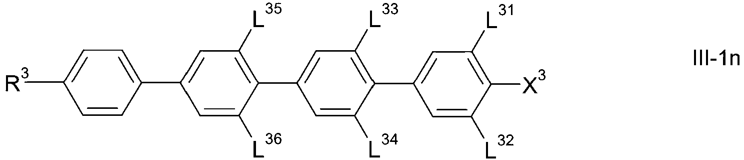 in which the parameters have the meanings given above and preferably in which
X³ denotes F, Cl, halogenated alkyl or alkoxy having 1 to 3 C atoms or halogenated alkenyl or alkenyloxy having 2 or 3 C atoms, F, Cl, -OCF₃, -OCHF₂, -O-CH₂CF₃, -O-CH=CF₂, -O-CH=CH₂ or -CF₃, preferably denotes F, -OCF₃, or -CF₃,
L³³ and L³¹, independently of one another, denote H or F,
L³¹ and L³⁶, independently of one another, denote H or F, preferably H.

The liquid-crystal medium preferably comprises one or more compounds of the formula III-1c, which are preferably selected from the group of the compounds of the formulae III-1c-1 to III-1c-5, preferably of formulae III-1c-1 and/or III-1c-2, most preferably of formula III-1c-1: 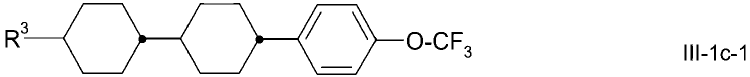 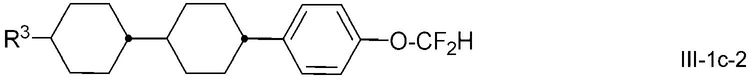 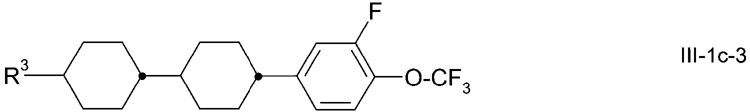 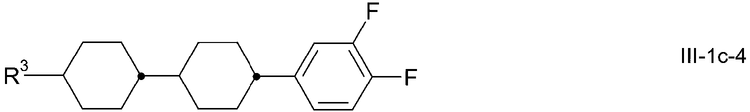 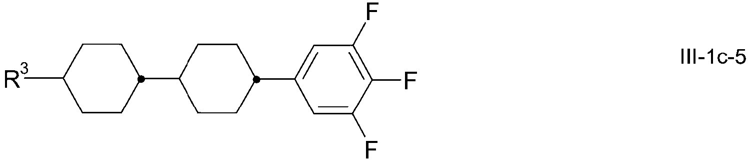 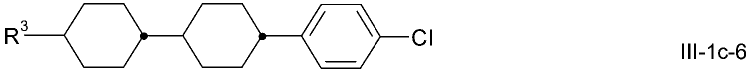 in which R³ has the meaning indicated above and wherein the respective rings, and preferably the phenylene rings, optionally may each be substituted by one or two alkyl groups, preferably by methyl and/or ethyl groups, preferably by one methyl group.

The liquid-crystal medium preferably comprises one or more compounds of the formula III-1d, which are preferably selected from the group of the compounds of the formulae III-1d-1 to III-1d-2, preferably of formulae III-1d-1: 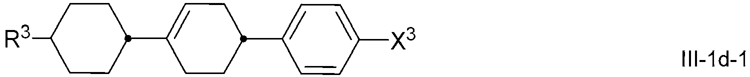 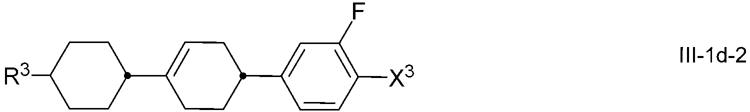 in which
R³ is defined as above, and X³ is CF₃, F, OCF₃, -OCF₂H or -OCH₂F, preferably F or CF₃.

The liquid-crystal medium preferably comprises one or more compounds of the formula III-1g, which are preferably selected from the group of the compounds of the formulae III-1g-1 to III-1g-6, preferably of formulae
III-1g-1 and/or III-1g-2 and/or III-1g-3 and /or III-1g-6, more preferably of formula III-1g-3 and/or III-1g-6, more preferably of formula III-1g-6: 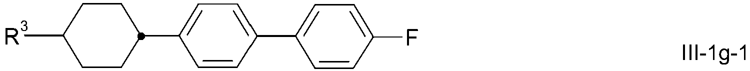 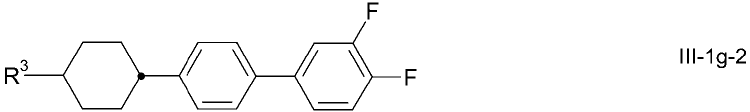 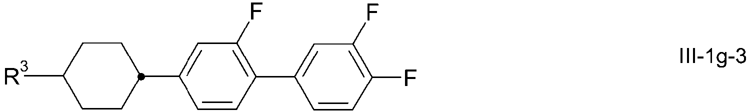 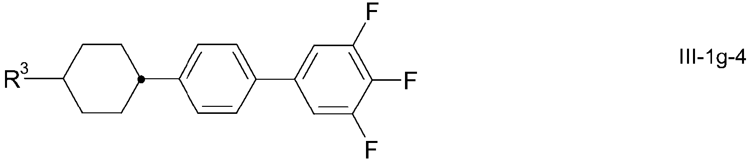 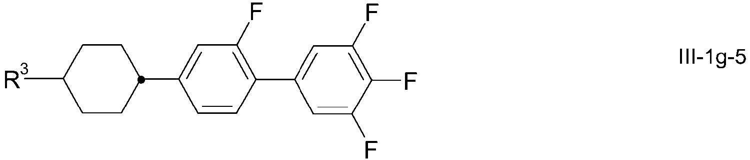 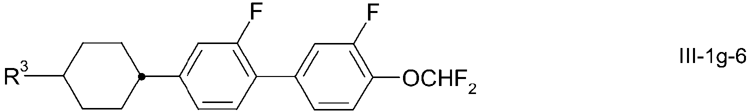 in which R³ has the meaning indicated above and wherein the respective rings, and preferably the phenylene rings, optionally may each be substituted by one or two alkyl groups, preferably by methyl and/or ethyl groups, preferably by one methyl group.

The liquid-crystal medium preferably comprises one or more compounds of the formula III-1g, which are preferably selected from the group of the compounds of the formulae III-1h-1 to III-1h-6, preferably of formula
III-1h-3: 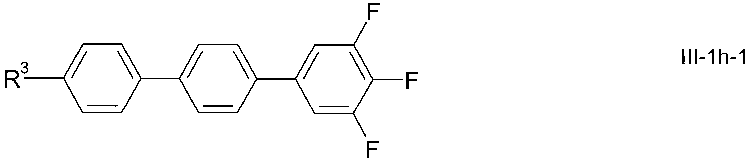 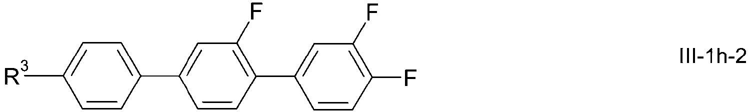 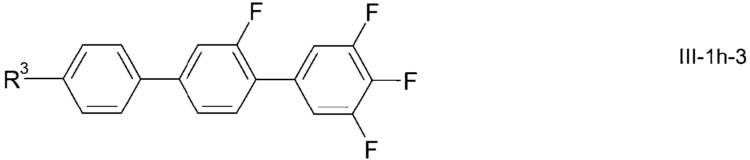 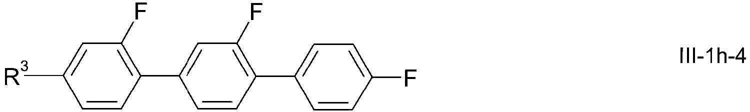 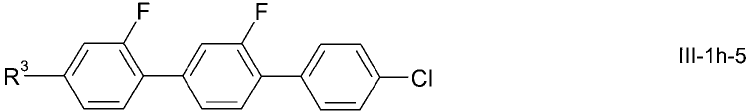 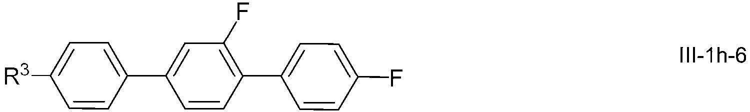 in which R³ has the meaning indicated above and wherein the respective rings, and preferably the phenylene rings, optionally may each be substituted by one or two alkyl groups, preferably by methyl and/or ethyl groups, preferably by one methyl group.

The liquid-crystal medium preferably comprises one or more compounds selected from the formulae III-1j, III-1 k, III-1m and III-1n, which are preferably selected from the group of the compounds of the formulae III-1j-1, III-1k-1, III-1m-1 and III-1n-1, preferably of the formula III-1j-1: 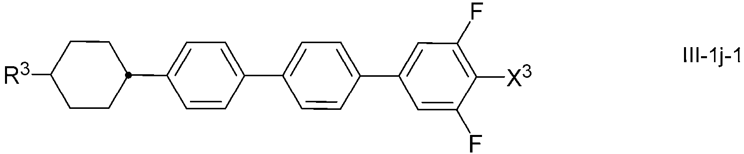 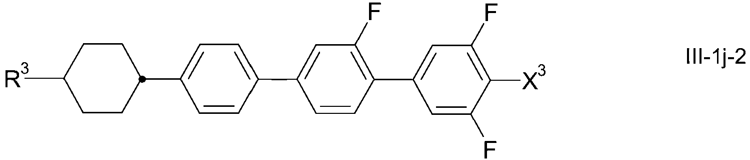 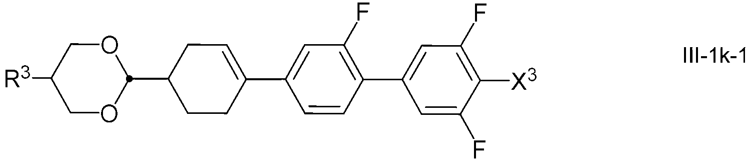 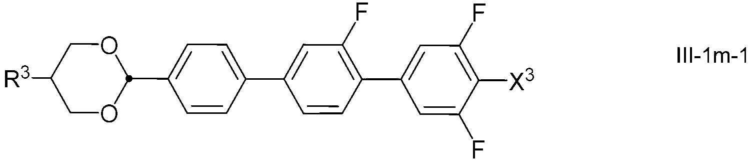 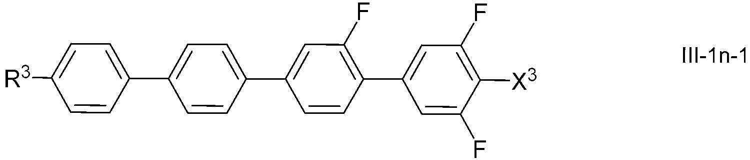 in which the parameters have the meanings given above, and X³ preferably denotes F, -OCF₃, -OCHF₂ or -OCH₂F.

The liquid-crystal medium preferably comprises one or more compounds of the formula III-1k, which are preferably selected from the group of the compounds of the formulae III-1n-1 and III-1n-2, preferably of the formula III-1n-1: 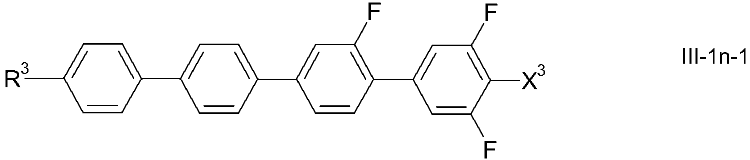 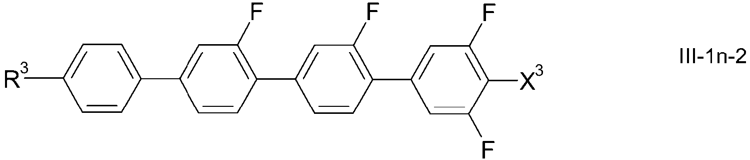 in which the parameters have the meanings given above and wherein the respective rings, and preferably the phenylene rings, optionally may each be substituted by one or two alkyl groups, preferably by methyl and/or ethyl groups, preferably by one methyl group.

The liquid-crystalline media in accordance with the present invention preferably comprise one or more dielectrically neutral compounds having a dielectric anisotropy in the range from -1.5 to 3, preferably selected from the group of the compounds of the formulae VI, VII, VIII and IX.

In the present application, the elements all include their respective isotopes. In particular, one or more H in the compounds may be replaced by D, and this is also particularly preferred in some embodiments. An increased degree of deuteration of the corresponding compounds enables, for example, detection and recognition of the compounds.

In the present application,
- alkyl: particularly preferably denotes straight-chain alkyl, in particular CH₃-, C₂H₅-, *n*-C₃H₇-, *n*-C₄H₉- or *n*-C₅H₁₁-, and
- alkenyl: particularly preferably denotes CH₂=CH-, *E*-CH₃-CH=CH-, CH₂=CH-CH₂-CH₂-, *E*-CH₃-CH=CH-CH₂-CH₂- or *E*-(*n*-C₃H₇)-CH=CH-.

In a further preferred embodiment, the medium comprises one or more compounds of formula IV, preferably of formula IVa or IVb 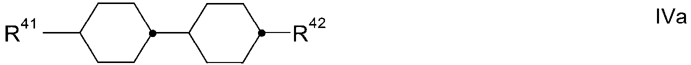 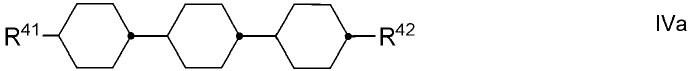 in which
- R⁴¹: denotes an unsubstituted alkyl radical having 1 to 7 C atoms where, in addition, one or more CH₂ groups may be replaced by 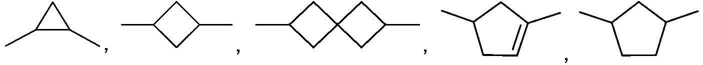 or an unsubstituted alkenyl radical having 2 to 7 C atoms, preferably an n-alkyl radical, particularly preferably having 2, 3, 4 or 5 C atoms, and
- R⁴²: denotes an unsubstituted alkyl radical having 1 to 7 C atoms or an unsubstituted alkoxy radical having 1 to 6 C atoms, both preferably having 2 to 5 C atoms, or an unsubstituted alkenyl radical having 2 to 7 C atoms, preferably having 2, 3 or 4 C atoms, more preferably a vinyl radical or a 1-propenyl radical.

In a particularly preferred embodiment, the medium comprises one or more compounds of formula IV selected from the group of the compounds of the formulae IV-1 to IV-11, preferably of formula IV-1, 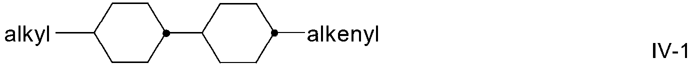 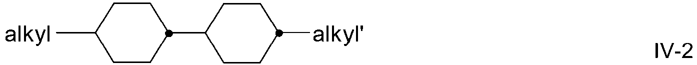 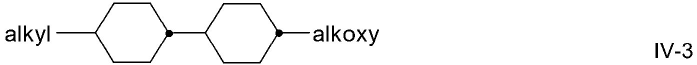 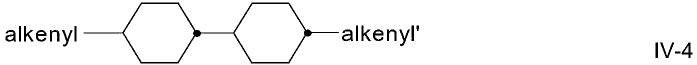 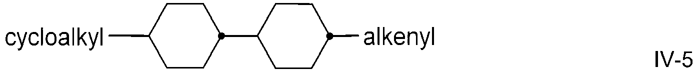 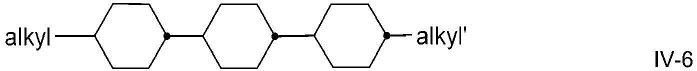 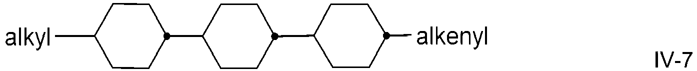 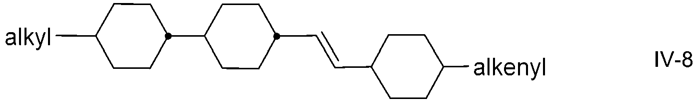 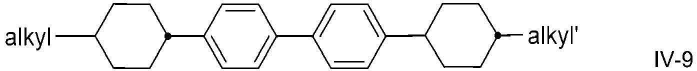 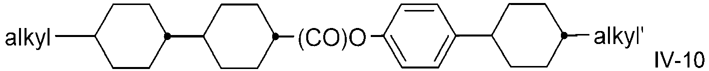 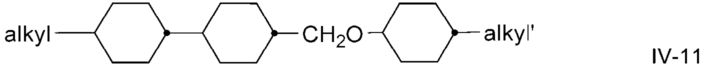 in which
- alkyl and alkyl',: independently of one another, denote alkyl having 1 to 7 C atoms, preferably having 2 to 5 C atoms,
- alkenyl,: denotes alkenyl having 2 to 5 C atoms, preferably having 2 to 4 C atoms, particularly preferably 2 C atoms,
- alkenyl': denotes alkenyl having 2 to 5 C atoms, preferably having 2 to 4 C atoms, particularly preferably having 2 to 3 C atoms,
- cycloalkyl: denotes a partly or fully cyclic alkyl group having 3 to 12 C atoms, preferably cyclopropyl, cyclobutyl, cyclopentyl, cyclopropylalkyl, cyclobutylalkyl or cyclopentylalkyl, very preferably selected from the group consisting of 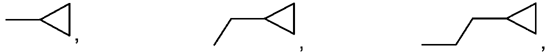 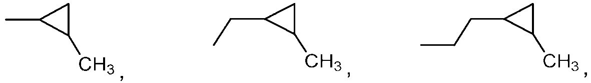 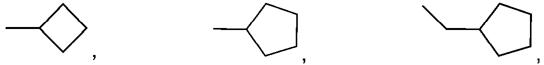 most preferably cyclopropyl, and
- alkoxy: denotes alkoxy having 1 to 5 C atoms, preferably having 2 to 4 C atoms.

In a preferred embodiment, the media according to the invention comprise one or more compounds of formula IV-1 and/or one or more compounds of formula IV-2.

In another preferred embodiment the media according to the invention comprise two or more compounds of formula IV-1 selected from the formulae 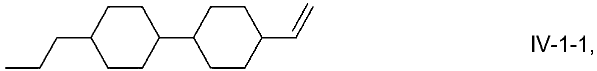 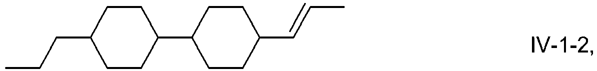 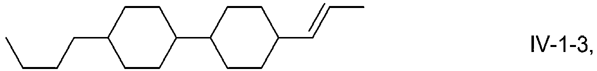 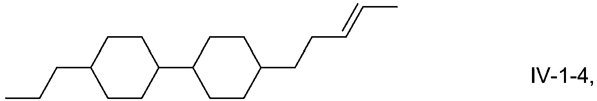 particularly a combination of the compounds IV-1-1 and IV-1-4.

In a further preferred embodiment, the medium comprises one or more compounds of formula IV, selected from the group of the compounds of the formulae IV-2 and IV-3, 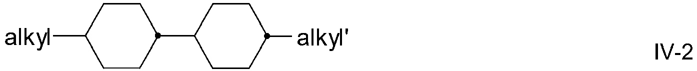 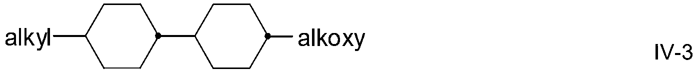 in which
- alkyl and alkyl',: independently of one another, denote alkyl having 1 to 7 C atoms, preferably having 2 to 5 C atoms,
- alkoxy: denotes alkoxy having 1 to 5 C atoms, preferably having 2 to 4 C atoms.

In a further preferred embodiment, the medium comprises one or more compounds of formula V. In this further preferred embodiment, the medium comprises one or more compounds of formula V selected from the group of the compounds of the formulae V-1 to V-5, preferably one or more of formulae V-1, V-3, V-4 and V-5, 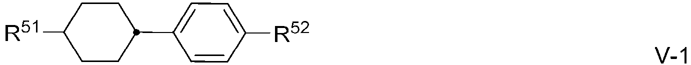 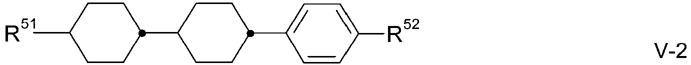 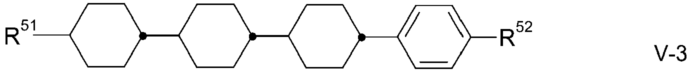 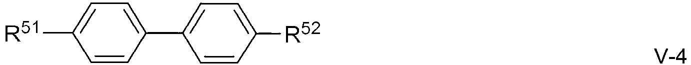 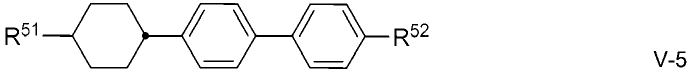 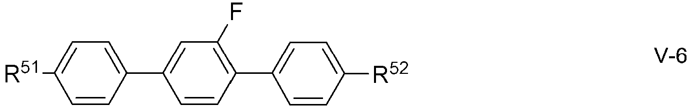 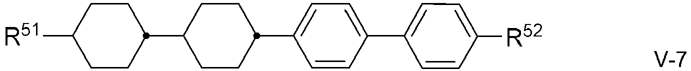 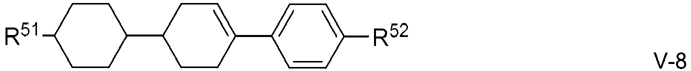 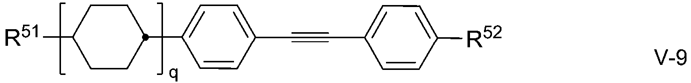 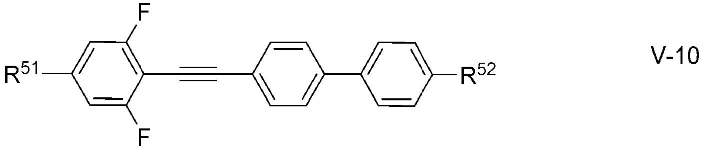 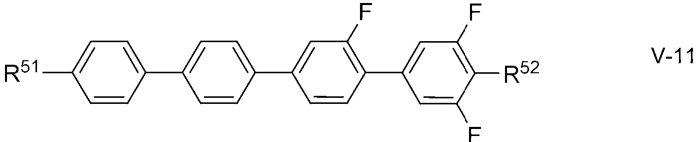 in which the parameters have the meanings given above under formula V, and preferably
- q: is 0 or 1, preferably 0,
- R⁵¹: denotes alkyl having 1 to 7 C atoms or alkenyl having 2 to 7 C atoms, and
- R⁵²: denotes alkyl having 1 to 7 C atoms, alkenyl having 2 to 7 C atoms or alkoxy having 1 to 6 C atoms, preferably alkyl or alkenyl.

In a further preferred embodiment the medium contains one or more compounds of the formula V-1 selected from the following formulae: 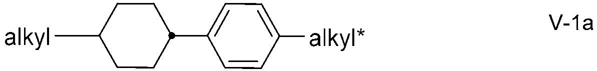 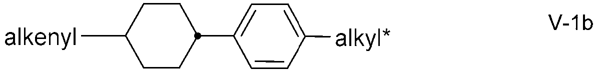 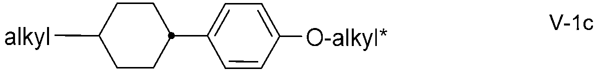 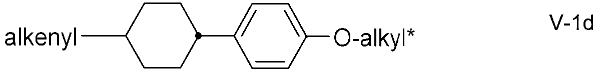 in which "alkyl" has the definition given above, and is preferably methyl, ethyl, propyl or butyl. Particular preference is given to compounds of the formula V-1a, V-1b and V-1c.

In a further preferred embodiment the medium comprises one or more compounds of the formula V-2 in which at least one of the R⁵¹ and R⁵² radicals is alkenyl having 2 to 6 carbon atoms, preferably those selected from the following formula: 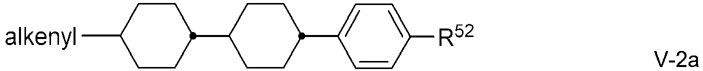 wherein alkenyl preferably denotes alkenyl having 2 to 5 C atoms, preferably having 2 to 4 C atoms, particularly preferably vinyl or 3-buten-1-yl, and R⁵² is defined as above, and preferably is methyl or ethyl.

In a further preferred embodiment the medium comprises one or more compounds of the formula V-4 in which one or both of the R⁵¹ and R⁵² radicals is alkenyl having 2 to 6 carbon atoms, preferably those selected from the following formulae: 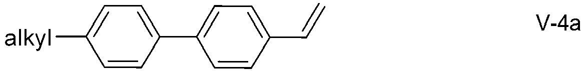 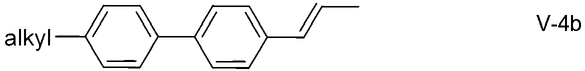 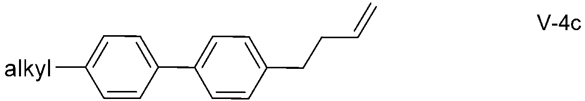 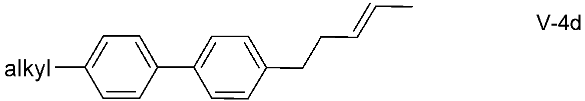 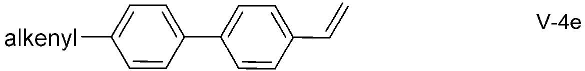 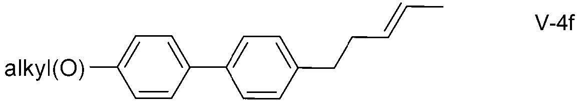 in which
- alkyl: denotes straight-chain alkyl radical having 1 to 6 carbon atoms, especially methyl, ethyl or n-propyl, and
- alkenyl: denotes alkenyl having 2 to 5 C atoms, preferably having 2 to 4 C atoms, particularly preferably vinyl.

Particular preference is given to compounds of the formula V-4d.

In a further preferred embodiment, the medium comprises one or more compounds of formula V-8 selected from the group of the compounds of the formulae V-8a to V-8c, preferably V-8a: 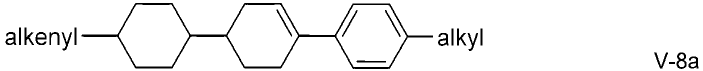 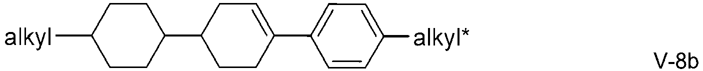 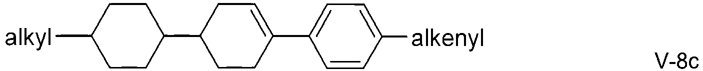 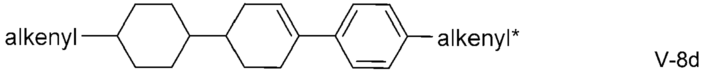 in which
- alkyl and alkyl*: are each independently straight-chain alkyl radical having 1 to 6 carbon atoms, especially methyl, ethyl or n-propyl, and
- alkenyl and alkenyl*: preferably denotes alkenyl having 2 to 5 C atoms, preferably having 2 to 4 C atoms, particularly preferably vinyl.

Among the compounds of formula V-8b the following structures are preferred:

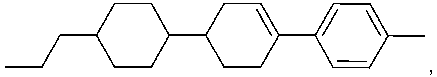

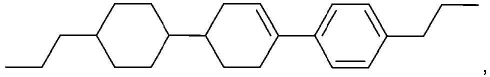

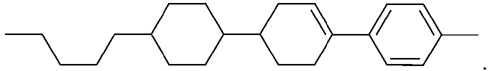

In a further preferred embodiment, the medium comprises one or more compounds of formula V-6 selected from the group of the compounds of the formulae V-4a to V-6c, 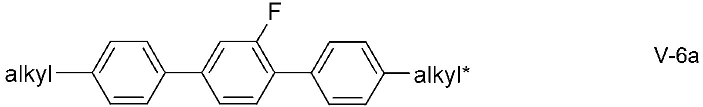 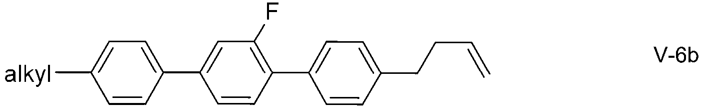 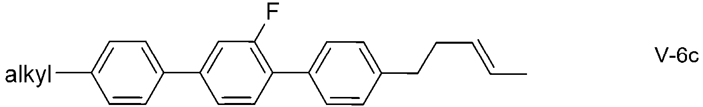 in which
- alkyl and alkyl*: are each independently straight-chain alkyl radical having 1 to 6 carbon atoms, especially methyl, ethyl, n-propyl and pentyl.

The liquid crystalline medium preferably comprises two, three or more compounds selected from the group of compounds of formulae V-6a, V-6b and V-6c.

In a further preferred embodiment the medium comprises one or more compounds of the formula V-7 selected from the group of the following formulae: 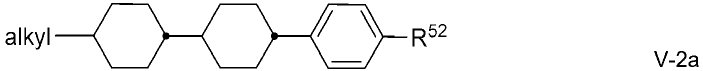 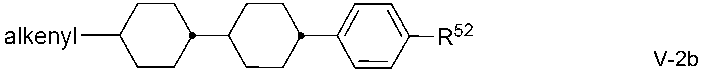 in which
- alkyl: denotes straight-chain alkyl radical having 1 to 6 carbon atoms, especially methyl, ethyl or n-propyl, and
- alkenyl: preferably denotes alkenyl having 2 to 5 C atoms, preferably having 2 to 4 C atoms, particularly preferably vinyl or 3-buten-1-yl, and
- R⁵²: is defined as above, and preferably is alkyl or alkoxy, more preferably methyl or ethyl, more preferably ethyl.

The media according to the invention preferably comprise the following compounds in the total concentrations indicated:

| | |
|---|---|
| 1 - 20 % | by weight of one or more compounds selected from the group of the compounds of formula I and |
| 2-60% | by weight of one or more compounds of formula II, preferably selected from the group of the compounds of the formulae II-1 and II-2 and/or |
| 1 - 60 % | by weight of one or more compounds of formula III, and/or |
| 5-80% | by weight of one or more compounds of formula IV, and/or |
| 0-20% | by weight of one or more compounds of formula T, and/or |
| 0-50% | by weight of one or more compounds of formula V, and/or |
| 0-20% | by weight of one or more compounds of formula VI, and/or |
| 0-20% | by weight of one or more compounds of formula VII, and/or |
| 0-20% | by weight of one or more compounds of formula VIII, preferably selected from the group of the compounds of the formulae VIII-1 and VIII-2 and/or |
| 0-20% | by weight of one or more compounds of formula IX, and/ or |
| 0-30% | by weight of one or more compounds selected from the group of the compounds of formula X |

where the total content of all compounds of formula I, T, of formulae II to IX and of formula X, which are present in the medium, preferably is 95 % or more, more preferably 97 % or more and, most preferably, 100 %, but always not more than 100 %.

The latter condition holds for all media according to the present application.

In a further preferred embodiment, the media in accordance with the present invention in addition to the compounds of formula I or the preferred sub-formulae thereof, preferably comprise one or more dielectrically neutral compounds selected from the group of compounds of formulae IV and V preferably in a total concentration in the range from 5 % or more to 90 % by weight or less, preferably from 10 % or more to 80 % or less, particularly preferably from 20 % or more to 70 % or less.

The medium according to the invention in a particularly preferred embodiment comprises independently
one or more compounds of formula I in a total concentration in the range from 3 % or more to 30 % or less, preferably in the range from 5 % or more to 25 % or less, and/or
one or more compounds of formula T in a total concentration in the range from 1 % or more to 40 % or less, preferably in the range from 5 % or more to 25 % or less, and/or
one or more compounds of formula II in a total concentration in the range from 5 % or more to 50 % or less, preferably in the range from 10 % or more to 40 % or less, and/or
one or more compounds of formula III in a total concentration in the range from 5 % or more to 50 % or less, preferably in the range from 10 % or more to 40 % or less, and/or
one or more compounds of formula IV in a total concentration in the range from 5 % or more to 55 % or less, preferably in the range from 25 % or more to 50 % or less, and/or
one or more compounds of formula V in a total concentration in the range from 3 % or more to 30 % or less, preferably in the range from 5 % or more to 30 % or less, and/or
one or more compounds of the formulae VI and/or VII and/or VIII and/or IX in a total concentration in the range from 0 % or more to 30 % or less, preferably 0 % or more to 20 % or less, more preferably 0 % or more to 15 % or less of the formulae.

All percentages (%) of mixture components within this disclosure are to be understood in % by weight.

Preferably the concentration of the compounds of formula I in the media according to the invention is in the range from 1 % or more to 20 % or less, more preferably from 1.5 % or more to 20 % or less, most preferably from 2 % or more to 12 % or less.

In a preferred embodiment of the present invention the concentration of the compounds of formula II in the media is in the range from 3 % or more to 60 % or less, more preferably from 5 % or more to 45 % or less, more preferably from 5 % or more to 20 % or less and, most preferably, from 7 % or more to 16 % or less.

In a preferred embodiment of the present invention the concentration of the compounds of formula I, T and V altogether in the media is in the range from 30 % or more to 70 % or less, more preferably from 35 % or more to 65 % or less.

In a preferred embodiment of the present invention the concentration of the compounds of formula IV-1, IV-2, IV-3 and V-1 altogether in the media is in the range from 45 % or more to 75 % or less, more preferably from 50 % or more to 70 % or less.

In a preferred embodiment of the present invention the concentration of the compounds of formula III-1d and V-5 altogether in the media is in the range from 4 % or more to 25 % or less, more preferably from 8 % or more to 20 % or less.

In a preferred embodiment of the present invention the concentration of the compounds of formula V-1a and V-5, preferably V-5a, altogether in the media is in the range from 12 % or more to 30 % or less, more preferably from 18 % or more to 28 % or less.

The present invention also relates to electro-optical displays or electro-optical components which contain liquid-crystalline media according to the invention. Preference is given to electro-optical displays which are based on the FFS, IPS, VA or ECB effect, preferably on the IPS or FFS effect, and in particular those which are addressed by means of an active-matrix addressing device.

Accordingly, the present invention likewise relates to the use of a liquid-crystalline medium according to the invention in an electro-optical display or in an electro-optical component, and to a process for the preparation of the liquid-crystalline media according to the invention, characterised in that one or more compounds of formula I are mixed with one or more additional mesogenic compounds and optionally one ore more additives.

Besides compounds of the formulae I, T, II to X and L, other constituents may also be present, for example in an amount of up to 45 %, but preferably up to 35 %, in particular up to 10 %, of the mixture as a whole.

The media according to the invention may optionally also comprise dielectrically negative compounds, whose total concentration is preferably 20 % or less, more preferably 10 % or less, based on the entire medium.

The liquid-crystal media in accordance with the present invention may comprise one or more chiral compounds.

Particularly preferred embodiments of the present invention meet one or more of the following conditions,
where the acronyms (abbreviations) are explained in Tables A to C and illustrated by examples in Table D.

Preferably the media according to the present invention fulfil one or more of the following conditions.
i. The liquid-crystalline medium has a birefringence of 0.060 or more, particularly preferably 0.070 or more.
ii. The liquid-crystalline medium has a birefringence of 0.200 or less, particularly preferably 0.180 or less.
iii. The liquid-crystalline medium has a birefringence in the range from 0.090 or more to 0.180 or less.
iv. The liquid-crystalline medium comprises one or more particularly preferred compounds of formula I, preferably selected from the (sub-) formulae I-a and I-b.
v. The liquid-crystalline medium comprises one or more preferred compounds of formula T.
vi. The total concentration of the compounds of formula II in the mixture as a whole is 0.5 % or more, preferably 1 % or more, and preferably 7 % or less, particularly preferably 8 % or less, and very particularly preferably in the range from 1 % or more to 40 % or less.
vii. The liquid-crystalline medium comprises one or more compounds of formula IV selected from the group of the compounds of the following formulae: CC-n-V and/or CC-n-Vm and/or CC-n-nVm and/or CC-V-V and/or CC-V-Vn and/or CC-nV-Vn, particularly preferably CC-3-V, preferably in a concentration of up to 60 % or less, particularly preferably up to 50 % or less, and optionally additionally to CC-3-V the compound(s) CC-3-V1, preferably in a concentration of up to 15 % or less, and/or CC-3-2V1, preferably in a concentration of up to 15 % or less, and/or CC-4-V1, preferably in a concentration of up to 20 % or less, particularly preferably up to 15 % or less, and/or CC-5-V, preferably in a concentration of up to 20 % or less.
viii. The media comprise the compound of formula CC-n-V, preferably CC-3-V, preferably in a concentration of 1 % or more to 60 % or less, more preferably in a concentration of 20 % or more to 55 % or less.
ix. The liquid-crystalline medium comprises one or more compounds of formula IV, preferably of the formulae IV-1 and/or IV-2, preferably in a total concentration of 20 % or more, in particular 30 % or more, and very particularly preferably 40 % or more, and is preferably in the range from 46 % to 56 % of compounds of formula IV-1.
x. The total concentration of the compounds of formula V in the mixture as a whole is 5 % or more, preferably 7 % or more, and is preferably in the range from 5 % or more to 40 % or less, particularly preferably in the range from 5 % or more to 30 % or less.
xi. The total concentration of the compounds of formula V-4 in the mixture as a whole preferably is 5 % or more to 25 % or less, preferably 5 % or more to 15 % or less.
xii. The total concentration of the compounds of formula V-6, preferably V-6a to V-6c, in the mixture as a whole preferably is 3 % or more to 30 % or less, preferably 10 % or more to 25 % or less.
xiii. The total concentration of the compounds selected from formula V-8, preferably V-8-a, and III-1d, preferably III-1d-1, in the mixture as a whole preferably is 2 % or more to 20 % or less, preferably 4 % or more to 15 % or less.

The invention furthermore relates to an electro-optical display having active-matrix addressing based on the IPS, FFS or UB-FFS effect, characterised in that it contains, as dielectric, a liquid-crystalline medium in accordance with the present invention.

The liquid-crystal mixture preferably has a nematic phase range having a width of at least 70 degrees.

The rotational viscosity γ₁ is preferably 350 mPa·s or less, preferably 250 mPa·s or less and, in particular, 150 mPa·s or less.

The mixtures according to the invention are suitable for all IPS and FFS-TFT applications using dielectrically positive liquid crystalline media, such as, e.g. XB-FFS.

The liquid-crystalline media according to the invention preferably virtually completely consist of 4 to 15, in particular 5 to 12, and particularly preferably 10 or less, compounds. These are preferably selected from the group of the compounds of the formulae I, T, II, III, IV, V, VI, VII, VIII and IX.

The liquid-crystalline media according to the invention may optionally also comprise more than 18 compounds. In this case, they preferably comprise 18 to 25 compounds.

In a preferred embodiment, the liquid-crystal media according to the invention predominantly comprise, preferably essentially consist of and, most preferably, virtually completely consist of compounds, which do not comprise a cyano group.

In a preferred embodiment, the liquid-crystal media according to the invention comprise compounds selected from the group of the compounds of the formulae I, T, II, III, and IV and V, preferably selected from the group of the compounds of the formulae la, T, II-1, II-2, III-1, III-2, IV, and V; they preferably consist predominantly, particularly preferably essentially and very particularly preferably virtually completely of the compounds of the said formulae.

The liquid-crystal media according to the invention preferably have a nematic phase from in each case at least -10°C or less to 70°C or more, particularly preferably from -20°C or less to 80°C or more, very particularly preferably from -30°C or less to 85°C or more and most preferably from -40°C or less to 90°C or more.

The expression "have a nematic phase" here means on the one hand that no smectic phase and no crystallisation are observed at low temperatures at the corresponding temperature and on the other hand that no clearing occurs on heating out of the nematic phase. The investigation at low temperatures is carried out in a flow viscometer at the corresponding temperature and checked by storage in test cells having a cell thickness corresponding to the electro-optical application for at least 100 hours. If the storage stability at a temperature of -20°C in a corresponding test cell is 1,000 h or more, the medium is regarded as stable at this temperature. At temperatures of -30°C and -40°C, the corresponding times are 500 h and 250 h respectively. At high temperatures, the clearing point is measured in capillaries by conventional methods.

In a preferred embodiment, the liquid-crystal media according to the invention are characterised by optical anisotropy values in the moderate to low range. The birefringence values are preferably in the range from 0.075 or more to 0.130 or less, particularly preferably in the range from 0.085 or more to 0.120 or less and very particularly preferably in the range from 0.090 or more to 0.115 or less.

Preferably the liquid-crystalline media according to the present invention, on the one hand, have a value of the dielectric anisotropy of 1.5 or more, preferably of 2.5 or more. At the other hand, they preferably have a dielectric anisotropy of 26 or less, preferably of 15 or less and most preferably of 10 or less.

In this embodiment, the liquid-crystal media according to the invention have a positive dielectric anisotropy Δε, which preferably is in the range from 2.0 or more to 20 or less, more preferably to 15 or less, more preferably from 2.0 or more to 10 or less, particularly preferably from 2.0 or more to 9.0 or less and very particularly preferably from 2.5 or more to 8.0 or less.

The liquid-crystal media according to the invention preferably have relatively low values for the threshold voltage (V₀) in the range from 1.1 V or more to 5.0 V or less, preferably to 2.7 V or less, preferably from 1.3 V or more to 2.6 V or less, particularly preferably from 1.4 V or more to 2.5 V or less.

In addition, the liquid-crystal media according to the invention have high values for the VHR in liquid-crystal cells.

In general, liquid-crystal media having a low addressing voltage or threshold voltage here have a lower VHR than those having a higher addressing voltage or threshold voltage, and vice versa.

These preferred values for the individual physical properties are preferably also in each case maintained by the media according to the invention in combination with one another.

In the present application, the term "compounds", also written as "compound(s)", means both one and also a plurality of compounds, unless explicitly indicated otherwise.

For the present invention, the following definitions apply in connection with the specification of the constituents of the compositions, unless indicated otherwise in individual cases:
- "comprise": the concentration of the constituents in question in the composition is preferably 5% or more, particularly preferably 10% or more, very particularly preferably 20% or more,
- "predominantly consist of": the concentration of the constituents in question in the composition is preferably 50% or more, particularly preferably 55% or more and very particularly preferably 60% or more,
- "essentially consist of": the concentration of the constituents in question in the composition is preferably 80% or more, particularly preferably 90% or more and very particularly preferably 95% or more, and
- "virtually completely consist of": the concentration of the constituents in question in the composition is preferably 98% or more, particularly preferably 99% or more and very particularly preferably 100.0%.

This applies both to the media as compositions with their constituents, which can be groups of compounds as well as individual compounds, and also to the groups of compounds with their respective constituents, the compounds. Only in relation to the concentration of an individual compound relative to the medium as a whole does the term comprise mean: the concentration of the compound or compounds in question is preferably 1% or more, particularly preferably 2% or more, very particularly preferably 4% or more.

For the present invention, "≤" means less than or equal to, preferably less than, and "≥" means greater than or equal to, preferably greater than.

For the present invention 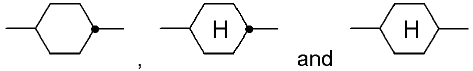 denote *trans*-1,4-cyclohexylene, 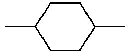 denotes a mixture of both *cis-* and *trans*-1,4-cyclohexylene and 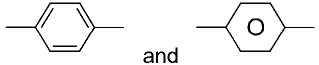 denote 1,4-phenylene.

Throughout this application 1,3-cyclopentenylene is a moiety selected from the group of the formulae 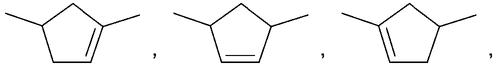 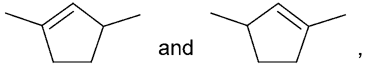 preferably 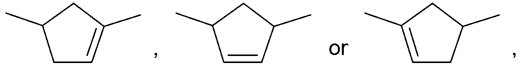 most preferably

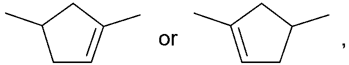

For the present invention, the expression "dielectrically positive compounds" means compounds having a Δε of > 1.5, the expression "dielectrically neutral compounds" means compounds having -1.5 ≤ Δε ≤ 1.5 and the expression "dielectrically negative compounds" means compounds having Δε < -1.5. The dielectric anisotropy of the compounds is determined here by dissolving 10 % by weight of the compounds in a liquid-crystalline host and determining the capacitance of the resultant mixture in each case in at least one test cell having a cell thickness of 20 µm with homeotropic and with homogeneous surface alignment at 1 kHz. The measurement voltage is typically 0.5 V to 1.0 V, but is always lower than the capacitive threshold of the respective liquid-crystal mixture investigated.

The host mixture used for dielectrically positive and dielectrically neutral compounds is ZLI-4792 and that used for dielectrically negative compounds is ZLI-2857, both from Merck KGaA, Germany. The values for the respective compounds to be investigated are obtained from the change in the dielectric constant of the host mixture after addition of the compound to be investigated and extrapolation to 100% of the compound employed. The compound to be investigated is dissolved in the host mixture in an amount of 10 % by weight. If the solubility of the substance is too low for this purpose, the concentration is halved in steps until the investigation can be carried out at the desired temperature.

The liquid-crystal media according to the invention may, if necessary, also comprise further additives, such as, for example, stabilisers and/or pleochroitic, e.g. dichroitic, dyes and/or chiral dopants in the usual amounts. The amount of these additives employed is preferably in total 0 % or more to 10 % by weight or less, based on the amount of the entire mixture, particularly preferably 0.1 % or more to 6 % or less. The concentration of the individual compounds employed is preferably 0.1 % or more to 3 % or less. The concentration of these and similar additives is generally not taken into account when specifying the concentrations and concentration ranges of the liquid-crystal compounds in the liquid-crystal media.

In another embodiment, the liquid-crystal media according to the invention comprise a polymer precursor which comprises one or more reactive compounds, preferably reactive mesogens, and, if necessary, also further additives, such as, for example, polymerisation initiators and/or polymerisation moderators, in the usual amounts. The amount of these additives employed is in total 0 % or more to 10 % by weight or less, based on the amount of the entire mixture, preferably 0.1 % or more to 2 % by weight or less. The concentration of these and similar additives is not taken into account when specifying the concentrations and concentration ranges of the liquid-crystal compounds in the liquid-crystal media.

The compositions consist of a plurality of compounds, preferably 3 or more to 30 or fewer, particularly preferably 6 or more to 20 or fewer and very particularly preferably 10 or more to 16 or fewer compounds, which are mixed in a conventional manner. In general, the desired amount of the compounds used in lesser amount is dissolved in the compounds making up the principal constituent of the mixture. This is advantageously carried out at elevated temperature. If the selected temperature is above the clearing point of the principal constituent, completion of the dissolution operation is particularly easy to observe. However, it is also possible to prepare the liquid-crystal mixtures in other conventional ways, for example using pre-mixes or from a so-called "multi-bottle system".

The mixtures according to the invention exhibit very broad nematic phase ranges having clearing points of 65°C or more, very favourable values for the capacitive threshold, relatively high values for the holding ratio and at the same time very good low-temperature stabilities at -30°C and -40°C. Furthermore, the mixtures according to the invention are distinguished by low rotational viscosities γ₁.

It goes without saying to the person skilled in the art that the media according to the invention for use in VA, IPS, FFS or PALC displays may also comprise compounds in which, for example, H, N, O, Cl, F have been replaced by the corresponding isotopes.

The structure of the FFS liquid-crystal displays according to the invention corresponds to the usual geometry, as described, for example, in US 2002/0041354 A1.

The liquid-crystal phases according to the invention can be modified by means of suitable additives in such a way that they can be employed in any type of, for example, IPS and FFS LCD display that has been disclosed to date.

Table E below indicates possible dopants which can be added to the mixtures according to the invention. If the mixtures comprise one or more dopants, it is (they are) employed in amounts of 0.01 % to 4 % by weight, preferably 0.1 % to 1.0 %. Stabilisers which can be added, for example, to the mixtures according to the invention, preferably in amounts of 0.01 % to 6 % by weight, in particular 0.1 % to 3 %, are shown below in Table F.

For the purposes of the present invention, all concentrations are, unless explicitly noted otherwise, indicated in per cent by weight and relate to the corresponding mixture as a whole or mixture constituents, again a whole, unless explicitly indicated otherwise. In this context the term "the mixture" describes the liquid crystalline medium.

All temperature values indicated in the present application, such as, for example, the melting point T(C,N), the smectic (S) to nematic (N) phase transition T(S,N) and the clearing point T(N,I), are indicated in degrees Celsius (°C) and all temperature differences are correspondingly indicated in differential degrees (° or degrees), unless explicitly indicated otherwise.

For the present invention, the term "threshold voltage" relates to the capacitive threshold (V₀), also known as the Freedericks threshold, unless explicitly indicated otherwise.

All physical properties are and have been determined in accordance with "Merck Liquid Crystals, Physical Properties of Liquid Crystals", status Nov. 1997, Merck KGaA, Germany, and apply for a temperature of 20°C, and Δn is determined at 436 nm, 589 nm and at 633 nm, and Δε at 1 kHz, unless explicitly indicated otherwise in each case.

The electro-optical properties, for example the threshold voltage (V₀) (capacitive measurement), are, as is the switching behaviour, determined in test cells produced at Merck Japan. The measurement cells have soda-lime glass substrates and are constructed in an ECB or VA configuration with polyimide alignment layers (SE-1211 with diluent **26 (mixing ratio 1:1), both from Nissan Chemicals, Japan), which have been rubbed perpendicularly to one another and effect homeotropic alignment of the liquid crystals. The surface area of the transparent, virtually square ITO electrodes is 1 cm².

Unless indicated otherwise, a chiral dopant is not added to the liquid-crystal mixtures used, but the latter are also particularly suitable for applications in which doping of this type is necessary.

The rotational viscosity is determined using the rotating permanent magnet method and the flow viscosity in a modified Ubbelohde viscometer. For liquid-crystal mixtures ZLI-2293, ZLI-4792 and MLC-6608, all products from Merck KGaA, Darmstadt, Germany, the rotational viscosity values determined at 20°C are 161 mPa·s, 133 mPa·s and 186 mPa·s respectively, and the flow viscosity values (v) are 21 mm²·s⁻¹, 14 mm²·s⁻¹ and 27 mm²·s⁻¹, respectively.

The dispersion of the materials may for practical purposes be conveniently characterized in the following way, which is used throughout this application unless explicitly stated otherwise. The values of the birefringence are determined at a temperature of 20°C at several fixed wavelengths using a modified Abbé refractometer with homeotropically aligning surfaces on the sides of the prisms in contact with the material. The birefringence values are determined at the specific wavelength values of 436 nm (respective selected spectral line of a low pressure mercury lamp), 589 nm (sodium "D" line) and 633 nm (wavelength of a HE-Ne laser (used in combination with an attenuator/diffusor in order to prevent damage to the eyes of the observers. In the following table Δn is given at 589 nm and Δ(Δn) is given as Δ(Δn) = Δn(436 nm) - Δn(633 nm).

The following symbols are used, unless explicitly indicated otherwise:
- V₀: threshold voltage, capacitive [V] at 20°C,
- nₑ: extraordinary refractive index measured at 20°C and 589 nm,
- nₒ: ordinary refractive index measured at 20°C and 589 nm,
- Δn: optical anisotropy measured at 20°C and 589 nm,
- λ: wavelength λ [nm],
- Δn(λ): optical anisotropy measured at 20°C and wavelength λ,
- Δ(Δn): change in optical anisotropy defined as: $Δn \left(20°C, 436 nm\right) - Δn \left(20°C, 633 nm\right) ,$
- Δ(Δn*): "relative change in optical anisotropy" defined as: $Δ \left(Δn\right) / Δn \left(20°C, 589 nm\right) ,$
- ε_{⊥}: dielectric susceptibility perpendicular to the director at 20°C and 1 kHz,
- ε_{∥}: dielectric susceptibility parallel to the director at 20°C and 1 kHz,
- Δε: dielectric anisotropy at 20°C and 1 kHz,
- T(N,I) or clp.: clearing point [°C],
- v: flow viscosity measured at 20°C [mm²·s⁻¹],
- γ₁: rotational viscosity measured at 20°C [mPa·s],
- K₁₁: elastic constant, "splay" deformation at 20°C [pN],
- K₂₂: elastic constant, "twist" deformation at 20°C [pN] (K₂₂ ≈ ½ K₁₁),
- K₃₃: elastic constant, "bend" deformation at 20°C [pN],
- K_{avg}: average elastic constant at 20°C [pN] defined here as ${K}_{avg} ≡ \left(\frac{3}{2} {K}_{11}+{K}_{33}\right) / 3 \approx \left({K}_{11}+{K}_{22}+{K}_{33}\right) / 3 ,$
- LTS: low-temperature stability of the phase, determined in test cells,
- VHR: voltage holding ratio,
- ΔVHR: decrease in the voltage holding ratio, and
- Sᵣₑₗ: relative stability of the VHR,

The following examples explain the present invention without limiting it. However, they show the person skilled in the art preferred mixture concepts with compounds preferably to be employed and the respective concentrations thereof and combinations thereof with one another. In addition, the examples illustrate the properties and property combinations that are accessible.

For the present invention and in the following examples, the structures of the liquid-crystal compounds are indicated by means of acronyms, with the transformation into chemical formulae taking place in accordance with Tables A to C below. All radicals CₙH₂ₙ₊₁, CₘH₂ₘ₊₁ and CₗH₂ₗ₊₁ or CₙH₂ₙ, CₘH₂ₘ and CₗH₂ₗ are straight-chain alkyl radicals or alkylene radicals, in each case having n, m and I C atoms respectively. Preferably n, m and I are independently of each other 1, 2, 3, 4, 5, 6, or 7. Table A shows the codes for the ring elements of the nuclei of the compound, Table B lists the bridging units, and Table C lists the meanings of the symbols for the left- and right-hand end groups of the molecules. The acronyms are composed of the codes for the ring elements with optional linking groups, followed by a first hyphen and the codes for the left-hand end group, and a second hyphen and the codes for the right-hand end group. Table D shows illustrative structures of compounds together with their respective abbreviations.

**Table A: Ring elements**

| | | | |
|---|---|---|---|
| **C** | 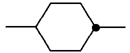 | | |
| **D** | 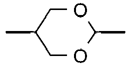 | **DI** | 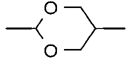 |
| **A** | 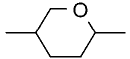 | **AI** | 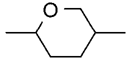 |
| **P** | 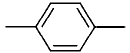 | | |
| **G** | 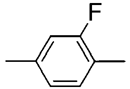 | **GI** | 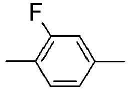 |
| **U** | 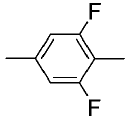 | **UI** | 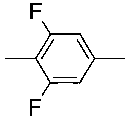 |
| **Y** | 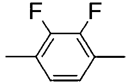 | **np** | 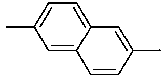 |
| **th** | 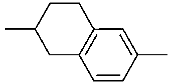 | **thl** | 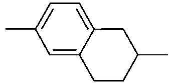 |
| **dh** | 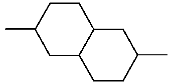 | **nf** | 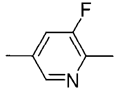 |
| **B** | 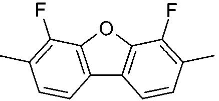 | **B(S)** | 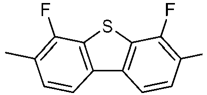 |
| **O** | 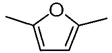 | **S** | 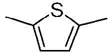 |
| **L** | 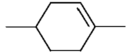 | **LI** | 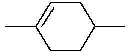 |
| **F** | 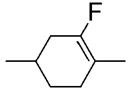 | **FI** | 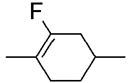 |

**Table B: Bridging units**

| | | | |
|---|---|---|---|
| **E** | -CH₂-CH₂- | | |
| **V** | -CH=CH- | | |
| **T** | -C≡C- | | |
| **W** | -CF₂-CF₂- | | |
| **B** | -CF=CF- | | |
| **Z** | -CO-O- | **ZI** | -O-CO- |
| **X** | -CF=CH- | **XI** | -CH=CF- |
| **O** | -CH₂-O- | **OI** | -O-CH₂- |
| **Q** | -CF₂-O- | **QI** | -O-CF₂- |

**Table C: End groups**

| **On the left individually or in combination** | | **On the right individually or in combination** | |
|---|---|---|---|
| **-n-** | CₙH₂ₙ₊₁- | **-n** | -CₙH₂ₙ₊₁ |
| **-nO-** | CₙH₂ₙ₊₁-O- | **-On** | -O- CₙH₂ₙ₊₁ |
| **-V-** | CH₂=CH- | **-V** | -CH=CH₂ |
| **-nV-** | CₙH₂ₙ₊₁-CH=CH- | **-nV** | -CₙH₂ₙ-CH=CH₂ |
| **-Vn-** | CH₂=CH- CₙH₂ₙ- | **-Vn** | -CH=CH-CₙH₂ₙ₊₁ |
| **-nVm-** | CₙH₂ₙ₊₁-CH=CH-CₘH₂ₘ- | **-nVm** | - CₙH₂ₙ-CH=CH-CₘH₂ₘ₊₁ |
| **-N-** | N≡C- | **-N** | -C≡N |
| **-S-** | S=C=N- | **-S** | -N=C=S |
| **-F-** | F- | **-F** | -F |
| **-CL-** | CI- | **-CL** | -CI |
| **-M-** | CFH₂- | **-M** | -CFH₂ |
| **-D-** | CF₂H- | **-D** | -CF₂H |
| **-T-** | CF₃- | **-T** | -CF₃ |
| **-MO-** | CFH₂O - | **-OM** | -OCFH₂ |
| **-DO-** | CF₂HO - | **-OD** | -OCF₂H |
| **-TO-** | CF₃O - | **-OT** | -OCF₃ |
| **-A-** | H-C≡C- | **-A** | -C≡C-H |
| **-nA-** | CₙH₂ₙ₊₁-C≡C- | **-An** | -C≡C-CₙH₂ₙ₊₁ |
| **-NA-** | N≡C-C≡C- | **-AN** | -C≡C-C≡N |
| **-(C5)-** | cyclopentyl | **-(C5)** | cyclopentyl |
| **-(C3)-** | cyclopropyl | **-(C3)** | cyclopropyl |
| | | **-Nap** | 2-naphthyl |

| **On the left only in combination** | | **On the right only in combination** | |
|---|---|---|---|
| **-...n...-** | -CₙH₂ₙ- | **-...n...** | -CₙH₂ₙ- |
| **-...M...-** | -CFH- | **-...M...** | -CFH- |
| **-...D...-** | -CF₂- | **-...D...** | -CF₂- |
| **-...V...-** | -CH=CH- | **-... V...** | -CH=CH- |
| **-...Z...-** | -CO-O- | **-...z...** | -CO-O- |
| **-...ZI...-** | -O-CO- | **-...ZI...** | -O-CO- |
| **-...K...-** | -CO- | **-... K...** | -CO- |
| **-...W...-** | -CF=CF- | **-...W...** | -CF=CF- |
| **-...O...-** | -O- | **-...O...-** | -O- |

in which n and m are each integers, and the three dots "..." are placeholders for other abbreviations from this table.

Besides the compounds of formula I, the mixtures according to the invention preferably comprise one or more compounds of the compounds mentioned below.

The following abbreviations are used:
(n, m, k and I are, independently of one another, each an integer, preferably 1 to 9 preferably 1 to 7, k and I possibly may be also 0 and preferably are 0 to 4, more preferably 0 or 2 and most preferably 2, n preferably is 1, 2, 3, 4 or 5, in the combination "-nO-" it preferably is 1, 2, 3 or 4, preferably 2 or 4, m preferably is 1, 2, 3, 4 or 5, in the combination "-Om" it preferably is 1, 2, 3 or 4, more preferably 2 or 4. The combination "-IVm" preferably is "2V1".)

**Table D**

| |
|---|
| Exemplary, preferred dielectrically positive compounds |
| 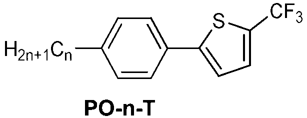 |
| 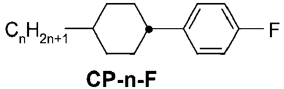 |
| 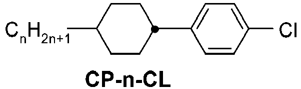 |
| 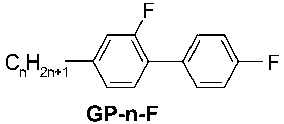 |
| 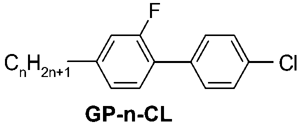 |
| 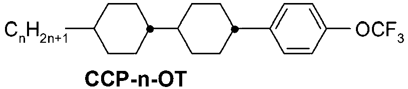 |
| 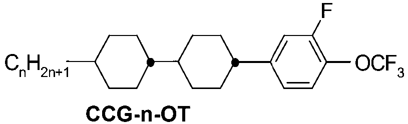 |
| 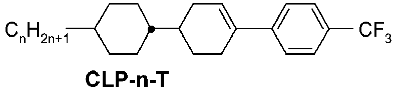 |
| 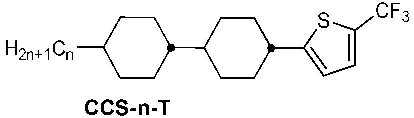 |
| 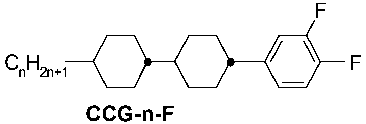 |
| 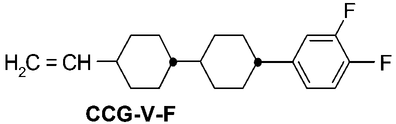 |
| 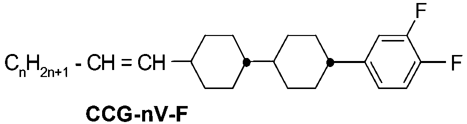 |
| 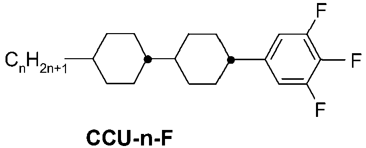 |
| 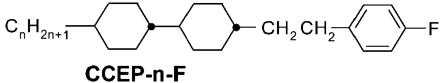 |
| 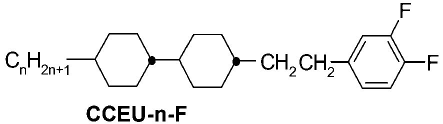 |
| 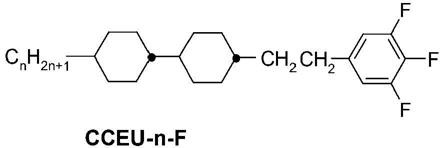 |
| 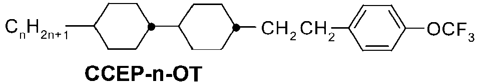 |
| 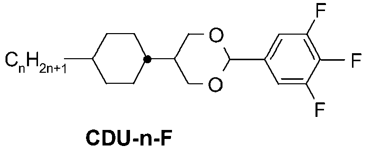 |
| 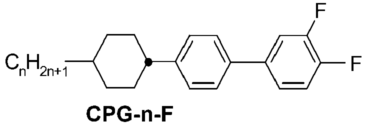 |
| 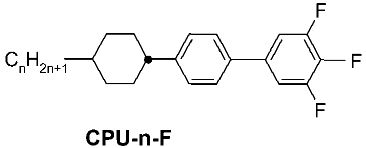 |
| 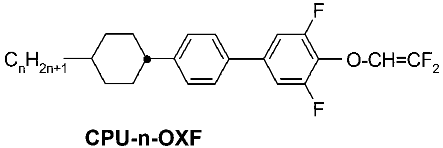 |
| 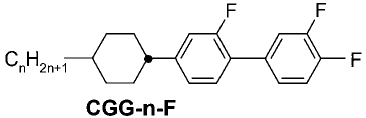 |
| 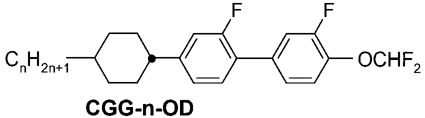 |
| 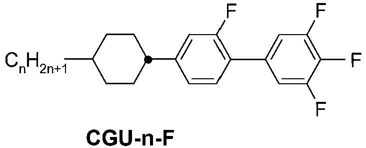 |
| 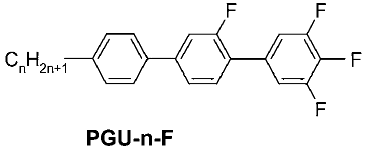 |
| 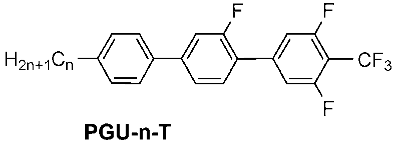 |
| 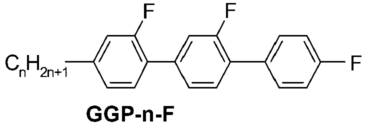 |
| 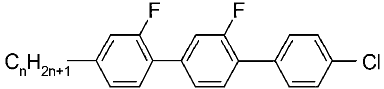 |
| 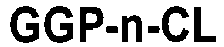 |
| 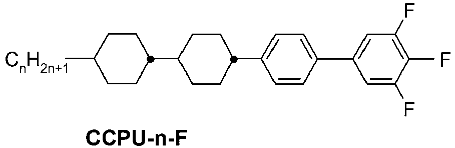 |
| 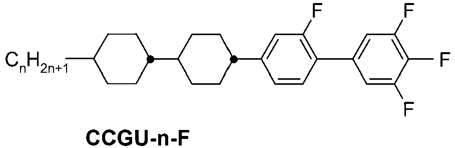 |
| 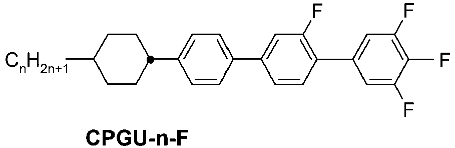 |
| 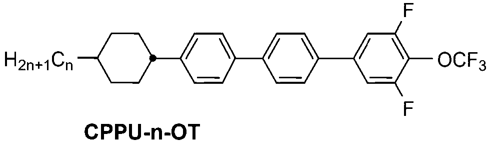 |
| 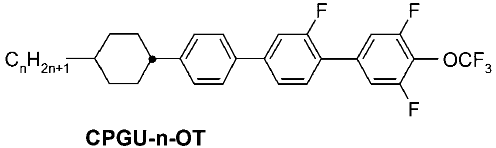 |
| 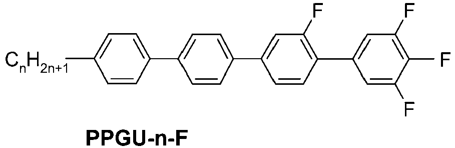 |
| 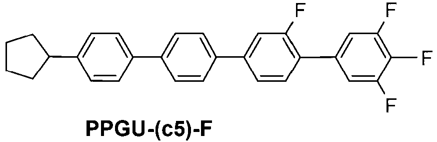 |
| 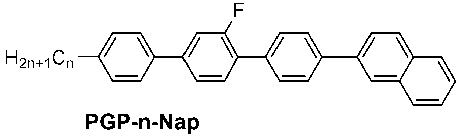 |
| 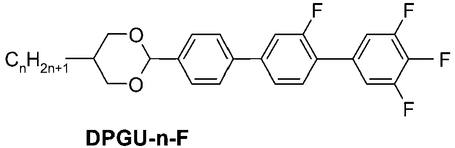 |
| 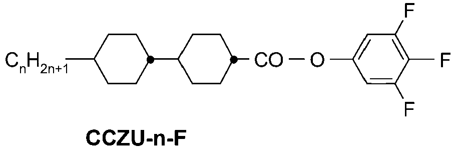 |
| 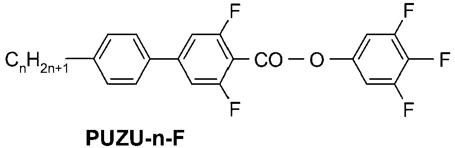 |
| 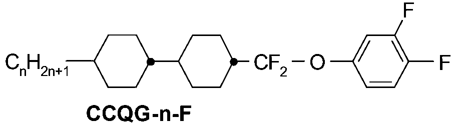 |
| 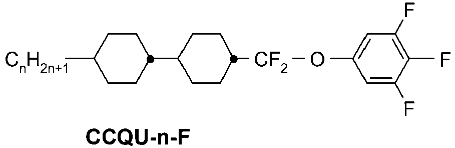 |
| 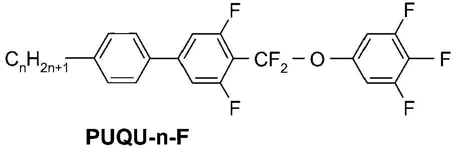 |
| 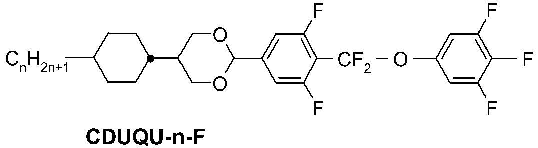 |
| 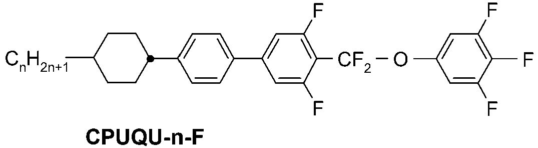 |
| 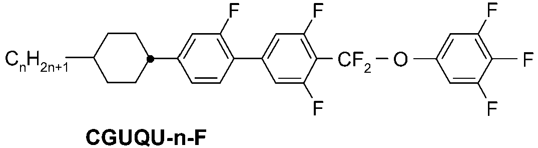 |
| 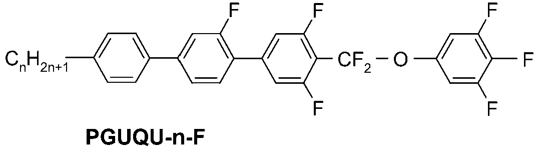 |
| 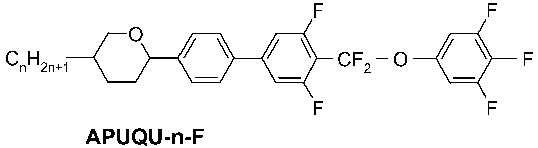 |
| 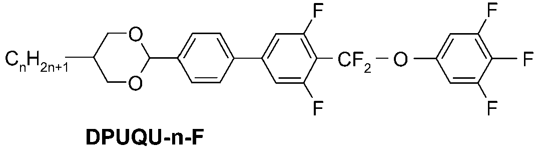 |
| 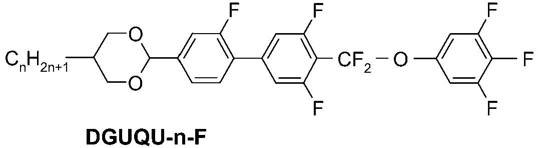 |
| 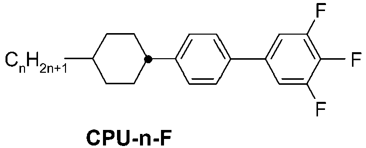 |
| 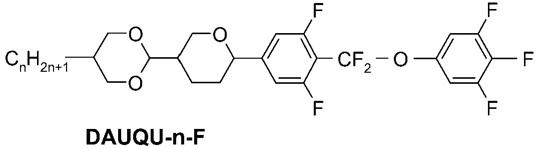 |
| 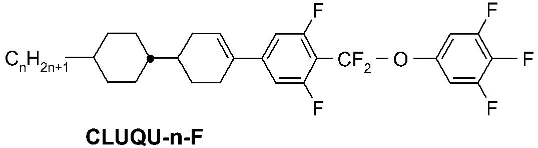 |
| 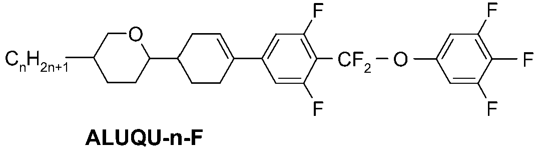 |
| 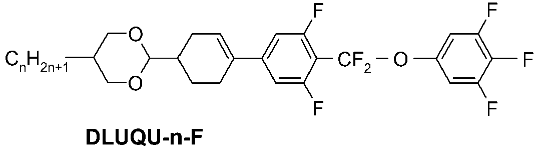 |
| 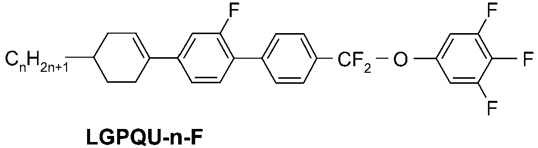 |
| Exemplary, preferred dielectrically neutral compounds |
| 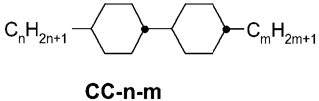 |
| 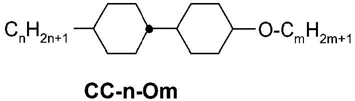 |
| 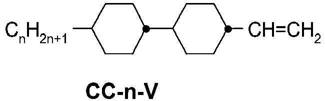 |
| 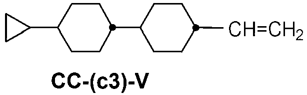 |
| 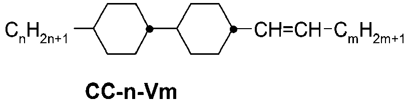 |
| 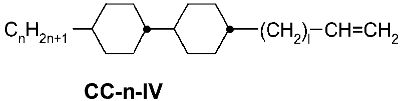 |
| 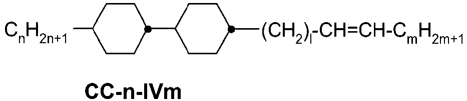 |
| 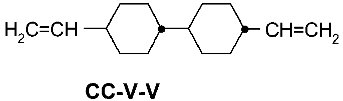 |
| 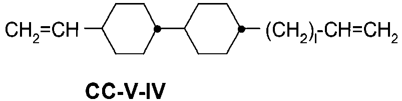 |
| 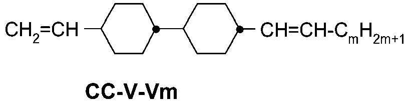 |
| 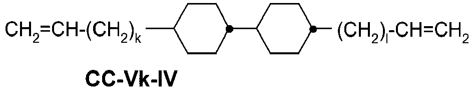 |
| 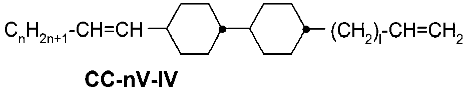 |
| 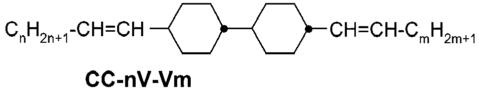 |
| 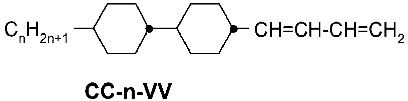 |
| 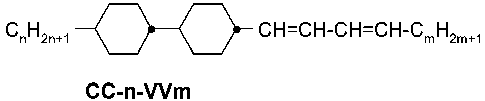 |
| 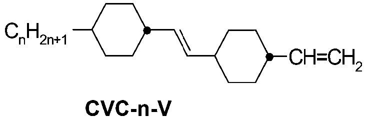 |
| 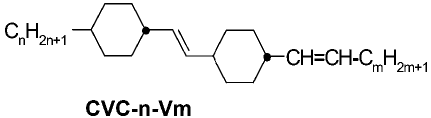 |
| 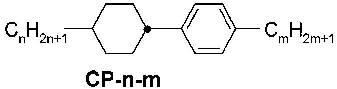 |
| 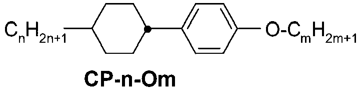 |
| 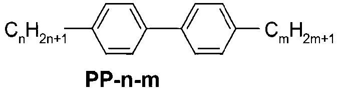 |
| 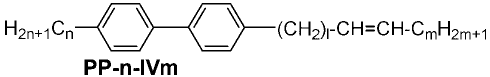 |
| 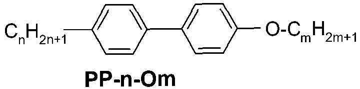 |
| 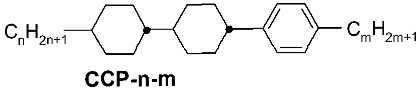 |
| 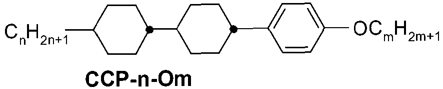 |
| 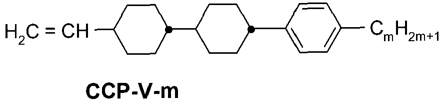 |
| 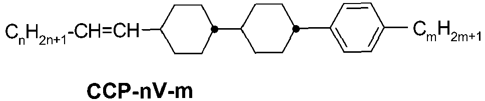 |
| 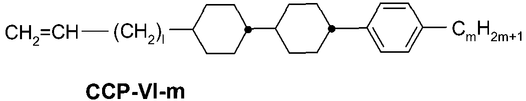 |
| 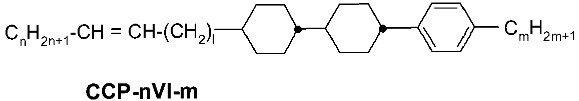 |
| 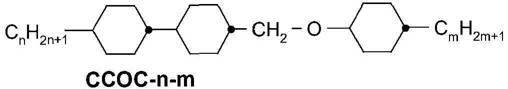 |
| 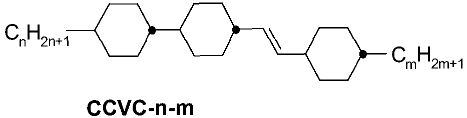 |
| 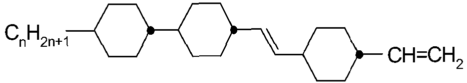 |
| 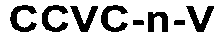 |
| 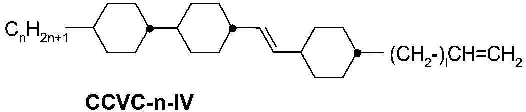 |
| 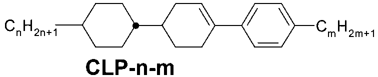 |
| 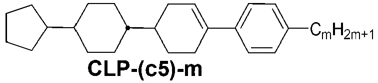 |
| 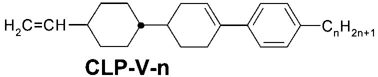 |
| 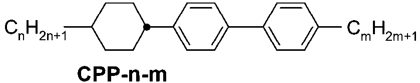 |
| 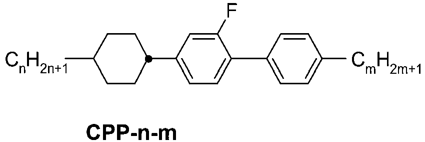 |
| 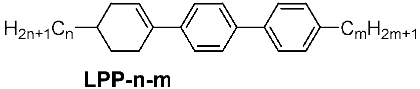 |
| 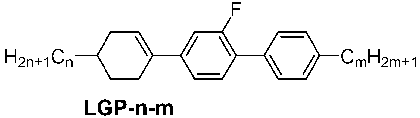 |
| 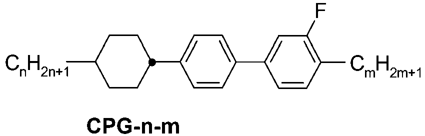 |
| 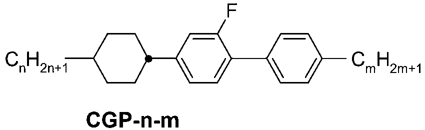 |
| 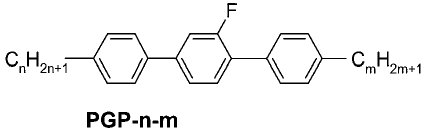 |
| 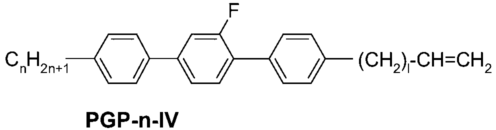 |
| 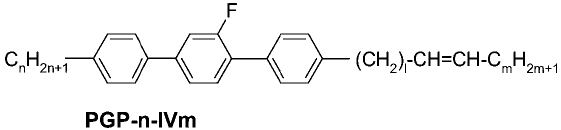 |
| 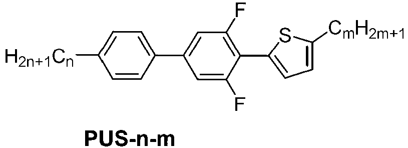 |
| 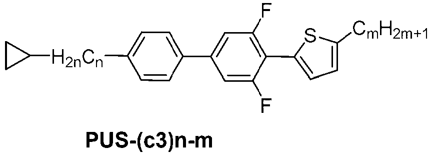 |
| 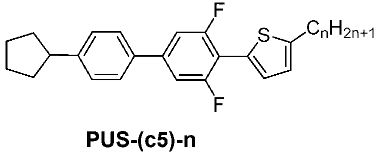 |
| 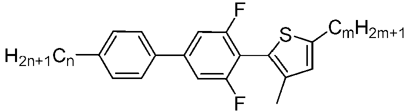 |
| 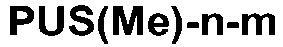 |
| 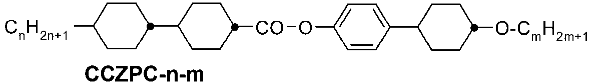 |
| 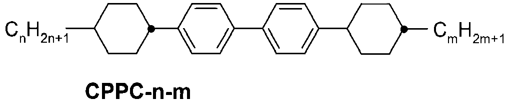 |
| 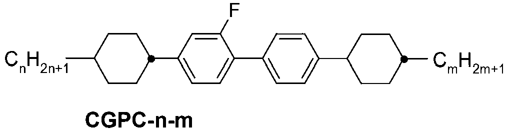 |
| 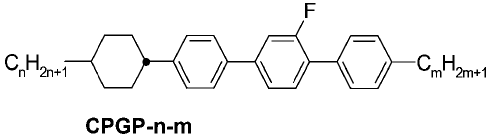 |
| 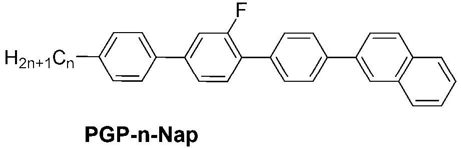 |
| 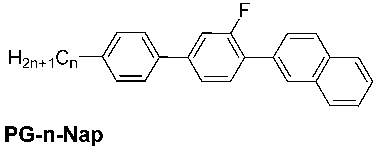 |
| Exemplary compounds having a high ε_{⊥}: |
| 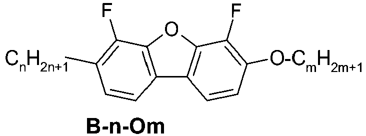 |
| 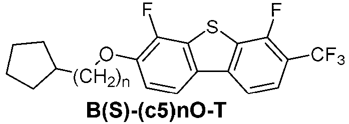 |
| 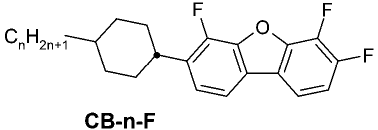 |
| 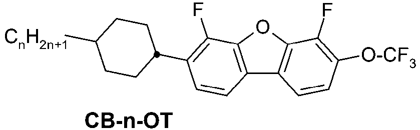 |
| 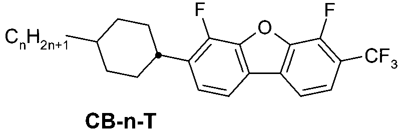 |
| 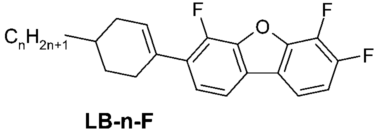 |
| 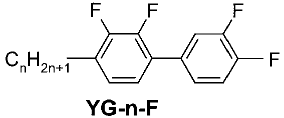 |
| 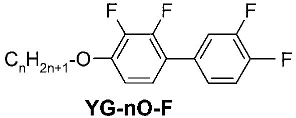 |
| Exemplary, preferred dielectrically negative compounds |
| 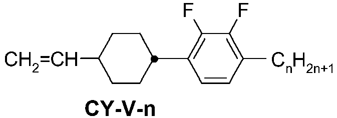 |
| 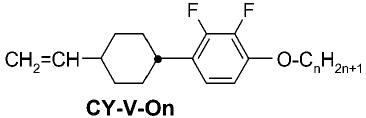 |
| 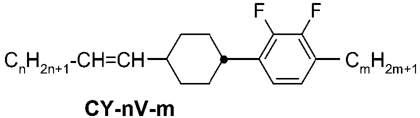 |
| 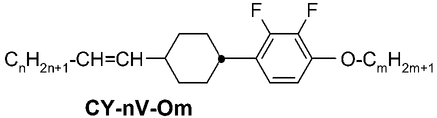 |
| 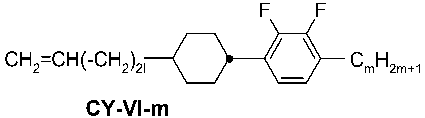 |
| 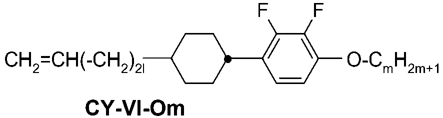 |
| 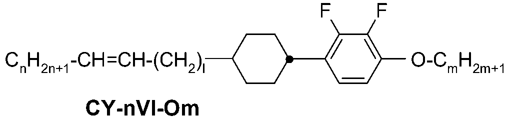 |
| 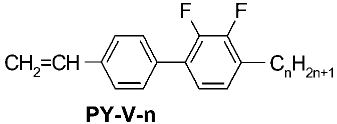 |
| 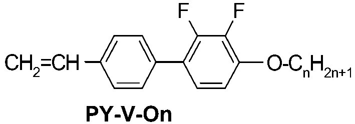 |
| 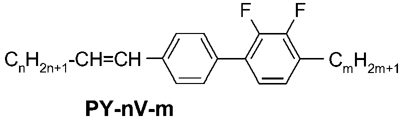 |
| 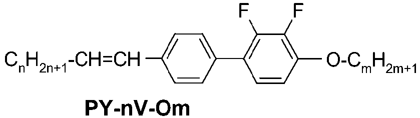 |
| 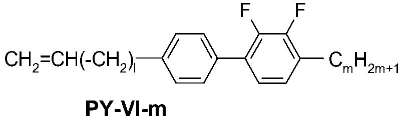 |
| 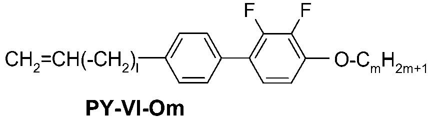 |
| 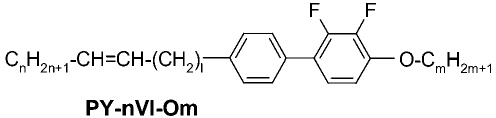 |
| 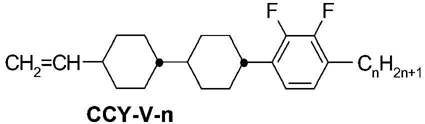 |
| 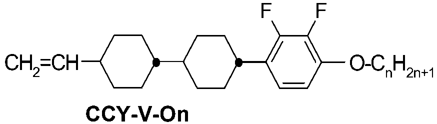 |
| 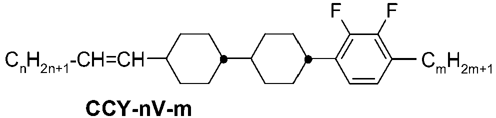 |
| 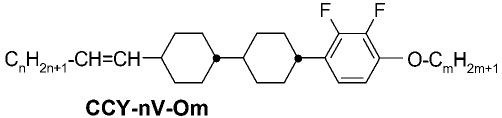 |
| 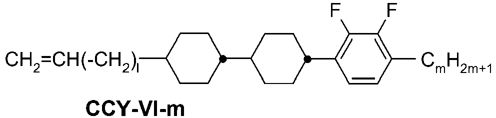 |
| 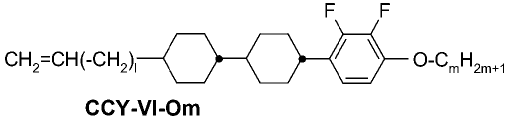 |
| 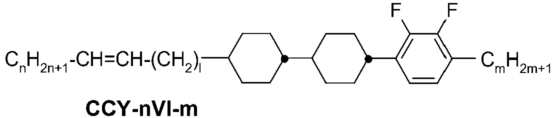 |
| 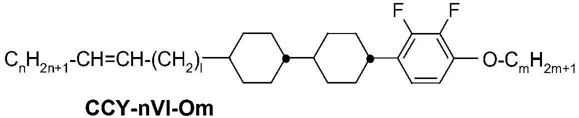 |
| 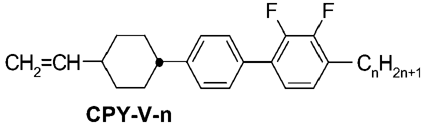 |
| 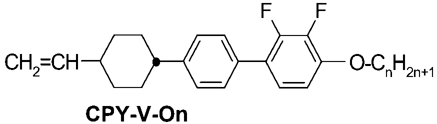 |
| 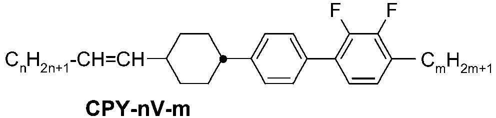 |
| 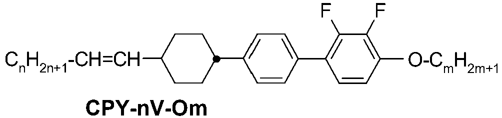 |
| 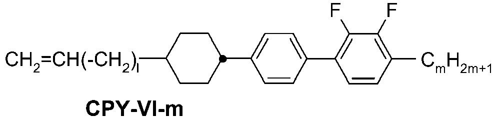 |
| 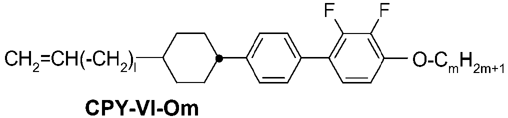 |
| 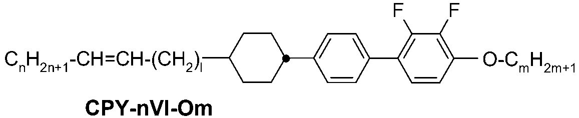 |
| 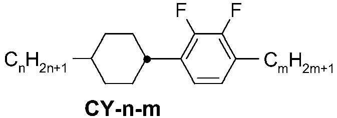 |
| 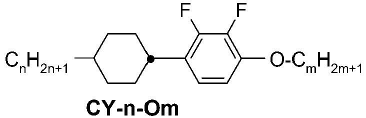 |
| 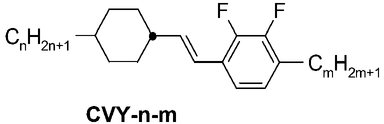 |
| 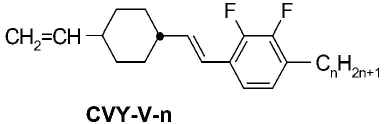 |
| 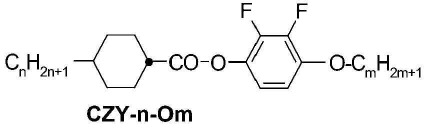 |
| 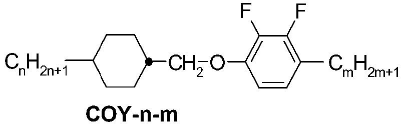 |
| 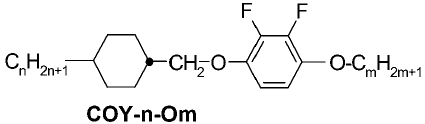 |
| 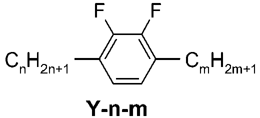 |
| 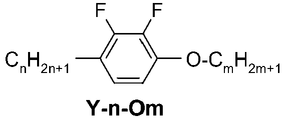 |
| 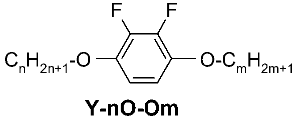 |
| 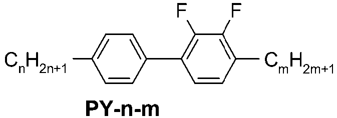 |
| 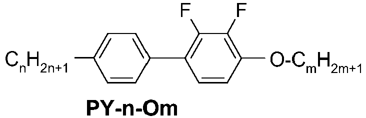 |
| 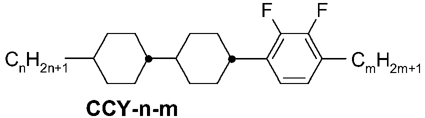 |
| 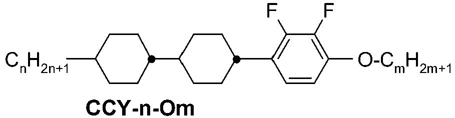 |
| 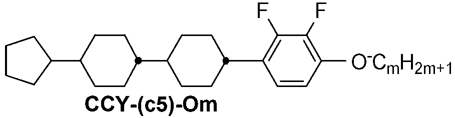 |
| 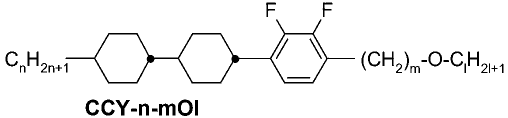 |
| 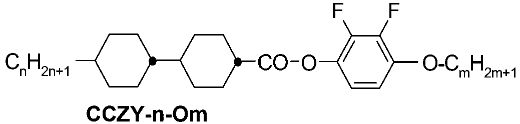 |
| 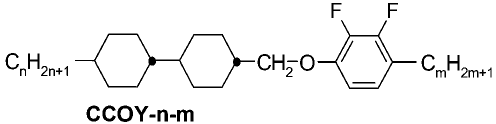 |
| 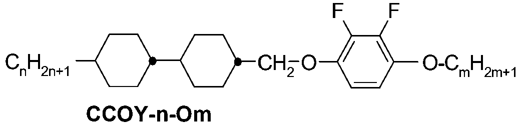 |
| 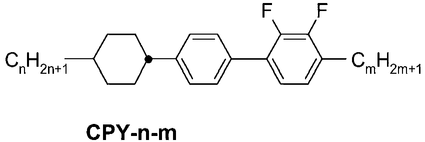 |
| 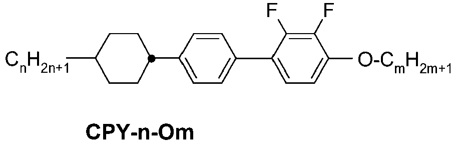 |
| 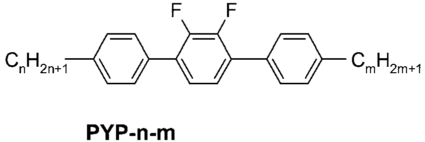 |
| 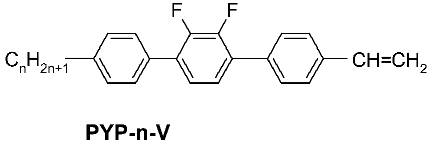 |
| 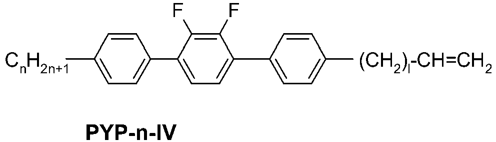 |
| 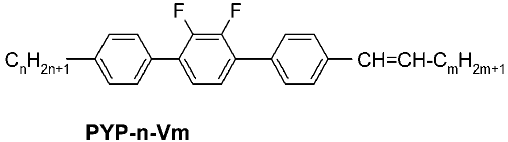 |
| 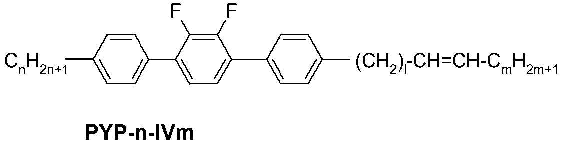 |
| 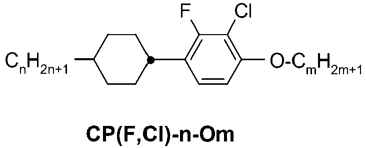 |
| 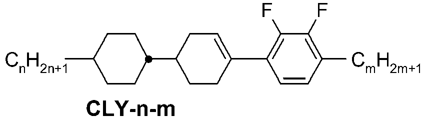 |
| 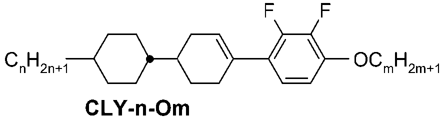 |

Table E shows chiral dopants which are preferably employed in the mixtures according to the invention.

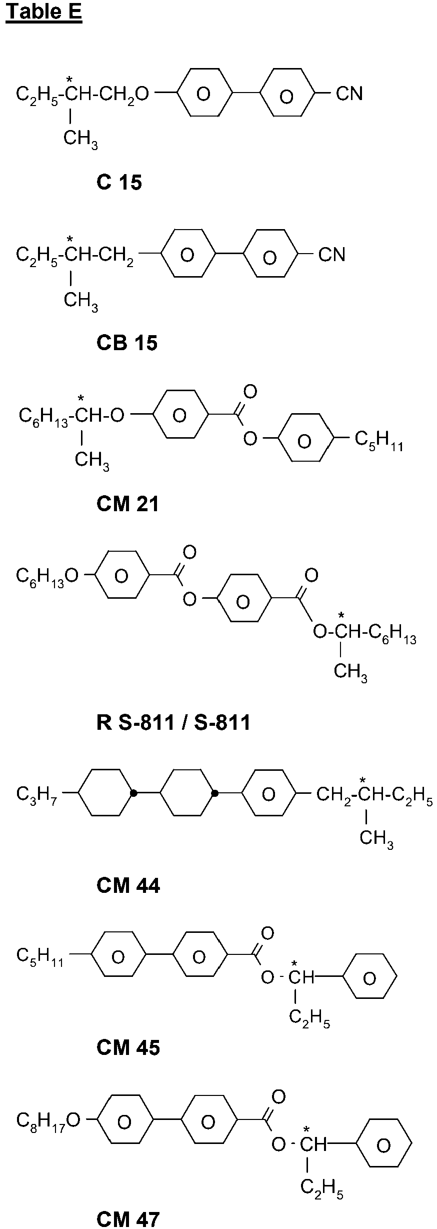

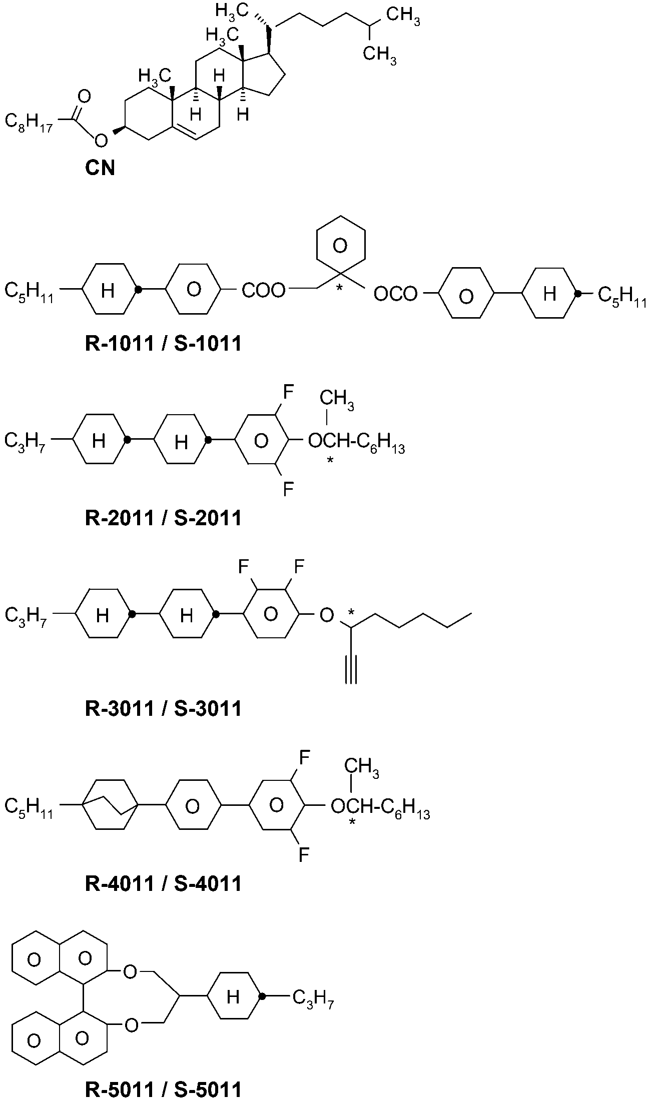

In a preferred embodiment of the present invention, the media according to the invention comprise one or more compounds selected from the group of the compounds from Table E.

Table F shows stabilisers which can preferably be employed in addition in the mixtures according to the invention. The parameter n here denotes an integer in the range from 1 to 12. In particular, the phenol derivatives shown can be employed as additional stabilisers since they act as antioxidants.

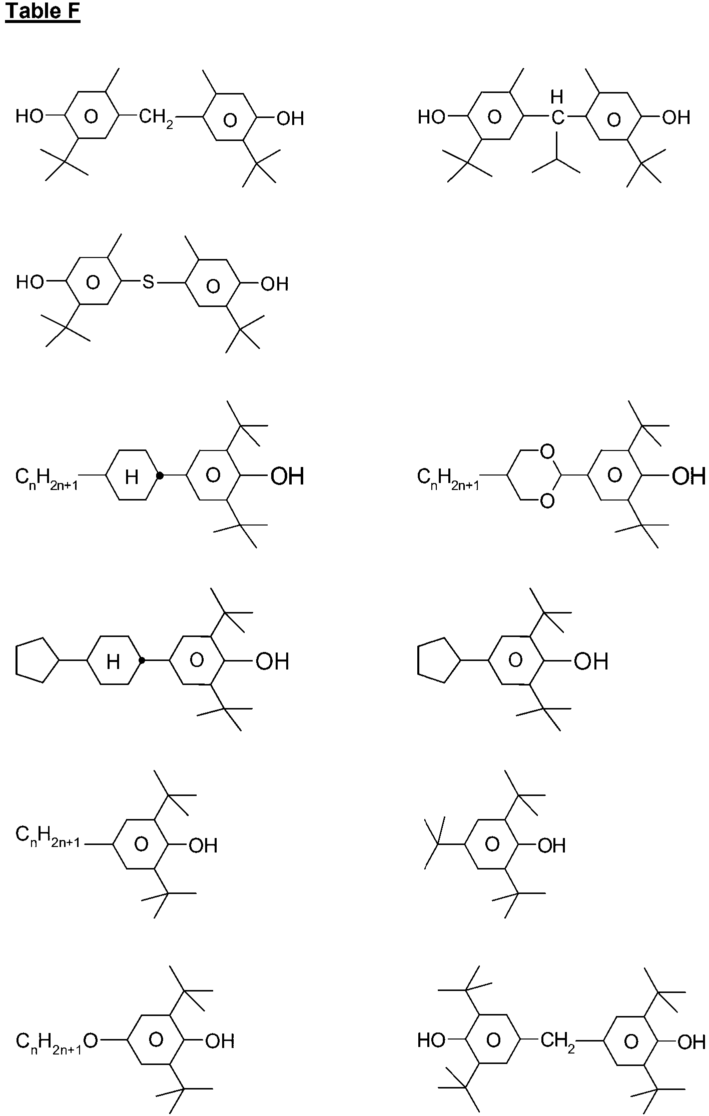

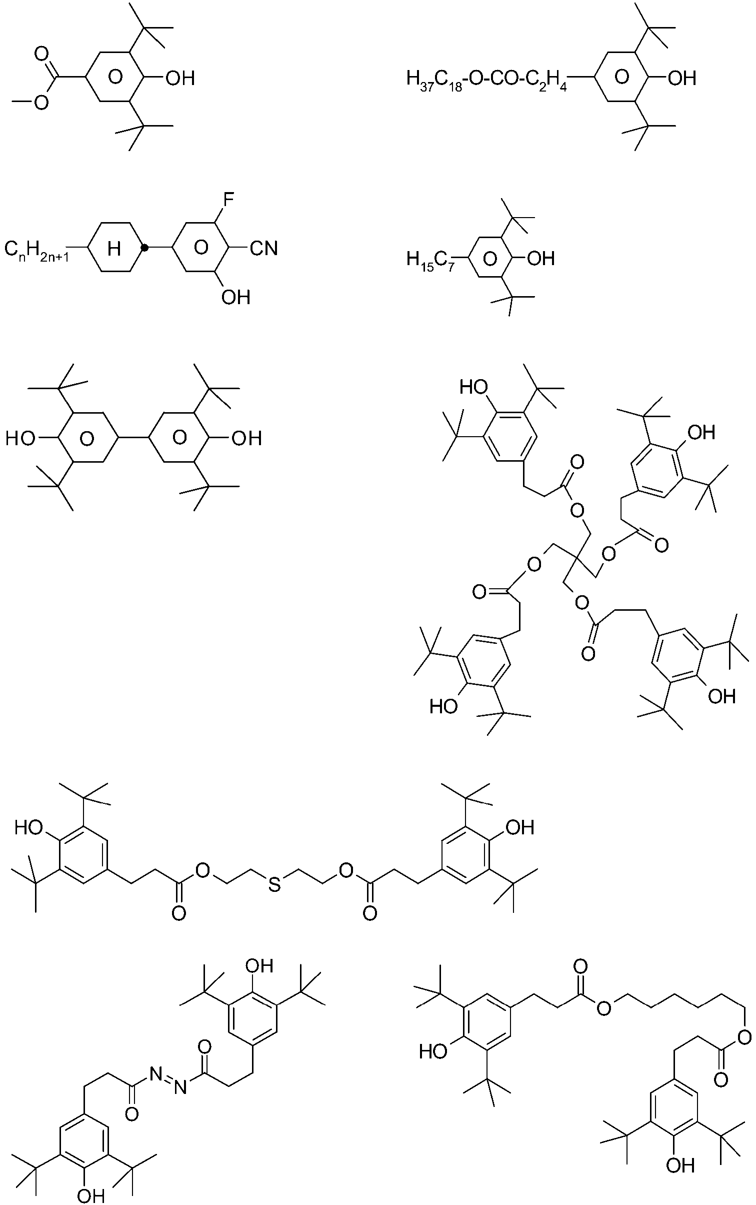

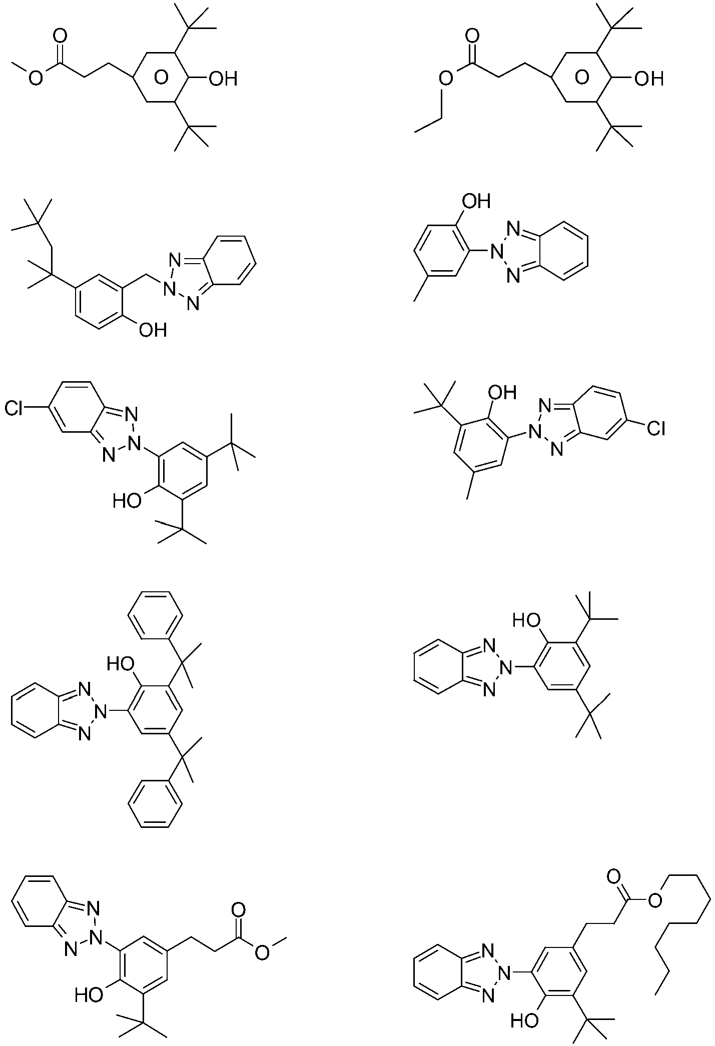

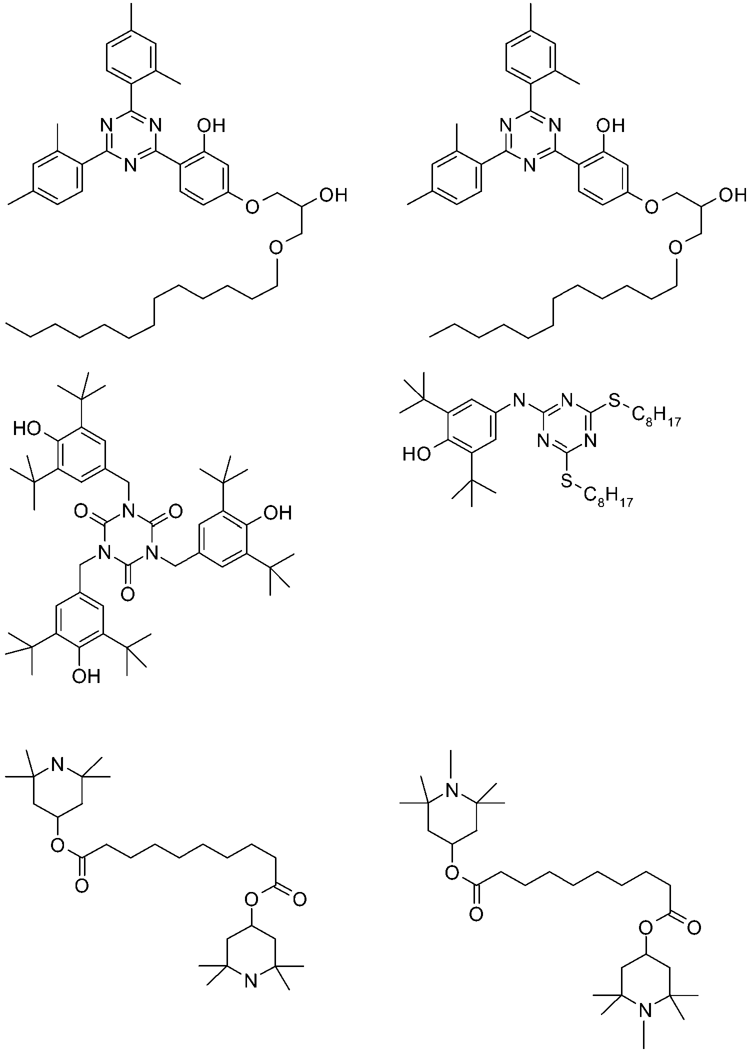

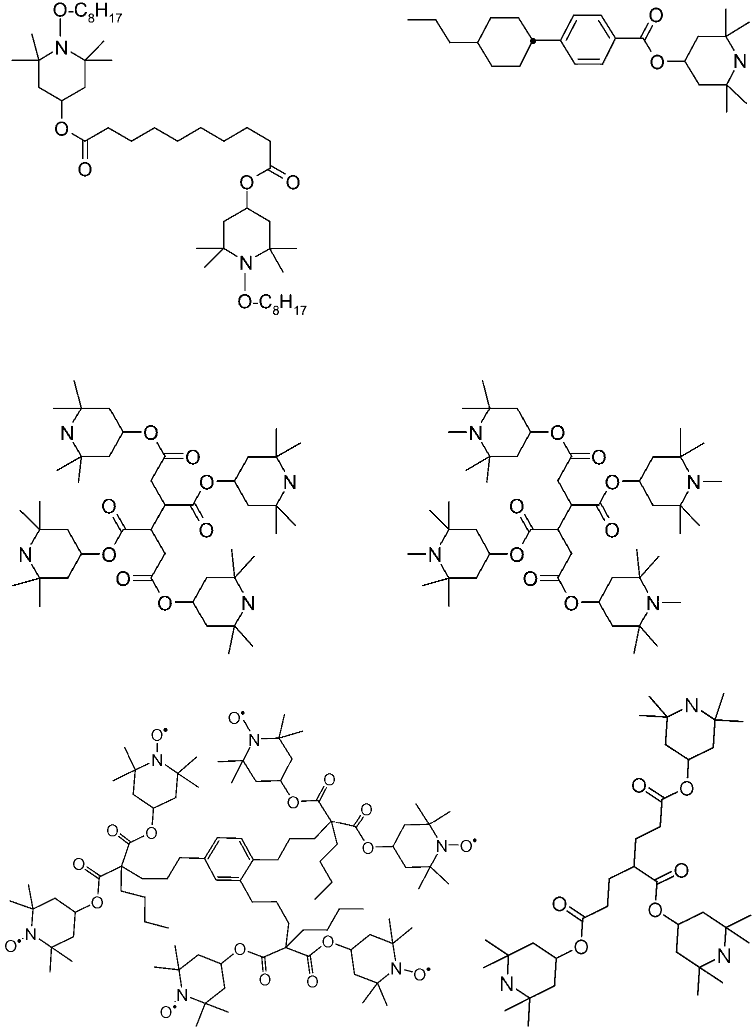

In a preferred embodiment of the present invention, the media according to the invention comprise one or more compounds selected from the group of the compounds from Table F, in particular one or more compounds selected from the group of the compounds of the following two formulae

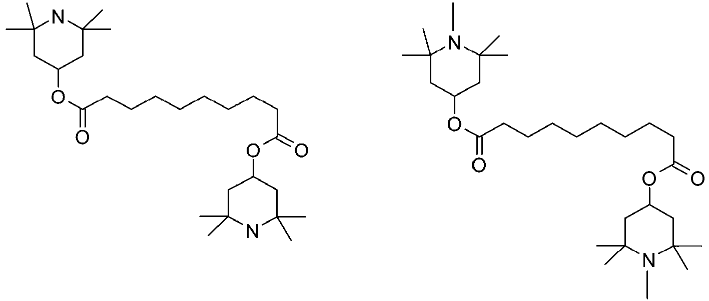

### Examples

The following examples explain the present invention without restricting it in any way. However, the physical properties make it clear to the person skilled in the art what properties can be achieved and in what ranges they can be modified. In particular, the combination of the various properties which can preferably be achieved is thus well defined for the person skilled in the art.

### Synthesis Examples

### Synthesis Example 1

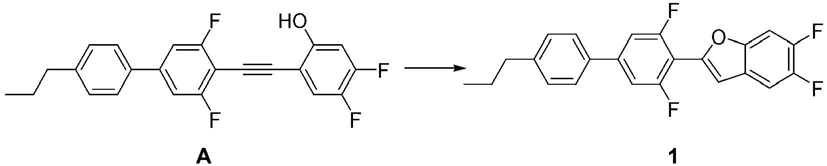

Compound **1** is prepared as follows.

50.5 g (0.13 mol) of alkyne A are placed in 600 ml of anhydrous tetrahydrofuran in a 2000ml four-neck flask under nitrogen and cooled to -18°C with good stirring using an ice/salt bath. 50.0 ml (0.13 mol) of n-butyl-lithium (2.7m in heptane) are slowly added at temperatures below -15°C over 60 minutes, followed by stirring for 20 minutes. Then, 55.0 ml (0.04 mol) of zinc chloride (0.7m in THF) and 18.0 g (0.08 mol) of anhydrous zinc bromide are added, and the mixture is allowed to come to room temperature over 30 minutes. 1000 ml of anhydrous toluene is added, and the mixture is heated to 120°C with distillation of the tetrahydrofuran and stirred well for an additional 90 minutes. A TLC check indicates substantial conversion at this point, after which the mixture is cooled to room temperature. The mixture is completely evaporated at 35°C using a rotary evaporator, and the residue of 81.9 g is dissolved in 500ml of petroleum ether and filtered through 1200 ml of silica gel. The solubility is found to be poor, so further rinsing is done with warm petroleum ether. A clean total fraction of 50.4 g is obtained, which is largely dissolved in 2800 ml of petroleum ether and slowly concentrated to 1000 ml. After cooling, standing overnight at 4°C, filtration, and air-drying, 47.9 g of white crystals remain, which are then recrystallized as described. After filtration and air-drying, 47.0 g (93%) of white crystals remain, which are found to be pure by DC and GC-MS.

¹H NMR (500 MHz, DMSO) δ 7.97 (dd, *J* = 10.4, 6.6 Hz, 1H), 7.87 - 7.73 (m, 3H), 7.69 (d, *J* = 11.0 Hz, 2H), 7.45 - 7.25 (m, 3H), 2.62 (t, *J =* 7.6 Hz, 2H), 1.63 (h, *J* = 7.4 Hz, 2H), 0.92 (t, *J* = 7.3 Hz, 3H)
Compound **(1)** has the following phase characteristics:
K 124 SmA 161 N 182.6 I
Δε: 23
Δn: 0.33
γ₁: 215 mPas

### Synthesis Example 2

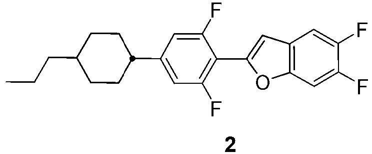

Compound **(2)** is prepared in analogy to Synthesis Example 1.

Compound **(2)** has the following phase characteristics:
K 114 N 168 I
Δε: 19
Δn: 0.203
γ₁: 299 mPas

### Further Compound Examples

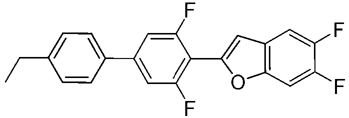

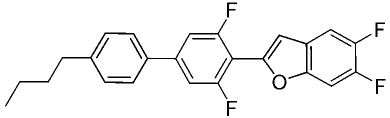

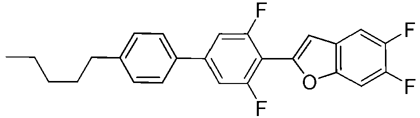

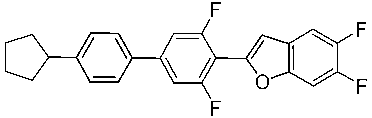

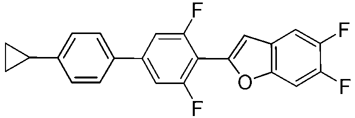

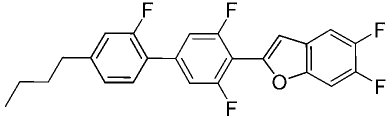

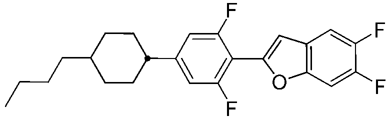

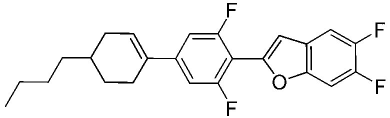

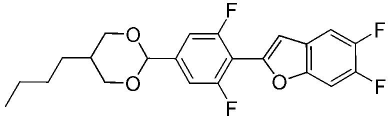

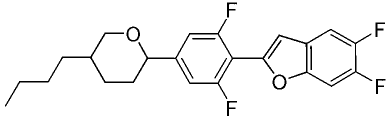

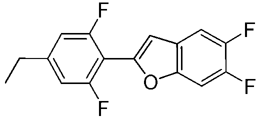

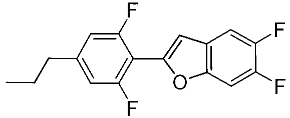

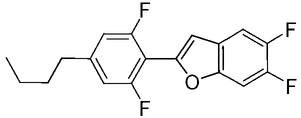

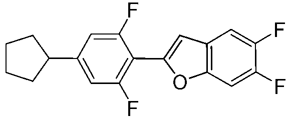

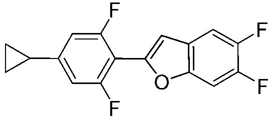

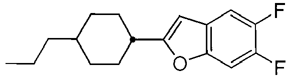

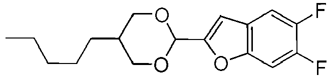

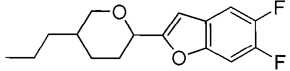

In the following table the following abbreviations for the end groups are used

| | |
|---|---|
| *c*-C₃H₅ | 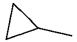 |
| *c*-C₃H₅CH₂ | 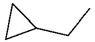 |
| *c*-C₄H₇ | 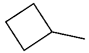 |
| *c*-C₅H₇ | 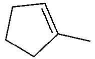 |
| *c*-C₅H₉ | 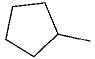 |

### Further exemplary compounds:

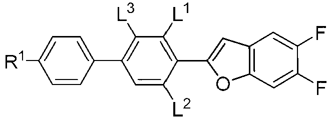

| **No.** | **R¹** | **L¹** | **L²** | **L³** |
|---|---|---|---|---|
| 1. | CH₃ | H | H | H |
| 2. | C₂H₅ | H | H | H |
| 3. | *n*-C₃H₇ | H | H | H |
| 4. | *n*-C₄H₉ | H | H | H |
| 5. | *n*-C₅H₁₁ | H | H | H |
| 6. | *n*-C₆H₁₃ | H | H | H |
| 7. | *n-*C₇H₁₅ | H | H | H |
| 8. | *n*-C₈H₁₇ | H | H | H |
| 9. | *c*-C₃H₅ | H | H | H |
| 10. | *c*-C₃H₅-CH₂ | H | H | H |
| 11. | *c*-C₄H₇ | H | H | H |
| 12. | *c*-C₅H₇ | H | H | H |
| 13. | *c*-C₅H₉ | H | H | H |
| 14. | *c*-C₅H₉CH₂ | H | H | H |
| 15. | CH₂=CH | H | H | H |
| 16. | CH₃CH=CH | H | H | H |
| 17. | CH₂=CH(CH₂)₂ | H | H | H |
| 18. | CH₃CH=CH(CH₂)₂ | H | H | H |
| 19. | CH₃ | F | H | H |
| 20. | C₂H₅ | F | H | H |
| 21. | *n*-C₃H₇ | F | H | H |
| 22. | *n*-C₄H₉ | F | H | H |
| 23. | *n*-C₅H₁₁ | F | H | H |
| 24. | *n*-C₆H₁₃ | F | H | H |
| 25. | *n-*C₇H₁₅ | F | H | H |
| 26. | *n*-C₈H₁₇ | F | H | H |
| 27. | *c*-C₃H₅ | F | H | H |
| 28. | *c*-C₃H₅-CH₂ | F | H | H |
| 29. | *c*-C₄H₇ | F | H | H |
| 30. | *c*-C₅H₇ | F | H | H |
| 31. | *c*-C₅H₉ | F | H | H |
| 32. | *c*-C₅H₉CH₂ | F | H | H |
| 33. | CH₂=CH | F | H | H |
| 34. | CH₃CH=CH | F | H | H |
| 35. | CH₂=CH(CH₂)₂ | F | H | H |
| 36. | CH₃CH=CH(CH₂)₂ | F | H | H |
| 37. | CH₃ | F | F | H |
| 38. | C₂H₅ | F | F | H |
| 39. | *n*-C₃H₇ | F | F | H |
| 40. | *n*-C₄H₉ | F | F | H |
| 41. | *n*-C₅H₁₁ | F | F | H |
| 42. | *n*-C₆H₁₃ | F | F | H |
| 43. | *n-*C₇H₁₅ | F | F | H |
| 44. | *n*-C₈H₁₇ | F | F | H |
| 45. | *c*-C₃H₅ | F | F | H |
| 46. | *c*-C₃H₅-CH₂ | F | F | H |
| 47. | *c*-C₄H₇ | F | F | H |
| 48. | *c*-C₅H₇ | F | F | H |
| 49. | *c*-C₅H₉ | F | F | H |
| 50. | *c*-C₅H₉CH₂ | F | F | H |
| 51. | CH₂=CH | F | F | H |
| 52. | CH₃CH=CH | F | F | H |
| 53. | CH₂=CH(CH₂)₂ | F | F | H |
| 54. | CH₃CH=CH(CH₂)₂ | F | F | H |
| 55. | CH₃ | F | H | F |
| 56. | C₂H₅ | F | H | F |
| 57. | *n*-C₃H₇ | F | H | F |
| 58. | *n*-C₄H₉ | F | H | F |
| 59. | *n*-C₅H₁₁ | F | H | F |
| 60. | *n*-C₆H₁₃ | F | H | F |
| 61. | *n-*C₇H₁₅ | F | H | F |
| 62. | *n*-C₈H₁₇ | F | H | F |
| 63. | *c*-C₃H₅ | F | H | F |
| 64. | *c*-C₃H₅-CH₂ | F | H | F |
| 65. | *c*-C₄H₇ | F | H | F |
| 66. | *c*-C₅H₇ | F | H | F |
| 67. | *c*-C₅H₉ | F | H | F |
| 68. | *c*-C₅H₉CH₂ | F | H | F |
| 69. | CH₂=CH | F | H | F |
| 70. | CH₃CH=CH | F | H | F |
| 71. | CH₂=CH(CH₂)₂ | F | H | F |
| 72. | CH₃CH=CH(CH₂)₂ | F | H | F |

### Mixture Examples

In the following exemplary mixtures are disclosed. All % values are % by weight.

### Mixture Example 1

The following mixture (M-1) is prepared and investigated.

| Compound | % | Properties | |
|---|---|---|---|
| CC-3-2V1 | 16 | T(N,I) [°C]: | 97.5 |
| CC-3-V | 29.25 | Δn [589 nm, 20°C]: | 0.108 |
| CC-3-V1 | 12 | nₑ [589 nm, 20°C]: | 1.593 |
| CC-4-V1 | 5.0 | nₒ [589 nm, 20°C]: | 1.486 |
| CCP-3-1 | 13 | Δε [1 kHz, 20°C]: | 3.9 |
| DLGU-3-F | 11.25 | ε_{∥} [1 kHz, 20°C]: | 6.5 |
| Compound **1** | 2.5 | ε_{⊥} [1 kHz, 20°C]: | 2.6 |
| PGS-2-1 | 5.5 | γ₁ [mPa s, 20°C]: | 78 |
| PP-1-2V1 | 3.0 | K₁₁ [pN, 20°C]: | 21.7 |
| Σ | 100.0 | K₃₃ [pN, 20°C]: | 20.3 |
| | | V₀ [V, 20°C] | 2.49 |

This mixture has an advantageously high average elastic constant K_{avg} (17.6 pN) and moderate optical anisotropy Δn. Response time parameter γ₁ /K₁₁ is also low.

### Mixture Examples 1.1 to 1.4

Alternatively, 0.05 % of the compounds of one of the following formulae 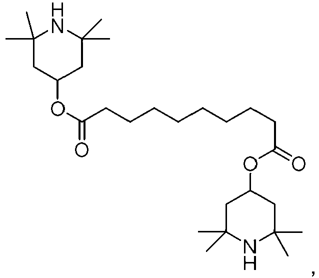 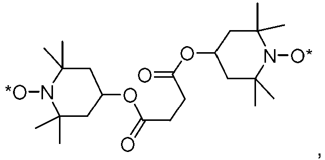 wherein the two O atoms bonded to the N atoms indicate radicals, and 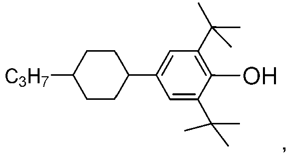 and 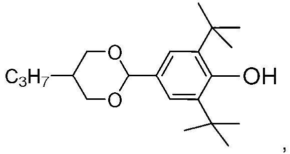 respectively
are added to the mixture M-1. The resultant mixtures M-1.1, M-1.2, M-1.3 and M-1.4 respectively are characterized by an improved stability against severe conditions, especially against exposure to light.

### Mixture Example 2

The following mixture (M-2) is prepared and investigated.

| Compound | % | Properties | |
|---|---|---|---|
| CC-3-2V1 | 14 | T(N,I) [°C]: | 104 |
| CC-3-V | 29.75 | Δn [589 nm, 20°C]: | 0.1068 |
| CC-3-V1 | 10 | nₑ [589 nm, 20°C]: | 15.930 |
| CCP-3-1 | 9.0 | nₒ [589 nm, 20°C]: | 14.862 |
| CDUQU-3-F | 10.5 | Δε [1 kHz, 20°C]: | 3.9 |
| CLP-V-1 | 8.0 | ε_{∥} [1 kHz, 20°C]: | 6.9 |
| CLY-3-O2 | 4.75 | ε_{⊥} [1 kHz, 20°C]: | 3.0 |
| DPGU-4-F | 3.0 | γ₁ [mPa s, 20°C]: | 88 |
| Compound **1** | 3.0 | K₁₁ [pN, 20°C]: | 21.6 |
| CP-3-O2 | 1.0 | K₃₃ [pN, 20°C]: | 20.5 |
| PGS-2-1 | 7.0 | V₀ [V, 20°C] | 2.48 |
| Σ | 100.0 | | |

This mixture has an advantageously high average elastic constant K_{avg} (17.6 pN) and moderate optical anisotropy Δn. Response time parameter γ₁ /K₁₁ is also low.

### Mixture Example 3

The following mixture (M-3) is prepared and investigated.

| Compound | % | Properties | |
|---|---|---|---|
| CC-3-2V1 | 14 | T(N,I) [°C]: | 98.5 |
| CC-3-V | 30.6 | Δn [589 nm, 20°C]: | 0.108 |
| CC-3-V1 | 10 | nₑ [589 nm, 20°C]: | 15.950 |
| CCP-3-1 | 9.0 | nₒ [589 nm, 20°C]: | 14.870 |
| CDUQU-3-F | 9.5 | Δε [1 kHz, 20°C]: | 3.7 |
| CLP-V-1 | 8.0 | ε_{∥} [1 kHz, 20°C]: | 6.5 |
| CLY-3-O2 | 1.9 | ε_{⊥} [1 kHz, 20°C]: | 2.8 |
| DPGU-4-F | 3.0 | γ₁ [mPa s, 20°C]: | 77 |
| Compound 1 | 3.0 | K₁₁ [pN, 20°C]: | 20.8 |
| CP-3-O2 | 1.0 | K₃₃ [pN, 20°C]: | 19.6 |
| PGS-2-1 | 7.0 | V₀ [V, 20°C] | 2.48 |
| PP-1-2V1 | 3.0 | | |
| Σ | 100.0 | | |

This mixture has an advantageously high average elastic constant K_{avg} (16.9 pN) and moderate optical anisotropy Δn. Response time parameter γ₁ /K₁₁ is also low.

### Mixture Example 4

The following mixture (M-4) is prepared and investigated.

| Compound | % | Properties | |
|---|---|---|---|
| CC-3-2V1 | 14 | T(N,I) [°C]: | 98.5 |
| CC-3-V | 29.5 | Δn [589 nm, 20°C]: | 0.1122 |
| CC-3-V1 | 11 | nₑ [589 nm, 20°C]: | 15.996 |
| CC-4-V1 | 3 | nₒ [589 nm, 20°C]: | 14.874 |
| CCP-3-1 | 11 | Δε [1 kHz, 20°C]: | 4.0 |
| CDUQU-3-F | 0.5 | ε_{∥} [1 kHz, 20°C]: | 6.7 |
| CLP-V-1 | 3.75 | ε_{⊥} [1 kHz, 20°C]: | 2.7 |
| CLY-3-O2 | 1 | γ₁ [mPa s, 20°C]: | 82 |
| DLGU-3-F | 11 | K₁₁ [pN, 20°C]: | 21.8 |
| Compound **1** | 5 | K₃₃ [pN, 20°C]: | 20.5 |
| CP-3-O2 | 1 | V₀ [V, 20°C] | 2.46 |
| PGS-2-1 | 5.5 | | |
| PP-1-2V1 | 3.75 | | |
| Σ | 100.0 | | |

This mixture has an advantageously high dielectric K_{avg} (17.3 pN) and moderate optical anisotropy Δn. Response time parameter γ₁ /K₁₁ is also low.

### Mixture Example 5

The following mixture (M-5) is prepared and investigated.

| Compound | % | Properties | |
|---|---|---|---|
| APUQU-2-F | 4.5 | T(N,I) [°C]: | 83.5 |
| CC-3-V | 28.35 | Δn [589 nm, 20°C]: | 0.1147 |
| CC-3-V1 | 5.85 | nₑ [589 nm, 20°C]: | 16.042 |
| CCP-3-3 | 5.4 | nₒ [589 nm, 20°C]: | 14.895 |
| CCP-V-1 | 10.8 | Δε [1 kHz, 20°C]: | 3.1 |
| CCP-V2-1 | 10.8 | ε_{∥} [1 kHz, 20°C]: | 7.4 |
| CPGP-5-2 | 1.8 | ε_{⊥} [1 kHz, 20°C]: | 10.4 |
| PP-1-2V1 | 4.5 | γ₁ [mPa s, 20°C]: | 73 |
| PUQU-3-F | 18 | K₁₁ [pN, 20°C]: | 15.4 |
| Compound **1** | 10 | K₃₃ [pN, 20°C]: | 16.2 |
| | | V₀ [V, 20°C] | 1.47 |
| Σ | 100.0 | | |

### Comparative Mixture Example 1

The following mixture (C-1) is prepared and investigated.

| Compound | % | Properties | |
|---|---|---|---|
| CC-3-2V1 | 14 | T(N,I) [°C]: | 95 |
| CC-3-V | 25.25 | Δn [589 nm, 20°C]: | 0.1083 |
| CC-3-V1 | 11 | nₑ [589 nm, 20°C]: | 15.960 |
| CC-4-V1 | 3 | nₒ [589 nm, 20°C]: | 14.877 |
| CCP-3-1 | 11 | Δε [1 kHz, 20°C]: | 3.9 |
| CDUQU-3-F | 4.5 | ε_{∥} [1 kHz, 20°C]: | 6.6 |
| CLP-V-1 | 5 | ε_{⊥} [1 kHz, 20°C]: | 2.7 |
| CLY-3-O2 | 1 | γ₁ [mPa s, 20°C]: | 78 |
| DLGU-3-F | 10 | K₁₁ [pN, 20°C]: | 21.5 |
| CP-3-O2 | 1 | K₃₃ [pN, 20°C]: | 20 |
| PGS-2-1 | 5.5 | V₀ [V, 20°C] | 2.5 |
| PP-1-2V1 | 8.75 | | |
| Σ | 100.0 | | |

### Comparative Mixture Example 2

The following mixture (C-2) is prepared and investigated.

| Compound | % | Properties | |
|---|---|---|---|
| APUQU-2-F | 5 | T(N,I) [°C]: | 78.7 |
| CC-3-V | 31.5 | Δn [589 nm, 20°C]: | 0.100 |
| CC-3-V1 | 6.5 | nₑ [589 nm, 20°C]: | 15.87 |
| CCP-3-3 | 6 | nₒ [589 nm, 20°C]: | 14.87 |
| CCP-V-1 | 12 | Δε [1 kHz, 20°C]: | 6.0 |
| CCP-V2-1 | 12 | ε_{∥} [1 kHz, 20°C]: | 9.0 |
| CPGP-5-2 | 2 | ε_{⊥} [1 kHz, 20°C]: | 3.0 |
| PP-1-2V1 | 5 | γ₁ [mPa s, 20°C]: | 65 |
| PUQU-3-F | 20 | K₁₁ [pN, 20°C]: | 13.3 |
| Σ | 100.0 | K₃₃ [pN, 20°C]: | 15.7 |
| | | V₀ [V, 20°C] | 1.58 |

### Mixture Example 6

The following mixture (M-6) is prepared and investigated.

| Compound | % | Properties | |
|---|---|---|---|
| APUQU-2-F | 4.5 | T(N,I) [°C]: | 83.5 |
| CC-3-V | 28.35 | Δn [589 nm, 20°C]: | 0.114 |
| CC-3-V1 | 5.85 | nₑ [589 nm, 20°C]: | 16.04 |
| CCP-3-3 | 5.4 | nₒ [589 nm, 20°C]: | 14.90 |
| CCP-V-1 | 10.8 | Δε [1 kHz, 20°C]: | 7.4 |
| CCP-V2-1 | 10.8 | ε_{∥} [1 kHz, 20°C]: | 10.4 |
| CPGP-5-2 | 1.8 | ε_{⊥} [1 kHz, 20°C]: | 3.1 |
| PP-1-2V1 | 4.5 | γ₁ [mPa s, 20°C]: | 73 |
| PUQU-3-F | 18 | K₁₁ [pN, 20°C]: | 15.4 |
| Compound **1** | 10 | K₃₃ [pN, 20°C]: | 16.2 |
| Σ | 100.0 | V₀ [V, 20°C] | 1.47 |

**Table 1: Comparison of average elastic constant K_{avg} (scattering parameter) and response time parameter γ₁ /K₁₁ of mixture M-1 and M-6 with their counterparts C-1 and C-2.**

| **Mixture** | **Comment** | **K_{avg}** | **γ₁ /K₁₁** |
|---|---|---|---|
| M-1 | Invention | 17.6 pN | 3.59 mPas/pN |
| C-1 | Comparison | 17.4 pN | 3.63 mPas/pN |
| M-6 | Invention | 13.1 pN | 4.74 mPas/pN |
| C-2 | Comparison | 11.8 pN | 4.88 mPas/pN |

Higher values in the scattering parameter K_{avg} indicate good contrast due to improved dark level. Lower values in the response time parameter γ₁ /K₁₁ indicate fast switching.

**Table 2: Average elastic constant K_{avg} and response time parameter γ₁ /K₁₁ of some mixtures.**

| **Mixture** | **K_{avg}** | **γ₁ /K₁₁** |
|---|---|---|
| M-2 | 17.6 pN | 4.07 mPas/pN |
| M-3 | 16.9 pN | 3.70 mPas/pN |
| M-4 | 17.3 pN | 3.76 mPas/pN |
| M-5 | 13.1 pN | 4.74 mPas/pN |

### Mixture Example 7

The following mixture (M-7) is prepared and investigated.

| Compound | % | Properties | |
|---|---|---|---|
| APUQU-2-F | 4.5 | T(N,I) [°C]: | 86 |
| CC-3-V | 28.35 | nₑ [589 nm, 20°C]: | 1.6008 |
| CC-3-V1 | 5.85 | nₒ [589 nm, 20°C]: | 1.4881 |
| CCP-3-3 | 5.4 | Δn [589 nm, 20°C] | 0.1127 |
| CCP-V-1 | 10.8 | ε_{∥} [1 kHz, 20°C]: | 10.6 |
| CCP-V2-1 | 10.8 | ε_{⊥} [1 kHz, 20°C]: | 3.1 |
| CPGP-5-2 | 1.8 | Δε [1 kHz, 20°C]: | 7.5 |
| PP-1-2V1 | 4.5 | γ₁ [mPa s, 20°C]: | 81 |
| PUQU-3-F | 18 | K₁ [pN, 20°C]: | 14.2 |
| Compound **2** | 10.0 | K₃ [pN, 20°C]: | 17.0 |
| Σ | 100.0 | | |

### Mixture Example 8

The following mixture is prepared and investigated.

| Compound | % | Properties | |
|---|---|---|---|
| CC-3-2V1 | 14.0 | T(N,I) [°C]: | 96.5 |
| CC-3-V | 30.6 | Δn [589 nm, 20°C]: | 0.1073 |
| CC-3-V1 | 10.0 | ε_{∥} [1 kHz, 20°C]: | 6.5 |
| CCP-3-1 | 9.0 | ε_{⊥} [1 kHz, 20°C]: | 2.8 |
| CDUQU-3-F | 7.5 | Δε [1 kHz, 20°C]: | 3.7 |
| CLP-V-1 | 8.0 | γ₁ [mPa s, 20°C]: | |
| CLY-3-O2 | 1.9 | γ₁ [mPa s, 20°C]: | 75 |
| APUQU-3-F | 5.0 | K₁₁ [pN, 20°C]: | 20.2 |
| Compound **1** | 3.0 | K₃₃ [pN, 20°C]: | 19.5 |
| CP-3-O2 | 1.0 | V₀ [V, 20°C]: | |
| PGS-2-1 | 7.0 | LTS bulk [h, -20°C]: | |
| PP-1-2V1 | 3.0 | LTS bulk [h, -30°C]: | |
| Σ | 100.0 | LTS bulk [h, -10°C]: | |

### Mixture Example 9

The following mixture is prepared and investigated.

| Compound | % | Properties | |
|---|---|---|---|
| CC-3-2V1 | 14.0 | T(N,I) [°C]: | 96.5 |
| CC-3-V | 30.6 | Δn [589 nm, 20°C]: | 0.1072 |
| CC-3-V1 | 10.0 | ε_{∥} [1 kHz, 20°C]: | 6.5 |
| CCP-3-1 | 9.0 | ε_{⊥} [1 kHz, 20°C]: | 2.8 |
| CDUQU-3-F | 7.5 | Δε [1 kHz, 20°C]: | 3.7 |
| CLP-V-1 | 8.0 | γ₁ [mPa s, 20°C]: | |
| CLY-3-O2 | 1.9 | γ₁ [mPa s, 20°C]: | 78 |
| APUQU(1)-3-F | 5.0 | K₁₁ [pN, 20°C]: | 20.2 |
| Compound **1** | 3.0 | K₃₃ [pN, 20°C]: | 19.5 |
| CP-3-O2 | 1.0 | V₀ [V, 20°C]: | |
| PGS-2-1 | 7.0 | LTS bulk [h, -20°C]: | |
| PP-1-2V1 | 3.0 | LTS bulk [h, -30°C]: | |
| Σ | 100.0 | LTS bulk [h, -10°C]: | |

### Mixture Example 10

The following mixture is prepared and investigated.

| Compound | % | Properties | |
|---|---|---|---|
| CC-3-2V1 | 14.0 | T(N,I) [°C]: | 96.5 |
| CC-3-V | 30.6 | Δn [589 nm, 20°C]: | 0.1074 |
| CC-3-V1 | 10.0 | ε_{∥} [1 kHz, 20°C]: | 6.5 |
| CCP-3-1 | 9.0 | ε_{⊥} [1 kHz, 20°C]: | 2.8 |
| CDUQU-3-F | 7.5 | Δε [1 kHz, 20°C]: | 3.7 |
| CLP-V-1 | 8.0 | γ₁ [mPa s, 20°C]: | |
| CLY-3-O2 | 1.9 | γ₁ [mPa s, 20°C]: | 78 |
| APUQU-(c5)-F | 5.0 | K₁₁ [pN, 20°C]: | 20.2 |
| Compound **1** | 3.0 | K₃₃ [pN, 20°C]: | 19.5 |
| CP-3-O2 | 1.0 | V₀ [V, 20°C]: | |
| PGS-2-1 | 7.0 | LTS bulk [h, -20°C]: | |
| PP-1-2V1 | 3.0 | LTS bulk [h, -30°C]: | |
| Σ | 100.0 | LTS bulk [h, -10°C]: | |

### Mixture Example 11

The following mixture is prepared and investigated.

| Compound | % | Properties | |
|---|---|---|---|
| CC-3-2V1 | 14.0 | T(N,I) [°C]: | 98 |
| CC-3-V | 30.6 | Δn [589 nm, 20°C]: | 0.1097 |
| CC-3-V1 | 10.0 | ε_{∥} [1 kHz, 20°C]: | 6.4 |
| CCP-3-1 | 9.0 | ε_{⊥} [1 kHz, 20°C]: | 2.8 |
| CDUQU-3-F | 9.5 | Δε [1 kHz, 20°C]: | 3.6 |
| BCH-32 | 8.0 | γ₁ [mPa s, 20°C]: | |
| CLY-3-O2 | 1.9 | γ₁ [mPa s, 20°C]: | 75 |
| DPGU-4-F | 3.0 | K₁₁ [pN, 20°C]: | 20.5 |
| Compound **1** | 3.0 | K₃₃ [pN, 20°C]: | 18.9 |
| CP-3-O2 | 1.0 | V₀ [V, 20°C]: | |
| PGS-2-1 | 7.0 | LTS bulk [h, -20°C]: | |
| PP-1-2V1 | 3.0 | LTS bulk [h, -30°C]: | |
| Σ | 100.0 | LTS bulk [h, -10°C]: | |

### Mixture Example 12

The following mixture is prepared and investigated.

| Compound | % | Properties | |
|---|---|---|---|
| CC-3-2V1 | 14.0 | T(N,I) [°C]: | 99.5 |
| CC-3-V | 30.6 | Δn [589 nm, 20°C]: | 0.1103 |
| CC-3-V1 | 10.0 | ε_{∥} [1 kHz, 20°C]: | 6.4 |
| CCP-3-1 | 9.0 | ε_{⊥} [1 kHz, 20°C]: | 2.8 |
| CDUQU-3-F | 9.5 | Δε [1 kHz, 20°C]: | 3.6 |
| BCH-31 | 8.0 | γ₁ [mPa s, 20°C]: | |
| CLY-3-O2 | 1.9 | γ₁ [mPa s, 20°C]: | 75 |
| DPGU-4-F | 3.0 | K₁₁ [pN, 20°C]: | 20.5 |
| Compound **1** | 3.0 | K₃₃ [pN, 20°C]: | 19.0 |
| CP-3-O2 | 1.0 | V₀ [V, 20°C]: | |
| PGS-2-1 | 7.0 | LTS bulk [h, -20°C]: | |
| PP-1-2V1 | 3.0 | LTS bulk [h, -30°C]: | |
| Σ | 100.0 | LTS bulk [h, -10°C]: | |

### Mixture Example 13

The following mixture is prepared and investigated.

| Compound | % | Properties | |
|---|---|---|---|
| CC-3-2V1 | 14.0 | T(N,I) [°C]: | 99.5 |
| CC-3-V | 30.6 | Δn [589 nm, 20°C]: | 0.1111 |
| CC-3-V1 | 10.0 | ε_{∥} [1 kHz, 20°C]: | 6.4 |
| CCP-3-1 | 9.0 | ε_{⊥} [1 kHz, 20°C]: | 2.8 |
| CDUQU-3-F | 9.5 | Δε [1 kHz, 20°C]: | 3.6 |
| CPP-1V-2 | 8.0 | γ₁ [mPa s, 20°C]: | |
| CLY-3-O2 | 1.9 | γ₁ [mPa s, 20°C]: | 76 |
| DPGU-4-F | 3.0 | K₁₁ [pN, 20°C]: | 20.7 |
| Compound **1** | 3.0 | K₃₃ [pN, 20°C]: | 19.2 |
| CP-3-O2 | 1.0 | V₀ [V, 20°C]: | |
| PGS-2-1 | 7.0 | LTS bulk [h, -20°C]: | |
| PP-1-2V1 | 3.0 | LTS bulk [h, -30°C]: | |
| Σ | 100.0 | LTS bulk [h, -10°C]: | |

### Mixture Example 14

The following mixture is prepared and investigated.

| Compound | % | Properties | |
|---|---|---|---|
| CC-3-2V1 | 14.0 | T(N,I) [°C]: | 102 |
| CC-3-V | 30.6 | Δn [589 nm, 20°C]: | 0.1125 |
| CC-3-V1 | 10.0 | ε_{∥} [1 kHz, 20°C]: | 6.5 |
| CCP-3-1 | 9.0 | ε_{⊥} [1 kHz, 20°C]: | 2.8 |
| CDUQU-3-F | 9.5 | Δε [1 kHz, 20°C]: | 3.7 |
| CPP-3-2V1 | 8.0 | γ₁ [mPa s, 20°C]: | |
| CLY-3-O2 | 1.9 | γ₁ [mPa s, 20°C]: | 80 |
| DPGU-4-F | 3.0 | K₁₁ [pN, 20°C]: | 21.2 |
| Compound **1** | 3.0 | K₃₃ [pN, 20°C]: | 20.9 |
| CP-3-O2 | 1.0 | V₀ [V, 20°C]: | |
| PGS-2-1 | 7.0 | LTS bulk [h, -20°C]: | |
| PP-1-2V1 | 3.0 | LTS bulk [h, -30°C]: | |
| Σ | 100.0 | LTS bulk [h, -10°C]: | |

### Mixture Example 15

The following mixture is prepared and investigated.

| Compound | % | Properties | |
|---|---|---|---|
| CC-3-2V1 | 14.0 | T(N,I) [°C]: | 94.5 |
| CC-3-V | 30.6 | Δn [589 nm, 20°C]: | 0.1076 |
| CC-V-V1 | 10.0 | ε_{∥} [1 kHz, 20°C]: | 6.5 |
| CCP-3-1 | 9.0 | ε_{⊥} [1 kHz, 20°C]: | 2.8 |
| CDUQU-3-F | 9.5 | Δε [1 kHz, 20°C]: | 3.7 |
| CLP-V-1 | 8.0 | γ₁ [mPa s, 20°C]: | |
| CLY-3-O2 | 1.9 | γ₁ [mPa s, 20°C]: | 77 |
| DPGU-4-F | 3.0 | K₁₁ [pN, 20°C]: | 19.5 |
| Compound **1** | 3.0 | K₃₃ [pN, 20°C]: | 19.1 |
| CP-3-O2 | 1.0 | V₀ [V, 20°C]: | |
| PGS-2-1 | 7.0 | LTS bulk [h, -20°C]: | |
| PP-1-2V1 | 3.0 | LTS bulk [h, -30°C]: | |
| Σ | 100.0 | LTS bulk [h, -10°C]: | |

### Mixture Example 16

The following mixture is prepared and investigated.

| Compound | % | Properties | |
|---|---|---|---|
| CC-3-2V1 | 14.0 | T(N,I) [°C]: | 101.5 |
| CC-3-V | 30.6 | Δn [589 nm, 20°C]: | 0.1088 |
| CC-3-V1 | 10.0 | ε_{∥} [1 kHz, 20°C]: | 6.5 |
| CCP-3-O1 | 9.0 | ε_{⊥} [1 kHz, 20°C]: | 2.8 |
| CDUQU-3-F | 9.5 | Δε [1 kHz, 20°C]: | 3.7 |
| CLP-V-1 | 8.0 | γ₁ [mPa s, 20°C]: | |
| CLY-3-O2 | 1.9 | γ₁ [mPa s, 20°C]: | 81 |
| DPGU-4-F | 3.0 | K₁₁ [pN, 20°C]: | 20.8 |
| Compound **1** | 3.0 | K₃₃ [pN, 20°C]: | 19.6 |
| CP-3-O2 | 1.0 | V₀ [V, 20°C]: | |
| PGS-2-1 | 7.0 | LTS bulk [h, -20°C]: | |
| PP-1-2V1 | 3.0 | LTS bulk [h, -30°C]: | |
| Σ | 100.0 | LTS bulk [h, -10°C]: | |

### Mixture Example 17

The following mixture is prepared and investigated.

| Compound | % | Properties | |
|---|---|---|---|
| CC-3-2V1 | 14.0 | T(N,I) [°C]: | 101 |
| CC-3-V | 30.6 | Δn [589 nm, 20°C]: | 0.1090 |
| CC-3-V1 | 10.0 | ε_{∥} [1 kHz, 20°C]: | 6.6 |
| CCP-3-1 | 9.0 | ε_{⊥} [1 kHz, 20°C]: | 2.8 |
| CDUQU-3-F | 9.5 | Δε [1 kHz, 20°C]: | 3.8 |
| CLP-V-1 | 8.0 | γ₁ [mPa s, 20°C]: | |
| CCPP-V-2 | 1.9 | γ₁ [mPa s, 20°C]: | 79 |
| DPGU-4-F | 3.0 | K₁₁ [pN, 20°C]: | 21.0 |
| Compound **1** | 3.0 | K₃₃ [pN, 20°C]: | 19.8 |
| CP-3-O2 | 1.0 | V₀ [V, 20°C]: | |
| PGS-2-1 | 7.0 | LTS bulk [h, -20°C]: | |
| PP-1-2V1 | 3.0 | LTS bulk [h, -30°C]: | |
| Σ | 100.0 | LTS bulk [h, -10°C]: | |

### Mixture Example 18

The following mixture is prepared and investigated.

| Compound | % | Properties | |
|---|---|---|---|
| CC-3-2V1 | 14.0 | T(N,I) [°C]: | 100 |
| CC-3-V | 30.6 | Δn [589 nm, 20°C]: | 0.1080 |
| CC-3-V1 | 10.0 | ε_{∥} [1 kHz, 20°C]: | 6.1 |
| CCP-3-1 | 9.0 | ε_{⊥} [1 kHz, 20°C]: | 2.8 |
| CDUQU-3-F | 9.5 | Δε [1 kHz, 20°C]: | 3.3 |
| CLP-V-1 | 8.0 | γ₁ [mPa s, 20°C]: | |
| CLY-3-O2 | 1.9 | γ₁ [mPa s, 20°C]: | 78 |
| CCPU-3-F | 3.0 | K₁₁ [pN, 20°C]: | 20.9 |
| Compound **1** | 3.0 | K₃₃ [pN, 20°C]: | 20.1 |
| CP-3-O2 | 1.0 | V₀ [V, 20°C]: | |
| PGS-2-1 | 7.0 | LTS bulk [h, -20°C]: | |
| PP-1-2V1 | 3.0 | LTS bulk [h, -30°C]: | |
| Σ | 100.0 | LTS bulk [h, -10°C]: | |

### Mixture Example 19

The following mixture is prepared and investigated.

| Compound | % | Properties | |
|---|---|---|---|
| CC-3-2V1 | 14.0 | T(N,I) [°C]: | 99.5 |
| CC-3-V | 30.6 | Δn [589 nm, 20°C]: | 0.1066 |
| CC-3-V1 | 10.0 | ε_{∥} [1 kHz, 20°C]: | 6.5 |
| CCP-3-1 | 9.0 | ε_{⊥} [1 kHz, 20°C]: | 2.8 |
| CDUQU-3-F | 9.5 | Δε [1 kHz, 20°C]: | 3.7 |
| CLP-3-3 | 8.0 | γ₁ [mPa s, 20°C]: | |
| CLY-3-O2 | 1.9 | γ₁ [mPa s, 20°C]: | 78 |
| DPGU-4-F | 3.0 | K₁₁ [pN, 20°C]: | 21.0 |
| Compound **1** | 3.0 | K₃₃ [pN, 20°C]: | 19.3 |
| CP-3-O2 | 1.0 | V₀ [V, 20°C]: | |
| PGS-2-1 | 7.0 | LTS bulk [h, -20°C]: | |
| PP-1-2V1 | 3.0 | LTS bulk [h, -30°C]: | |
| Σ | 100.0 | LTS bulk [h, -10°C]: | |

### Mixture Example 20

The following mixture is prepared and investigated.

| Compound | % | Properties | |
|---|---|---|---|
| CC-3-2V1 | 14.0 | T(N,I) [°C]: | 102.5 |
| CC-3-V | 30.6 | Δn [589 nm, 20°C]: | 0.1080 |
| CC-3-V1 | 10.0 | ε_{∥} [1 kHz, 20°C]: | 6.5 |
| CCP-3-1 | 9.0 | ε_{⊥} [1 kHz, 20°C]: | 2.8 |
| CDUQU-3-F | 9.5 | Δε [1 kHz, 20°C]: | 3.7 |
| CLP-3-O1 | 8.0 | γ₁ [mPa s, 20°C]: | |
| CLY-3-O2 | 1.9 | γ₁ [mPa s, 20°C]: | 82 |
| DPGU-4-F | 3.0 | K₁₁ [pN, 20°C]: | 21.2 |
| Compound **1** | 3.0 | K₃₃ [pN, 20°C]: | 20.4 |
| CP-3-O2 | 1.0 | V₀ [V, 20°C]: | |
| PGS-2-1 | 7.0 | LTS bulk [h, -20°C]: | |
| PP-1-2V1 | 3.0 | LTS bulk [h, -30°C]: | |
| Σ | 100.0 | LTS bulk [h, -10°C]: | |

### Mixture Example 21

The following mixture is prepared and investigated.

| Compound | % | Properties | |
|---|---|---|---|
| CC-3-2V1 | 14.0 | T(N,I) [°C]: | 98 |
| CC-3-V | 30.6 | Δn [589 nm, 20°C]: | 0.1119 |
| CC-3-V1 | 10.0 | ε_{∥} [1 kHz, 20°C]: | 6.4 |
| CCP-3-1 | 9.0 | ε_{⊥} [1 kHz, 20°C]: | 2.8 |
| CGUQU-3-F | 9.5 | Δε [1 kHz, 20°C]: | 3.6 |
| CLP-V-1 | 8.0 | γ₁ [mPa s, 20°C]: | |
| CLY-3-O2 | 1.9 | γ₁ [mPa s, 20°C]: | 79 |
| DPGU-4-F | 3.0 | K₁₁ [pN, 20°C]: | 19.9 |
| Compound **1** | 3.0 | K₃₃ [pN, 20°C]: | 19.4 |
| CP-3-O2 | 1.0 | V₀ [V, 20°C]: | |
| PGS-2-1 | 7.0 | LTS bulk [h, -20°C]: | |
| PP-1-2V1 | 3.0 | LTS bulk [h, -30°C]: | |
| Σ | 100.0 | LTS bulk [h, -10°C]: | |

### Mixture Example 22

The following mixture is prepared and investigated.

| Compound | % | Properties | |
|---|---|---|---|
| CC-3-2V1 | 14.0 | T(N,I) [°C]: | 95.0 |
| CC-3-V | 30.6 | Δn [589 nm, 20°C]: | 0.1024 |
| CC-3-V1 | 10.0 | ε_{∥} [1 kHz, 20°C]: | 7.0 |
| CCP-3-1 | 9.0 | ε_{⊥} [1 kHz, 20°C]: | 2.8 |
| CDUQU-3-F | 9.5 | Δε [1 kHz, 20°C]: | 4.2 |
| CLP-V-1 | 8.0 | γ₁ [mPa s, 20°C]: | |
| CLY-3-O2 | 1.9 | γ₁ [mPa s, 20°C]: | 79 |
| DPGU-4-F | 3.0 | K₁₁ [pN, 20°C]: | 18.9 |
| Compound **1** | 3.0 | K₃₃ [pN, 20°C]: | 19.4 |
| CP-3-O2 | 1.0 | V₀ [V, 20°C]: | |
| PGP-3-F | 7.0 | LTS bulk [h, -20°C]: | |
| PP-1-2V1 | 3.0 | LTS bulk [h, -30°C]: | |
| Σ | 100.0 | LTS bulk [h, -10°C]: | |

### Mixture Example 23

The following mixture is prepared and investigated.

| Compound | % | Properties | |
|---|---|---|---|
| CC-3-2V1 | 14.0 | T(N,I) [°C]: | 96 |
| CC-3-V | 30.6 | Δn [589 nm, 20°C]: | 0.1097 |
| CC-3-V1 | 10.0 | ε_{∥} [1 kHz, 20°C]: | 6.6 |
| CCP-3-1 | 9.0 | ε_{⊥} [1 kHz, 20°C]: | 2.8 |
| CDUQU-3-F | 7.5 | Δε [1 kHz, 20°C]: | 3.8 |
| CLP-V-1 | 8.0 | γ₁ [mPa s, 20°C]: | |
| CLY-3-O2 | 1.9 | γ₁ [mPa s, 20°C]: | 79 |
| PGUQU(1)-3-F | 5.0 | K₁₁ [pN, 20°C]: | 20.1 |
| Compound **1** | 3.0 | K₃₃ [pN, 20°C]: | 19.7 |
| CP-3-O2 | 1.0 | V₀ [V, 20°C]: | |
| PGS-2-1 | 7.0 | LTS bulk [h, -20°C]: | |
| PP-1-2V1 | 3.0 | LTS bulk [h, -30°C]: | |
| Σ | 100.0 | LTS bulk [h, -10°C]: | |

### Mixture Example 24

The following mixture is prepared and investigated.

| Compound | % | Properties | |
|---|---|---|---|
| CC-3-2V1 | 14.0 | T(N,I) [°C]: | 96 |
| CC-3-V | 30.6 | Δn [589 nm, 20°C]: | 0.1102 |
| CC-3-V1 | 10.0 | ε_{∥} [1 kHz, 20°C]: | 6.5 |
| CCP-3-1 | 9.0 | ε_{⊥} [1 kHz, 20°C]: | 2.8 |
| CDUQU-3-F | 7.5 | Δε [1 kHz, 20°C]: | 3.7 |
| CLP-V-1 | 8.0 | γ₁ [mPa s, 20°C]: | |
| CLY-3-O2 | 1.9 | γ₁ [mPa s, 20°C]: | 78 |
| PGUQU-(c5)-F | 5.0 | K₁₁ [pN, 20°C]: | 19.8 |
| Compound **1** | 3.0 | K₃₃ [pN, 20°C]: | 19.3 |
| CP-3-O2 | 1.0 | V₀ [V, 20°C]: | |
| PGS-2-1 | 7.0 | LTS bulk [h, -20°C]: | |
| PP-1-2V1 | 3.0 | LTS bulk [h, -30°C]: | |
| Σ | 100.0 | LTS bulk [h, -10°C]: | |

### Mixture Example 25

The following mixture is prepared and investigated.

| Compound | % | Properties | |
|---|---|---|---|
| CC-3-2V1 | 14.0 | T(N,I) [°C]: | 90.5 |
| CC-3-V | 30.6 | Δn [589 nm, 20°C]: | 0.1057 |
| CC-3-V1 | 10.0 | ε_{∥} [1 kHz, 20°C]: | 6.3 |
| CCP-3-1 | 9.0 | ε_{⊥} [1 kHz, 20°C]: | 2.8 |
| CDUQU-3-F | 7.5 | Δε [1 kHz, 20°C]: | 3.5 |
| CLP-V-1 | 8.0 | γ₁ [mPa s, 20°C]: | |
| CLY-3-O2 | 1.9 | γ₁ [mPa s, 20°C]: | 71 |
| PUQU(1)-3-F | 5.0 | K₁₁ [pN, 20°C]: | 18.7 |
| Compound **1** | 3.0 | K₃₃ [pN, 20°C]: | 18.4 |
| CP-3-O2 | 1.0 | V₀ [V, 20°C]: | |
| PGS-2-1 | 7.0 | LTS bulk [h, -20°C]: | |
| PP-1-2V1 | 3.0 | LTS bulk [h, -30°C]: | |
| Σ | 100.0 | LTS bulk [h, -10°C]: | |

### Mixture Example 26

The following mixture is prepared and investigated.

| Compound | % | Properties | |
|---|---|---|---|
| CC-3-2V1 | 14.0 | T(N,I) [°C]: | 9.5 |
| CC-3-V | 30.6 | Δn [589 nm, 20°C]: | 0.1047 |
| CC-3-V1 | 10.0 | ε_{∥} [1 kHz, 20°C]: | 6.2 |
| CCP-3-1 | 9.0 | ε_{⊥} [1 kHz, 20°C]: | 2.8 |
| CDUQU-3-F | 7.5 | Δε [1 kHz, 20°C]: | 3.4 |
| CLP-V-1 | 8.0 | γ₁ [mPa s, 20°C]: | |
| CLY-3-O2 | 1.9 | γ₁ [mPa s, 20°C]: | 72 |
| PUQU-(c5)-F | 5.0 | K₁₁ [pN, 20°C]: | 19.0 |
| Compound **1** | 3.0 | K₃₃ [pN, 20°C]: | 18.4 |
| CP-3-O2 | 1.0 | V₀ [V, 20°C]: | |
| PGS-2-1 | 7.0 | LTS bulk [h, -20°C]: | |
| PP-1-2V1 | 3.0 | LTS bulk [h, -30°C]: | |
| Σ | 100.0 | LTS bulk [h, -10°C]: | |

### Mixture Example 27

The following mixture is prepared and investigated.

| Compound | % | Properties | |
|---|---|---|---|
| CC-3-2V1 | 14.0 | T(N,I) [°C]: | 98.5 |
| CC-3-V | 30.6 | Δn [589 nm, 20°C]: | 0.1061 |
| CC-3-V1 | 10.0 | ε_{∥} [1 kHz, 20°C]: | 6.3 |
| CCP-3-1 | 9.0 | ε_{⊥} [1 kHz, 20°C]: | 2.8 |
| CDUQU-3-F | 9.5 | Δε [1 kHz, 20°C]: | 3.5 |
| CLP-V-1 | 8.0 | γ₁ [mPa s, 20°C]: | |
| CLY-3-O2 | 1.9 | γ₁ [mPa s, 20°C]: | 79 |
| CDEPU-3-F | 3.0 | K₁₁ [pN, 20°C]: | 20.9 |
| Compound **1** | 3.0 | K₃₃ [pN, 20°C]: | 20.1 |
| CP-3-O2 | 1.0 | V₀ [V, 20°C]: | |
| PGS-2-1 | 7.0 | LTS bulk [h, -20°C]: | |
| PP-1-2V1 | 3.0 | LTS bulk [h, -30°C]: | |
| Σ | 100.0 | LTS bulk [h, -10°C]: | |

### Mixture Example 28

The following mixture is prepared and investigated.

| Compound | % | Properties | |
|---|---|---|---|
| CC-3-2V1 | 14.0 | T(N,I) [°C]: | 97.5 |
| CC-3-V | 30.6 | Δn [589 nm, 20°C]: | 0.1051 |
| CC-3-2V | 10.0 | ε_{∥} [1 kHz, 20°C]: | 6.5 |
| CCP-3-1 | 9.0 | ε_{⊥} [1 kHz, 20°C]: | 2.8 |
| CDUQU-3-F | 9.5 | Δε [1 kHz, 20°C]: | 3.7 |
| CLP-V-1 | 8.0 | γ₁ [mPa s, 20°C]: | |
| CLY-3-O2 | 1.9 | γ₁ [mPa s, 20°C]: | 75 |
| DPGU-4-F | 3.0 | K₁₁ [pN, 20°C]: | 21.1 |
| Compound **1** | 3.0 | K₃₃ [pN, 20°C]: | 19.3 |
| CP-3-O2 | 1.0 | V₀ [V, 20°C]: | |
| PGS-2-1 | 7.0 | LTS bulk [h, -20°C]: | |
| PP-1-2V1 | 3.0 | LTS bulk [h, -30°C]: | |
| Σ | 100.0 | LTS bulk [h, -10°C]: | |

### Mixture Example 29

The following mixture is prepared and investigated.

| Compound | % | Properties | |
|---|---|---|---|
| CC-3-2V1 | 14.0 | T(N,I) [°C]: | 99.5 |
| CC-3-V | 30.6 | Δn [589 nm, 20°C]: | 0.1067 |
| CC-3-V1 | 10.0 | ε_{∥} [1 kHz, 20°C]: | 6.6 |
| CCP-3-1 | 9.0 | ε_{⊥} [1 kHz, 20°C]: | 2.8 |
| CDUQU-3-F | 9.5 | Δε [1 kHz, 20°C]: | 3.8 |
| CLP-V-1 | 8.0 | γ₁ [mPa s, 20°C]: | |
| CCC-3-V | 1.9 | γ₁ [mPa s, 20°C]: | 76 |
| DPGU-4-F | 3.0 | K₁₁ [pN, 20°C]: | 20.9 |
| Compound **1** | 3.0 | K₃₃ [pN, 20°C]: | 19.7 |
| CP-3-O2 | 1.0 | V₀ [V, 20°C]: | |
| PGS-2-1 | 7.0 | LTS bulk [h, -20°C]: | |
| PP-1-2V1 | 3.0 | LTS bulk [h, -30°C]: | |
| Σ | 100.0 | LTS bulk [h, -10°C]: | |

### Mixture Example 30

The following mixture is prepared and investigated.

| Compound | % | Properties | |
|---|---|---|---|
| CC-3-2V1 | 14.0 | T(N,I) [°C]: | 98 |
| CC-3-V | 30.6 | Δn [589 nm, 20°C]: | 0.1074 |
| CC-3-V1 | 10.0 | ε_{∥} [1 kHz, 20°C]: | 6.5 |
| CCP-3-1 | 9.0 | ε_{⊥} [1 kHz, 20°C]: | 2.8 |
| CDUQU(1)-3-F | 9.5 | Δε [1 kHz, 20°C]: | 3.7 |
| CLP-V-1 | 8.0 | γ₁ [mPa s, 20°C]: | |
| CLY-3-O2 | 1.9 | γ₁ [mPa s, 20°C]: | 82 |
| DPGU-4-F | 3.0 | K₁₁ [pN, 20°C]: | 20.8 |
| Compound **1** | 3.0 | K₃₃ [pN, 20°C]: | 19.7 |
| CP-3-O2 | 1.0 | V₀ [V, 20°C]: | |
| PGS-2-1 | 7.0 | LTS bulk [h, -20°C]: | |
| PP-1-2V1 | 3.0 | LTS bulk [h, -30°C]: | |
| ∑ | 100.0 | LTS bulk [h, -10°C]: | |

### Mixture Example 31

The following mixture is prepared and investigated.

| Compound | % | Properties | |
|---|---|---|---|
| CC-3-2V1 | 14.0 | T(N,I) [°C]: | 98.5 |
| CC-3-V | 30.6 | Δn [589 nm, 20°C]: | 0.1066 |
| CC-3-V1 | 10.0 | ε_{∥} [1 kHz, 20°C]: | 6.5 |
| CCP-3-1 | 9.0 | ε_{⊥} [1 kHz, 20°C]: | 2.8 |
| CDUQU-3-F | 9.5 | Δε [1 kHz, 20°C]: | 3.7 |
| CLG-3-1 | 8.0 | γ₁ [mPa s, 20°C]: | |
| CLY-3-O2 | 1.9 | γ₁ [mPa s, 20°C]: | 78 |
| DPGU-4-F | 3.0 | K₁₁ [pN, 20°C]: | 20.8 |
| Compound **1** | 3.0 | K₃₃ [pN, 20°C]: | 19.4 |
| CP-3-O2 | 1.0 | V₀ [V, 20°C]: | |
| PGS-2-1 | 7.0 | LTS bulk [h, -20°C]: | |
| PP-1-2V1 | 3.0 | LTS bulk [h, -30°C]: | |
| ∑ | 100.0 | LTS bulk [h, -10°C]: | |

### Mixture Example 32

The following mixture is prepared and investigated.

| Compound | % | Properties | |
|---|---|---|---|
| CC-3-2V1 | 14.0 | T(N,I) [°C]: | 99.5 |
| CC-3-V | 30.6 | Δn [589 nm, 20°C]: | 0.1070 |
| CC-3-V1 | 10.0 | ε_{∥} [1 kHz, 20°C]: | 6.2 |
| CCP-3-1 | 9.0 | ε_{⊥} [1 kHz, 20°C]: | 2.8 |
| CDUQU-3-F | 9.5 | Δε [1 kHz, 20°C]: | 3.4 |
| CLP-V-1 | 8.0 | γ₁ [mPa s, 20°C]: | |
| CLY-3-O2 | 1.9 | γ₁ [mPa s, 20°C]: | 78 |
| CLGU-4-F | 3.0 | K₁₁ [pN, 20°C]: | 20.7 |
| Compound **1** | 3.0 | K₃₃ [pN, 20°C]: | 19.7 |
| CP-3-O2 | 1.0 | V₀ [V, 20°C]: | |
| PGS-2-1 | 7.0 | LTS bulk [h, -20°C]: | |
| PP-1-2V1 | 3.0 | LTS bulk [h, -30°C]: | |
| ∑ | 100.0 | LTS bulk [h, -10°C]: | |

### Mixture Example 33

The following mixture is prepared and investigated.

| Compound | % | Properties | |
|---|---|---|---|
| CC-3-2V1 | 14.0 | T(N,I) [°C]: | 96 |
| CC-3-V | 30.6 | Δn [589 nm, 20°C]: | 0.1059 |
| CC-3-V1 | 10.0 | ε_{∥} [1 kHz, 20°C]: | 7.1 |
| CCP-3-1 | 9.0 | ε_{⊥} [1 kHz, 20°C]: | 2.8 |
| CDUQU-3-F | 9.5 | Δε [1 kHz, 20°C]: | 4.3 |
| CLP-(c5)-T | 8.0 | γ₁ [mPa s, 20°C]: | |
| CLY-3-O2 | 1.9 | γ₁ [mPa s, 20°C]: | 82 |
| DPGU-4-F | 3.0 | K₁₁ [pN, 20°C]: | 21.4 |
| Compound **1** | 3.0 | K₃₃ [pN, 20°C]: | 19.8 |
| CP-3-O2 | 1.0 | V₀ [V, 20°C]: | |
| PGS-2-1 | 7.0 | LTS bulk [h, -20°C]: | |
| PP-1-2V1 | 3.0 | LTS bulk [h, -30°C]: | |
| ∑ | 100.0 | LTS bulk [h, -10°C]: | |

### Mixture Example 34

The following mixture is prepared and investigated.

| Compound | % | Properties | |
|---|---|---|---|
| CC-3-2V1 | 14.0 | T(N,I) [°C]: | 96.5 |
| CC-3-V | 30.6 | Δn [589 nm, 20°C]: | 0.1058 |
| CC-3-V1 | 10.0 | ε_{∥} [1 kHz, 20°C]: | 7.1 |
| CCP-3-1 | 9.0 | ε_{⊥} [1 kHz, 20°C]: | 2.8 |
| CDUQU-3-F | 9.5 | Δε [1 kHz, 20°C]: | 4.3 |
| CLP-3-T | 8.0 | γ₁ [mPa s, 20°C]: | |
| CLY-3-O2 | 1.9 | γ₁ [mPa s, 20°C]: | 78 |
| DPGU-4-F | 3.0 | K₁₁ [pN, 20°C]: | 21.5 |
| Compound **1** | 3.0 | K₃₃ [pN, 20°C]: | 19.6 |
| CP-3-O2 | 1.0 | V₀ [V, 20°C]: | |
| PGS-2-1 | 7.0 | LTS bulk [h, -20°C]: | |
| PP-1-2V1 | 3.0 | LTS bulk [h, -30°C]: | |
| ∑ | 100.0 | LTS bulk [h, -10°C]: | |

### Mixture Example 35

The following mixture is prepared and investigated.

| Compound | % | Properties | |
|---|---|---|---|
| CC-3-2V1 | 14.0 | T(N,I) [°C]: | 93 |
| CC-3-V | 30.6 | Δn [589 nm, 20°C]: | 0.1046 |
| CC-3-V1 | 10.0 | ε_{∥} [1 kHz, 20°C]: | 7.9 |
| CCP-3-1 | 9.0 | ε_{⊥} [1 kHz, 20°C]: | 2.8 |
| CDUQU-3-F | 9.5 | Δε [1 kHz, 20°C]: | 5.1 |
| CLU-3-T | 8.0 | γ₁ [mPa s, 20°C]: | |
| CLY-3-O2 | 1.9 | γ₁ [mPa s, 20°C]: | 77 |
| DPGU-4-F | 3.0 | K₁₁ [pN, 20°C]: | 19.6 |
| Compound **1** | 3.0 | K₃₃ [pN, 20°C]: | 18.3 |
| CP-3-O2 | 1.0 | V₀ [V, 20°C]: | |
| PGS-2-1 | 7.0 | LTS bulk [h, -20°C]: | |
| PP-1-2V1 | 3.0 | LTS bulk [h, -30°C]: | |
| ∑ | 100.0 | LTS bulk [h, -10°C]: | |

### Mixture Example 36

The following mixture is prepared and investigated.

| Compound | % | Properties | |
|---|---|---|---|
| CC-3-2V1 | 14.0 | T(N,I) [°C]: | 96 |
| CC-3-V | 30.6 | Δn [589 nm, 20°C]: | 0.1057 |
| CC-3-V1 | 10.0 | ε_{∥} [1 kHz, 20°C]: | 6.7 |
| CCP-3-1 | 9.0 | ε_{⊥} [1 kHz, 20°C]: | 2.8 |
| CDUQU-3-F | 9.5 | Δε [1 kHz, 20°C]: | 3.9 |
| CLP-V-1 | 8.0 | γ₁ [mPa s, 20°C]: | |
| CLY-3-O2 | 1.9 | γ₁ [mPa s, 20°C]: | 76 |
| DGUQU-4-F | 3.0 | K₁₁ [pN, 20°C]: | 20.0 |
| Compound **1** | 3.0 | K₃₃ [pN, 20°C]: | 19.4 |
| CP-3-O2 | 1.0 | V₀ [V, 20°C]: | |
| PGS-2-1 | 7.0 | LTS bulk [h, -20°C]: | |
| PP-1-2V1 | 3.0 | LTS bulk [h, -30°C]: | |
| ∑ | 100.0 | LTS bulk [h, -10°C]: | |

### Mixture Example 37

The following mixture is prepared and investigated.

| Compound | % | Properties | |
|---|---|---|---|
| CC-3-2V1 | 14.0 | T(N,I) [°C]: | 96 |
| CC-3-V | 30.6 | Δn [589 nm, 20°C]: | 0.1059 |
| CC-3-V1 | 10.0 | ε_{∥} [1 kHz, 20°C]: | 6.6 |
| CCP-3-1 | 9.0 | ε_{⊥} [1 kHz, 20°C]: | 2.8 |
| CDUQU-3-F | 9.5 | Δε [1 kHz, 20°C]: | 3.8 |
| CLP-V-1 | 8.0 | γ₁ [mPa s, 20°C]: | |
| CLY-3-O2 | 1.9 | γ₁ [mPa s, 20°C]: | 78 |
| DGUQU-(c5)-F | 3.0 | K₁₁ [pN, 20°C]: | 19.8 |
| Compound **1** | 3.0 | K₃₃ [pN, 20°C]: | 19.3 |
| CP-3-O2 | 1.0 | V₀ [V, 20°C]: | |
| PGS-2-1 | 7.0 | LTS bulk [h, -20°C]: | |
| PP-1-2V1 | 3.0 | LTS bulk [h, -30°C]: | |
| Σ | 100.0 | LTS bulk [h, -10°C]: | |

### Mixture Example 38

The following mixture is prepared and investigated.

| Compound | % | Properties | |
|---|---|---|---|
| CC-3-2V1 | 14.0 | T(N,I) [°C]: | 96 |
| CC-3-V | 30.6 | Δn [589 nm, 20°C]: | 0.1057 |
| CC-3-V1 | 10.0 | ε_{∥} [1 kHz, 20°C]: | 6.6 |
| CCP-3-1 | 9.0 | ε_{⊥} [1 kHz, 20°C]: | 2.8 |
| CDUQU-3-F | 9.5 | Δε [1 kHz, 20°C]: | 3.8 |
| CLP-V-1 | 8.0 | γ₁ [mPa s, 20°C]: | |
| CLY-3-O2 | 1.9 | γ₁ [mPa s, 20°C]: | 77 |
| DGUQU(1)-4-F | 3.0 | K₁₁ [pN, 20°C]: | 20.0 |
| Compound **1** | 3.0 | K₃₃ [pN, 20°C]: | 19.4 |
| CP-3-O2 | 1.0 | V₀ [V, 20°C]: | |
| PGS-2-1 | 7.0 | LTS bulk [h, -20°C]: | |
| PP-1-2V1 | 3.0 | LTS bulk [h, -30°C]: | |
| Σ | 100.0 | LTS bulk [h, -10°C]: | |

### Mixture Example 39

The following mixture is prepared and investigated.

| Compound | % | Properties | |
|---|---|---|---|
| CC-3-2V1 | 14.0 | T(N,I) [°C]: | 98 |
| CC-3-V | 30.6 | Δn [589 nm, 20°C]: | 0.1071 |
| CC-3-V1 | 10.0 | ε_{∥} [1 kHz, 20°C]: | 6.5 |
| CCP-3-1 | 9.0 | ε_{⊥} [1 kHz, 20°C]: | 2.8 |
| CDUQU-3-F | 9.5 | Δε [1 kHz, 20°C]: | 3.7 |
| CLP-V-1 | 8.0 | γ₁ [mPa s, 20°C]: | |
| CLY-3-O2 | 1.9 | γ₁ [mPa s, 20°C]: | 77 |
| DLGU-3-F | 3.0 | K₁₁ [pN, 20°C]: | 20.9 |
| Compound **1** | 3.0 | K₃₃ [pN, 20°C]: | 19.7 |
| CP-3-O2 | 1.0 | V₀ [V, 20°C]: | |
| PGS-2-1 | 7.0 | LTS bulk [h, -20°C]: | |
| PP-1-2V1 | 3.0 | LTS bulk [h, -30°C]: | |
| Σ | 100.0 | LTS bulk [h, -10°C]: | |

### Mixture Example 40

The following mixture is prepared and investigated.

| Compound | % | Properties | |
|---|---|---|---|
| CC-3-2V1 | 14.0 | T(N,I) [°C]: | 97.5 |
| CC-3-V | 30.6 | Δn [589 nm, 20°C]: | 0.1071 |
| CC-3-V1 | 10.0 | ε_{∥} [1 kHz, 20°C]: | 6.4 |
| CCP-3-1 | 9.0 | ε_{⊥} [1 kHz, 20°C]: | 2.8 |
| CDUQU-3-F | 9.5 | Δε [1 kHz, 20°C]: | 3.6 |
| CLP-V-1 | 8.0 | γ₁ [mPa s, 20°C]: | |
| CLY-3-O2 | 1.9 | γ₁ [mPa s, 20°C]: | 79 |
| DLGU-(c5)-F | 3.0 | K₁₁ [pN, 20°C]: | 20.8 |
| Compound **1** | 3.0 | K₃₃ [pN, 20°C]: | 19.5 |
| CP-3-O2 | 1.0 | V₀ [V, 20°C]: | |
| PGS-2-1 | 7.0 | LTS bulk [h, -20°C]: | |
| PP-1-2V1 | 3.0 | LTS bulk [h, -30°C]: | |
| Σ | 100.0 | LTS bulk [h, -10°C]: | |

### Mixture Example 41

The following mixture is prepared and investigated.

| Compound | % | Properties | |
|---|---|---|---|
| CC-3-2V1 | 14.0 | T(N,I) [°C]: | 98 |
| CC-3-V | 30.6 | Δn [589 nm, 20°C]: | 0.1069 |
| CC-3-V1 | 10.0 | ε_{∥} [1 kHz, 20°C]: | 6.5 |
| CCP-3-1 | 9.0 | ε_{⊥} [1 kHz, 20°C]: | 2.8 |
| CDUQU-3-F | 9.5 | Δε [1 kHz, 20°C]: | 3.7 |
| CLP-V-1 | 8.0 | γ₁ [mPa s, 20°C]: | |
| CLY-3-O2 | 1.9 | γ₁ [mPa s, 20°C]: | 79 |
| DLGU(1)-3-F | 3.0 | K₁₁ [pN, 20°C]: | 20.9 |
| Compound 1 | 3.0 | K₃₃ [pN, 20°C]: | 19.7 |
| CP-3-O2 | 1.0 | V₀ [V, 20°C]: | |
| PGS-2-1 | 7.0 | LTS bulk [h, -20°C]: | |
| PP-1-2V1 | 3.0 | LTS bulk [h, -30°C]: | |
| Σ | 100.0 | LTS bulk [h, -10°C]: | |

### Mixture Example 42

The following mixture is prepared and investigated.

| Compound | % | Properties | |
|---|---|---|---|
| CC-3-2V1 | 14.0 | T(N,I) [°C]: | 98.5 |
| CC-3-V | 30.6 | Δn [589 nm, 20°C]: | 0.1080 |
| CC-3-V1 | 10.0 | ε_{∥} [1 kHz, 20°C]: | 6.4 |
| CCP-3-1 | 9.0 | ε_{⊥} [1 kHz, 20°C]: | 2.8 |
| CDUQU-3-F | 9.5 | Δε [1 kHz, 20°C]: | 3.6 |
| CLP-V-1 | 8.0 | γ₁ [mPa s, 20°C]: | |
| CLY-3-O2 | 1.9 | γ₁ [mPa s, 20°C]: | 79 |
| DPGU-(c5)-F | 3.0 | K₁₁ [pN, 20°C]: | 20.7 |
| Compound 1 | 3.0 | K₃₃ [pN, 20°C]: | 19.5 |
| CP-3-O2 | 1.0 | V₀ [V, 20°C]: | |
| PGS-2-1 | 7.0 | LTS bulk [h, -20°C]: | |
| PP-1-2V1 | 3.0 | LTS bulk [h, -30°C]: | |
| Σ | 100.0 | LTS bulk [h, -10°C]: | |

### Mixture Example 43

The following mixture is prepared and investigated.

| Compound | % | Properties | |
|---|---|---|---|
| CC-3-2V1 | 14.0 | T(N,I) [°C]: | 98.5 |
| CC-3-V | 30.6 | Δn [589 nm, 20°C]: | 0.1080 |
| CC-3-V1 | 10.0 | ε_{∥} [1 kHz, 20°C]: | 6.5 |
| CCP-3-1 | 9.0 | ε_{⊥} [1 kHz, 20°C]: | 2.8 |
| CDUQU-3-F | 9.5 | Δε [1 kHz, 20°C]: | 3.7 |
| CLP-V-1 | 8.0 | γ₁ [mPa s, 20°C]: | |
| CLY-3-O2 | 1.9 | γ₁ [mPa s, 20°C]: | 79 |
| DPGU(1)-4-F | 3.0 | K₁₁ [pN, 20°C]: | 20.8 |
| Compound 1 | 3.0 | K₃₃ [pN, 20°C]: | 19.7 |
| CP-3-O2 | 1.0 | V₀ [V, 20°C]: | |
| PGS-2-1 | 7.0 | LTS bulk [h, -20°C]: | |
| PP-1-2V1 | 3.0 | LTS bulk [h, -30°C]: | |
| Σ | 100.0 | LTS bulk [h, -10°C]: | |

### Mixture Example 44

The following mixture is prepared and investigated.

| Compound | % | Properties | |
|---|---|---|---|
| CC-3-2V1 | 14.0 | T(N,I) [°C]: | 93.5 |
| CC-3-V | 30.6 | Δn [589 nm, 20°C]: | 0.1039 |
| CC-3-V1 | 10.0 | ε_{∥} [1 kHz, 20°C]: | 6.4 |
| CCP-3-1 | 9.0 | ε_{⊥} [1 kHz, 20°C]: | 2.8 |
| CDUQU-3-F | 9.5 | Δε [1 kHz, 20°C]: | 3.6 |
| CLP-V-1 | 8.0 | γ₁ [mPa s, 20°C]: | |
| CLY-3-O2 | 1.9 | γ₁ [mPa s, 20°C]: | 74 |
| DUQU-(c5)-F | 3.0 | K₁₁ [pN, 20°C]: | 19.7 |
| Compound 1 | 3.0 | K₃₃ [pN, 20°C]: | 18.9 |
| CP-3-O2 | 1.0 | V₀ [V, 20°C]: | |
| PGS-2-1 | 7.0 | LTS bulk [h, -20°C]: | |
| PP-1-2V1 | 3.0 | LTS bulk [h, -30°C]: | |
| Σ | 100.0 | LTS bulk [h, -10°C]: | |

### Mixture Example 45

The following mixture is prepared and investigated.

| Compound | % | Properties | |
|---|---|---|---|
| CC-3-2V1 | 14.0 | T(N,I) [°C]: | 100.5 |
| CC-3-V | 30.6 | Δn [589 nm, 20°C]: | 0.1102 |
| CC-3-V1 | 10.0 | ε_{∥} [1 kHz, 20°C]: | 6.7 |
| CCP-3-1 | 9.0 | ε_{⊥} [1 kHz, 20°C]: | 2.8 |
| CDUQU-3-F | 9.5 | Δε [1 kHz, 20°C]: | 3.9 |
| CLP-V-1 | 8.0 | γ₁ [mPa s, 20°C]: | |
| CLY-3-O2 | 1.9 | γ₁ [mPa s, 20°C]: | 80 |
| DPGU-4-F | 3.0 | K₁₁ [pN, 20°C]: | 21.0 |
| Compound 1 | 3.0 | K₃₃ [pN, 20°C]: | 19.9 |
| PPGU-3-F | 1.0 | V₀ [V, 20°C]: | |
| PGS-2-1 | 7.0 | LTS bulk [h, -20°C]: | |
| PP-1-2V1 | 3.0 | LTS bulk [h, -30°C]: | |
| Σ | 100.0 | LTS bulk [h, -10°C]: | |

### Mixture Example 46

The following mixture is prepared and investigated.

| Compound | % | Properties | |
|---|---|---|---|
| CC-3-2V1 | 14.0 | T(N,I) [°C]: | 100.5 |
| CC-3-V | 30.6 | Δn [589 nm, 20°C]: | 0.1100 |
| CC-3-V1 | 10.0 | ε_{∥} [1 kHz, 20°C]: | 6.7 |
| CCP-3-1 | 9.0 | ε_{⊥} [1 kHz, 20°C]: | 2.8 |
| CDUQU-3-F | 9.5 | Δε [1 kHz, 20°C]: | 3.9 |
| CLP-V-1 | 8.0 | γ₁ [mPa s, 20°C]: | |
| CLY-3-O2 | 1.9 | γ₁ [mPa s, 20°C]: | 81 |
| DPGU-4-F | 3.0 | K₁₁ [pN, 20°C]: | 20.8 |
| Compound 1 | 3.0 | K₃₃ [pN, 20°C]: | 19.8 |
| PPGU-(c5)-F | 1.0 | V₀ [V, 20°C]: | |
| PGS-2-1 | 7.0 | LTS bulk [h, -20°C]: | |
| PP-1-2V1 | 3.0 | LTS bulk [h, -30°C]: | |
| Σ | 100.0 | LTS bulk [h, -10°C]: | |

### Mixture Example 47

The following mixture is prepared and investigated.

| Compound | % | Properties | |
|---|---|---|---|
| CC-3-2V1 | 14.0 | T(N,I) [°C]: | 101.5 |
| CC-3-V | 30.6 | Δn [589 nm, 20°C]: | 0.1112 |
| CC-3-V1 | 10.0 | ε_{∥} [1 kHz, 20°C]: | 6.5 |
| CCP-3-1 | 9.0 | ε_{⊥} [1 kHz, 20°C]: | 2.8 |
| CDUQU-3-F | 9.5 | Δε [1 kHz, 20°C]: | 3.7 |
| CLP-V-1 | 8.0 | γ₁ [mPa s, 20°C]: | |
| CLY-3-O2 | 1.9 | γ₁ [mPa s, 20°C]: | 82 |
| DPGU-4-F | 3.0 | K₁₁ [pN, 20°C]: | 21.0 |
| Compound 1 | 3.0 | K₃₃ [pN, 20°C]: | 20.0 |
| PGP-5-Nap | 1.0 | V₀ [V, 20°C]: | |
| PGS-2-1 | 7.0 | LTS bulk [h, -20°C]: | |
| PP-1-2V1 | 3.0 | LTS bulk [h, -30°C]: | |
| Σ | 100.0 | LTS bulk [h, -10°C]: | |

## Claims

**1.** Liquid-crystalline medium having a nematic phase **characterized in that** it comprises one or more compounds of formula I 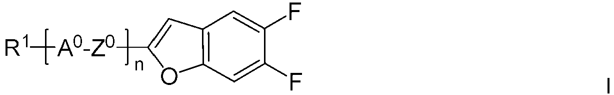 in which
R¹ an alkyl radical having 1 to 15 C atoms, wherein one or more CH₂ groups, including terminal C atoms, in this radical may each be replaced, independently of one another, by -C=C-, -CH=CH-, 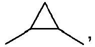 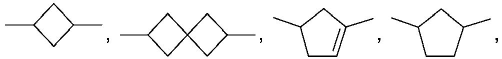 -O-, -S-, -(CO)-O-, -O-(CO)- in such a way that O or S atoms are not linked directly to one another, and in which, in addition, one or more H atoms may be replaced by F or Cl,
A⁰ independently of one another, on each appearance, denotes phenylene-1,4-diyl, in which, in addition, one or two CH groups may be replaced by N and one or more H atoms may be replaced by halogen, CN, CH₃, CHF₂, CH₂F, OCH₃, OCHF₂ or OCF₃, cyclohexane-1,4-diyl, in which, in addition, one or two non-adjacent CH₂ groups may be replaced, independently of one another, by O and/or S and one or more H atoms may be replaced by F, cyclohexene-1,4-diyl, bicyclo[1.1.1]pentane-1,3-diyl, bicyclo[2.2.2]octane-1,4-diyl, spiro[3.3]heptane-2,6-diyl, tetrahydropyran-2,5-diyl or 1,3-dioxane-2,5-diyl,
Z⁰ independently denotes a single bond, -CH₂O-, -(CO)O-, -CF₂O-, -CH₂CH₂CF₂O-, -CF₂CF₂-, -CH₂CF₂-, -CH₂CH₂-, -(CH₂)₄-, -OCH₂-, -OCF₂-, -CH=CH-, -CH=CF-, -CF=CF- or -C=C-, where asymmetrical bridges may be inserted in any orientation,
n denotes 1, 2 or 3, and
and one or more additional compounds.

**2.** Liquid-crystalline medium according to Claim 1, wherein in formula I
A⁰ denotes 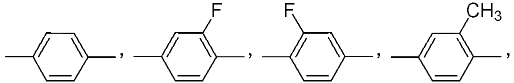 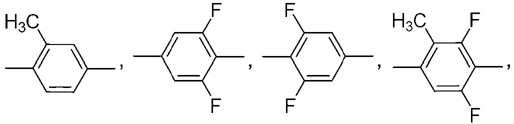 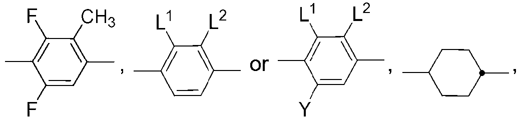 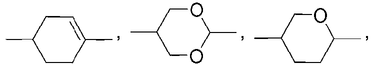
L¹, L² each, independently of one another, denote F, Cl, CH₃ or OCH₃, and
Y denotes CH₃ or CH₂CH₃.

**3.** Liquid-crystalline medium according to Claim 1 comprising one or more compounds of the formula T 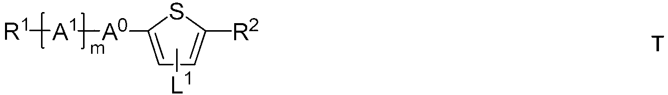 in which the individual radicals have the following meanings:
R¹ and R² independently denote H, F, Cl, Br, -CN or straight-chain or branched alkyl having 1 to 12 C atoms, in which, in addition, one or more CH₂ groups, including terminal C atoms, may each be replaced, independently of one another, by -CH=CH-, -C≡C-, 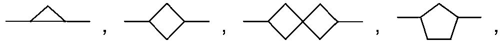 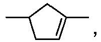 -O-, -S-, -CO-, -CO-O-, -O-CO-, in such a way that O or S atoms are not linked directly to one another, and in which, in addition, one or more H atoms may be replaced by F or CI,
L¹ H, F or CH₃,
A⁰ denotes 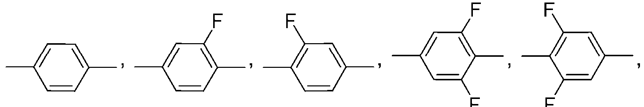 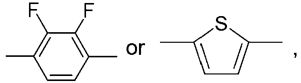 wherein the (fluoro)phenylene rings may optionally be substituted by one or two methyl and/or ethyl groups,
A¹ on each occurrence independently denotes phenylene-1,4-diyl, in which, in addition, one or two CH groups may be replaced by N and one or more H atoms may be replaced by halogen, CN, CH₃, CHF₂, CH₂F, OCH₃, OCHF₂ or OCF₃, cyclohexane-1,4-diyl, in which, in addition, one or two non-adjacent CH₂ groups may be replaced, independently of one another, by O and/or S and one or more H atoms may be replaced by F, cyclohexene-1,4-diyl, bicyclo[1.1.1]pentane-1,3-diyl, bicyclo[2.2.2]octane-1,4-diyl, spiro[3.3]heptane-2,6-diyl, tetrahydropyran-2,5-diyl or 1,3-dioxane-2,5-diyl, and
m in each case, independently of one another, denotes 0, 1 or 2.

**3.** Liquid-crystalline medium according to Claim 1 or 2, **characterized in that** it comprises one or more compounds selected from the group of compounds of formulae II and III. 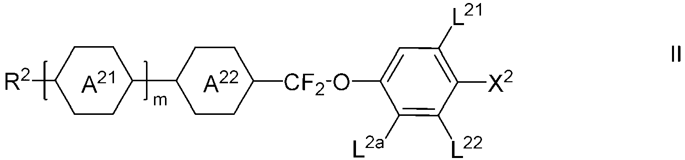 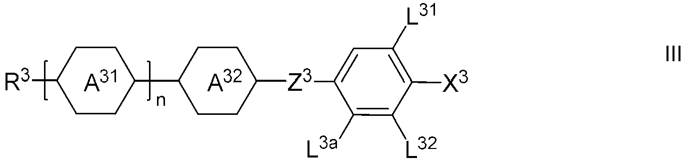 in which
R² independently denotes an alkyl radical having 1 to 15 C atoms, wherein one or more CH₂ groups, including terminal C atoms, in this radical may each be replaced, independently of one another, by -C=C-, -CH=CH-, 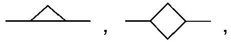 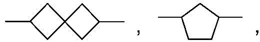 -O-, -S-, -(CO)-O-, -O-(CO)- in such a way that O or S atoms are not linked directly to one another, and in which, in addition, one or more H atoms may be replaced by F or Cl, or H, 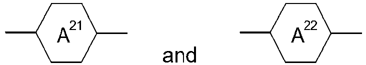 on each appearance, independently are 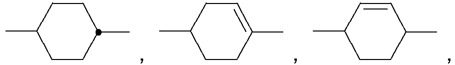 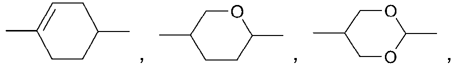 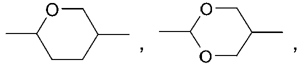 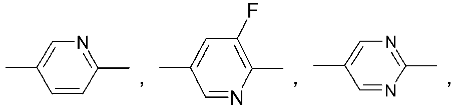 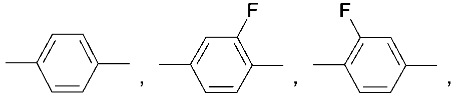 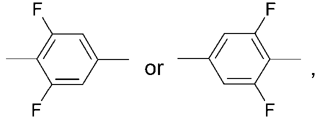 wherein the (fluoro)phenylene rings may optionally be substituted by one or two methyl and/or ethyl groups,
L²¹ and L²² denote H or F,
L^{2a} denotes H or CH₃,
X² denotes halogen, halogenated alkyl or alkoxy having 1 to 3 C atoms or halogenated alkenyl or alkenyloxy having 2 or 3 C atoms,
m denotes 0, 1, 2 or 3,
R³ denotes an alkyl radical having 1 to 15 C atoms, wherein one or more CH₂ groups, including terminal C atoms, in this radical may each be replaced, independently of one another, by -C≡C-, -CH=CH-, 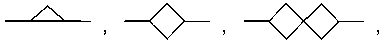 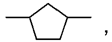 -O-, -S-, -(CO)-O-, -O-(CO)- in such a way that O or S atoms are not linked directly to one another, and in which, in addition, one or more H atoms may be replaced by F or Cl, 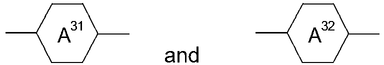 on each appearance, independently of one another, are 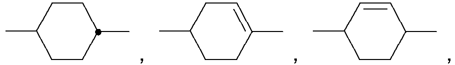 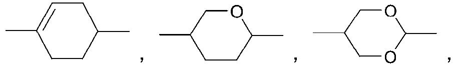 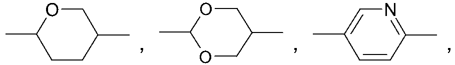 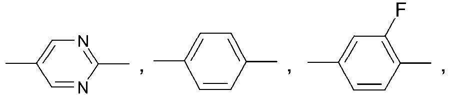 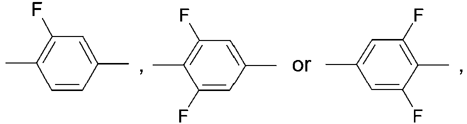 wherein the (fluoro)phenylene rings may optionally be substituted by one or two methyl and/or ethyl groups,
L³¹ and L³², independently of one another, denote H or F,
L^{3a} denotes H or CH₃,
X³ denotes halogen, halogenated alkyl or alkoxy having 1 to 3 C atoms or halogenated alkenyl or alkenyloxy having 2 or 3 C atoms, F, Cl, -OCF₃, -OCHF₂, -O-CH₂CF₃, -O-CH=CF₂, -O-CH=CH₂ or -CF₃,
Z³ denotes -CH₂CH₂-, -CF₂CF₂-, -COO-, trans-CH=CH-, trans-CF=CF-, -CH₂O- or a single bond, and
n denotes 0, 1, 2 or 3.

**4.** Liquid-crystalline medium according to one or more of Claims 1 to 3, **characterized in that** it comprises one or more dielectrically neutral compounds selected from the group of formulae IV and V: 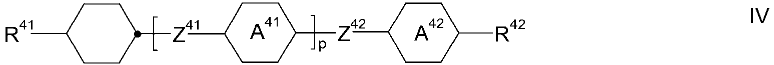 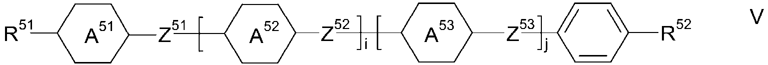 in which
R⁴¹ and R⁴², independently of one another, an alkyl radical having 1 to 15 C atoms, wherein one or more CH₂ groups, including terminal C atoms, in this radical may each be replaced, independently of one another, by -C≡C-, -CH=CH-, 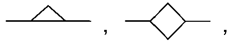 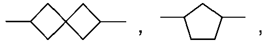 -O-, -S-,
-(CO)-O-, -O-(CO)- in such a way that O or S atoms are not linked directly to one another, and in which, in addition, one or more H atoms may be replaced by F or Cl, 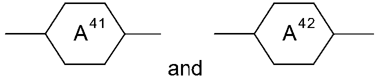 On each appearance, independently of one another denote 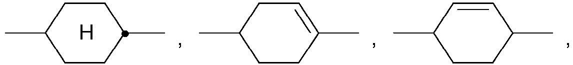 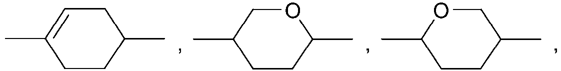 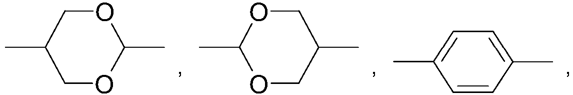 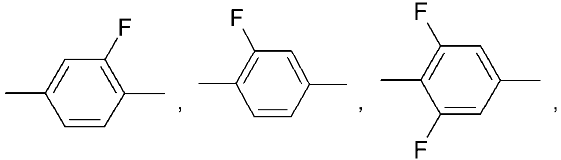 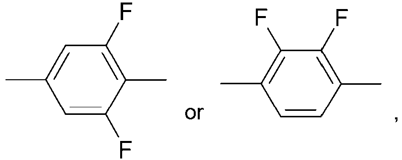 wherein the (fluoro)phenylene rings may optionally be substituted by one or two methyl and/or ethyl groups,
Z⁴¹ and Z⁴² on each appearance, independently of one another, denote -CH₂CH₂-, -COO-, trans-CH=CH-, *trans-*CF=CF-, -CH₂O-, -CF₂O-, -C≡C- or a single bond,
p denotes 0, 1 or 2,
R⁵¹ and R⁵², independently of one another, have one of the meanings given for R⁴¹ and R⁴² 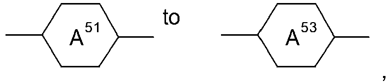 on each appearance, independently of one another, denote 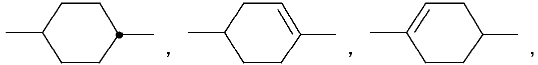 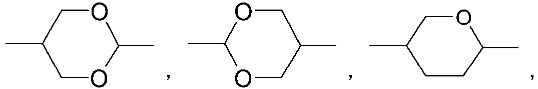 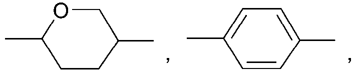 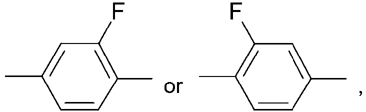 wherein the (fluoro)phenylene rings may optionally be substituted by one or two methyl and/or ethyl groups,
Z⁵¹ to Z⁵³ each, independently of one another, denote -CH₂-CH₂-, -CH₂-O-, -CH=CH-, -C≡C-, -COO- or a single bond, and
i and j each, independently of one another, denote 0 or 1.

**5.** Liquid-crystalline medium according to one or more of claims 1 to 4, **characterised in that** it comprises one or more compounds selected from formula 1-10 or I-11 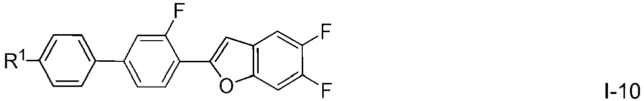 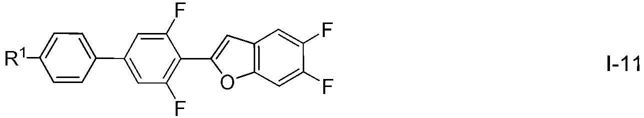 in which R¹ has the meanings indicated in Claim 1.

**6.** Liquid-crystalline medium according to one or more of Claims 1 to 5, **characterized in that** it additionally comprises one or more compounds selected from the formulae III-1j, III-1k, III-1m and III-1n: 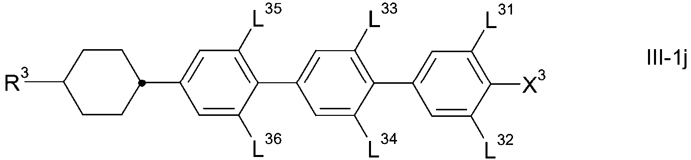 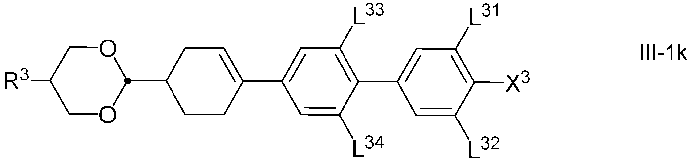 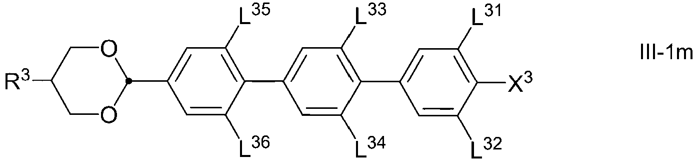 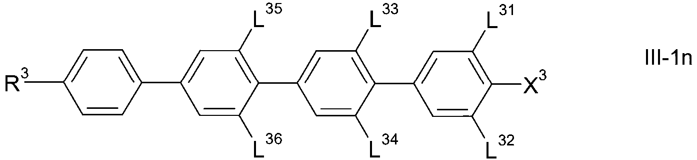 in which
R³ denotes alkyl, alkoxy, fluorinated alkyl or fluorinated alkoxy having 1 to 7 C atoms, alkenyl, alkenyloxy, alkoxyalkyl or fluorinated alkenyl having 2 to 7 C atoms,
X³ denotes F, Cl, halogenated alkyl or alkoxy having 1 to 3 C atoms or halogenated alkenyl or alkenyloxy having 2 or 3 C atoms, F, Cl, -OCF₃, -OCHF₂, -OCH₂F, -O-CH₂CF₃, -O-CH=CF₂, -O-CH=CH₂ or -CF₃,
L³³ and L³¹, independently of one another, denote H or F, and
L³⁵ and L³¹, independently of one another, denote H or F.

**7.** Liquid-crystalline medium according to one or more of Claims 1 to 6, which additionally comprises one or more compounds selected from the group of the compounds of the formulae IV-1 to IV-8, 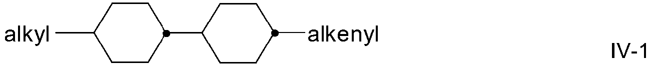 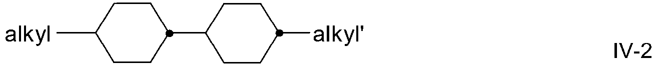 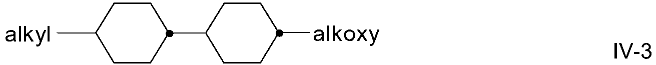 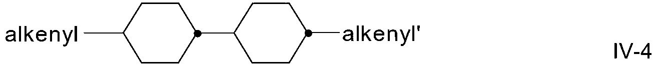 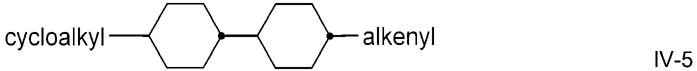 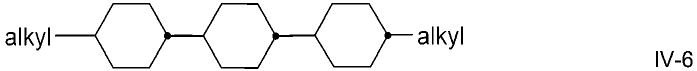 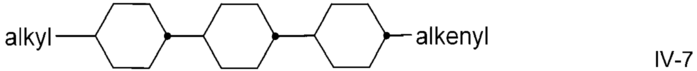 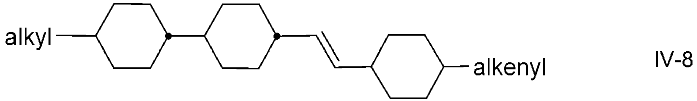 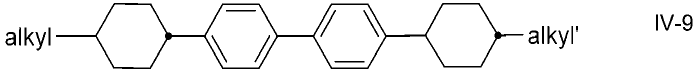 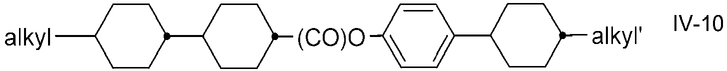 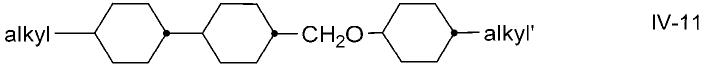 in which
alkyl and alkyl', independently of one another, denote alkyl having 1 to 7 C atoms,
alkenyl, denotes alkenyl having 2 to 5 C atoms,
alkenyl' denotes alkenyl having 2 to 5 C atoms, preferably having 2 to 4 C atoms,
cycloalkyl denotes denotes a cyclic alkyl group having 3 to 12 C atoms, preferably cyclopropyl, cyclobutyl, cyclopentyl, cyclopropylalkyl, cyclobutylalkyl or cyclopentylalkyl, and
alkoxy denotes alkoxy having 1 to 5 C atoms.

**8.** Liquid-crystalline medium according to at least one of Claims 1 to 7, **characterized in that** the total concentration of the compounds of formula I in the medium as a whole is 1 % or more but less than 30 %.

**9.** Liquid-crystalline medium according to at least one of Claims 1 to 8, **characterized in that** it additionally comprises one or more chiral compounds and/or stabilizers.

**10.** Liquid-crystalline compound of formula I according to Claim 1, preferably of formula I-a 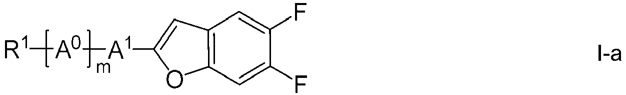 wherein R¹ is defined as in formula I of claim 1,
A¹ is 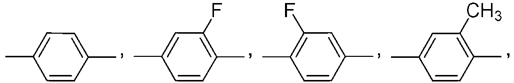 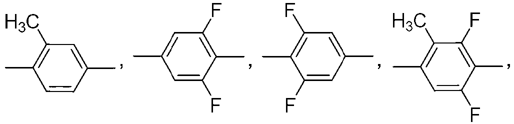 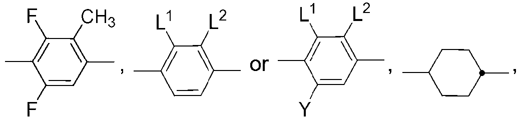 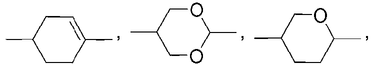
L¹, L² each, independently of one another, denote F, Cl, CH₃ or OCH₃, and
Y denotes CH₃ or CH₂CH₃,
m is 0, 1 or 2, and
A⁰ denotes

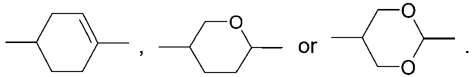

**11.** Electro-optical display or electro-optical component, **characterized in that** it comprises a liquid-crystalline medium according to at least one of Claims 1 to 9.

**12.** Display according to Claim 11, **characterized in that** it is based on the IPS- or FFS mode.

**13.** Display according to Claim 11 or 12, **characterized in that** it contains an active-matrix addressing device.

**14.** Use of a medium according to at least one of Claims 1 to 9 in an electro-optical display, in an electro-optical component or energy-saving applications.

**15.** Process for the preparation of a liquid-crystalline medium according to one or more of Claims 1 to 9, **characterized in that** one or more compounds of formula I are mixed with one or more additional mesogenic compounds and optionally one or more additives.
